(19) Europäisches Patentamt
European Patent Office
Office européen des brevets

(11) Publication number: 0 329 295 A1

(12) **EUROPEAN PATENT APPLICATION**

(21) Application number: 89300825.0

(51) Int. Cl.⁴: C07K 5/02 , A61K 37/64

(22) Date of filing: 27.01.89

(30) Priority: 01.02.88 US 151129

(43) Date of publication of application: 23.08.89 Bulletin 89/34

(84) Designated Contracting States: ES GR

(71) Applicant: THE UPJOHN COMPANY
301 Henrietta Street
Kalamazoo, Michigan 49001(US)

(72) Inventor: Bundy, Gordon L.
7622 Ravenswood Drive
Portage Michigan(US)
Inventor: Thaisrivongs, Suvit
1327 Edington
Portage Michigan(US)
Inventor: Nelson, Norman A.
1643 South 36th Street
Galesburg Michigan(US)
Inventor: Hester, Jackson B. Jr.
9219 East ML Avenue
Galesburg Michigan(US)
Inventor: Fisher, Jed F.
20337 Moorepark Road
Three Rivers Michigan(US)
Inventor: Lipton, Michael F.
828 Lakeway
Kalamazoo Michigan(US)

(74) Representative: Perry, Robert Edward et al
GILL JENNINGS & EVERY 53-64 Chancery Lane
London WC2A 1HN(GB)

(54) **Renin inhibiting peptides with polar end groups.**

(57) The present invention provides novel renin-inhibiting peptides having a noncleavable transition state insert corresponding to the 10,11- position of the renin substrate (angiotensinogen) and a polyhydroxy-substituted alkyl moiety bonded directly through carbon or nitrogen to the N-terminus. Such inhibitors are useful for the diagnosis and control of renin-dependent hypertension and other related diseases.

EP 0 329 295 A1

# RENIN INHIBITING PEPTIDES WITH POLAR END GROUPS

## BACKGROUND OF THE INVENTION

The present invention provides novel compounds. More particularly, the present invention provides novel renin-inhibiting peptide analogs. Most particularly, the present invention provides renin-inhibitory compounds having a variety of polar end groups at the N-terminus, specifically polyhydroxy-substituted-alkyl moieties. The renin inhibitors provided herein are useful for the diagnosis and control of renin-dependent hypertension, congestive heart failure, renin dependent hyperaldosteronism, and other renin dependent cardiovascular disorders.

Renin is an endopeptidase which specifically cleaves a particular peptide bond of its substrate (angiotensinogen), of which the N-terminal sequence in equine substrate is for example:

```
                                          Renin
                                            ↓
Asp-Arg-Val-Tyr-Ile-His-Pro-Phe-His-Leu-Leu-Val-Tyr-Ser-      IA
 1   2   3   4   5   6   7   8   9   10  11  12  13  14
```

as found by L.T. Skeggs el al, J. Exper. Med. 106, 439 (1957). Human renin substrate has a different sequence as recently discovered by D.A. Tewkesbury et al, Biochem. Biophys. Res. Comm. 99, 1311 (1981). It may be represented as follows:

```
Renin
 ↓
-Val-Ile-His-
 11  12  13                    IB
```

and having the sequence to the left of the arrow ( ) being as designated in formula IA above.

Renin cleaves angiotensinogen to produce angiotensin I, which is converted to the potent pressor angiotensin II. A number of angiotensin I converting enzyme inhibitors are known to be useful in the treatment of hypertension. Inhibitors of renin may also be useful in the treatment of hypertension.

A number of renin-inhibitory peptides have been disclosed. Thus, U.S. Patent 4,424,207; European published applications 45,665; 104,041; and 156,322; and U.S. patent application, Serial No. 825,250, filed 3 February 1986; disclose certain peptides with the dipeptide at the 10,11-position containing an isostere bond. A number of statine derivatives stated to renin inhibitors have been disclosed, see, e.g., European published applications 77,028; 81,783; 114,993; 156,319; and 156,321; and U.S. patents 4,478,826; 4,470,971; 4,479,941; and 4,485,099. Terminal disulfide cycles have also been disclosed in renin inhibiting peptides; see, e.g., U.S. patents 4,477,440 and 4,477,441. Aromatic and aliphatic amino acid residues at the 10,11 position of the renin substrate are disclosed in U.S. patents 4,478,827 and 4,455,303. Renin inhibitors containing a C-terminal amide cycle are disclosed in U.S. patent 4,485,099 and European published applications 156,320 and 156,318. Certain tetrapeptides are disclosed in European publications 111,266 and 77,027. Further, European published applications No. 118,223 discloses certain renin inhibiting peptide analogs where the 10-11 peptide link is replaced by a one to four atom carbon or carbon-nitrogen link. Additionally, Holladay et al., in "Synthesis of Hydroxyethylene and Ketomethylene Dipeptide Isosteres", Tetrahedron Letters, Vo. 24, No. 41, pp. 4401-4404, 1983 disclose various intermediates in a process to prepare stereo-directed "ketomethylene" and "hydroxyethylene" dipeptide isosteric functional groups disclosed in the above noted U.S. Patent No. 4,424,207. Evans, et al., J. Org. Chem., 50, 4615 (1985) discloses the synthesis of Hydroxyethylene Dipeptide Isosteres. See also, published European patent application 163,237, which discloses certain renin inhibiting peptides.

Additionally, published European Applications 45,161 and 53,017 disclose amide derivatives useful as inhibitors of angiotensin converting enyzmes.

Certain depeptide and tripeptides are disclosed in U.S. patents 4,514,332; 4,510,085; and 4,548,926 as well as in European published applications 128,762; 152,255; and 181,110. Pepstatin derived renin inhibitors

have been disclosed in U.S. patent 4,481,192. Retro-inverso bond modifications at positions 10-11 have been disclosed in U.S. patent 4,560,505 and in European published applications 127,234 and 127,235. Derivatives of isosteric bond replacements at positions 10-11 have been disclosed in European published applications 143,746 and 144,290; and U.S. patent application, Serial No. 904,149, filed 5 September 1986. Isosteric bond modifications at positions 11-12 and 12-13 have been disclosed in European published application 179,352. Certain peptides containing 2-substituted statine analogues have been disclosed in European published application 157,409. Certain peptides containing 3-aminodeoxystatine have been disclosed in European published application 161,588. Certain peptides containing 1-amino-2-hydroxybutane derivatives as positions 10-11 have been disclosed in European published application 172,346. Certain peptides containing 1-amino-2-hydroxypropane derivatives at positions 10-11 have been disclosed in European published application 172,347. Certain peptides containing N-terminal amide cycles have been disclosed in U.S. patent application, Serial No. 844,716, filed 27 March 1986. Certain peptides containing dihalostatine have been disclosed in PCT application, Serial No. 000,713, filed 7 April 1986. Certain peptides containing C-terminus truncated epoxy or azido or cyano groups or containing a position 10-11 diol and a position 11-12 retro bond have been disclosed in U.S. patent application, Serial No. 945,340, filed 22 December 1986.

European published applications 156,322; 114,993; and 118,223; and PCT patent application, Serial No. 002,227, filed 21 November 1986; U.S. patent application, Serial No. 825,250, filed 3 February 1986; U.S. patent application, Serial No. 904,149, filed 5 September 1986; and U.S. patent application, Serial No. 844,716, filed 27 March 1986; disclose hydroxamic acids or esters at the C-terminus.

E.P. 189,203 discloses new N-dihydroxyalkyl peptide derivatives which are useful as inhibitors of renin for treating hypertension.

E.P. 184,855 discloses new hydroxy substituted-statine peptide derivatives which are useful as inhibitors of renin for treating hypertension.

Derivatives of isosteric bond replacements at positions 10-11 as dihydroxy ethylene isosteres have been disclosed in U.S. patent application, Serial No. 904,149, filed 5 September 1986.

The theoretical principles of such transition-state mimetics of renin inhibitors have been recently reviewed by D.H. Rich, in Chapter 5, "Inhibitors of Aspartic Proteinases," Proteinase Inhibitors, Elsevier Science Publishers BV (Biomedical Division) (1986).

The following references disclose additional substituents at the 10, 11-position: A. Spaltenstein, P. Carpino, F. Miyake and P.B. Hyskins, Tetrahedron Letters, 27:2095 (1986); D.H. Rich and M.S..Bernatowicz, J. Med. Chem., 25:791 (1982); Roger, J. Med. Chem., 28:1062 (1985); D.M. Glick et al., Biochemistry, 21:3746 (1982); D.H. Rich, Biochemistry, 24:3165 (1985); R.L. Johnson, J. Med; Chem., 25:605 (1982); R.L. Johnson and K. Verschovor, J. Med. Chem., 26:1457 (1983); R.L. Johnson, J. Med. Chem., 27:1351 (1984); P.A. Bartlett and W.B. Kezer et al., J. Am. Chem. Soc., 106:4282 (1984); Peptides: Synthesis, Structure and Function (V.J. Hruby; D.H. Rich, eds.) Proc. 8th American Peptide Sym., Pierce Chemical Company, Rockford, Ill., pp. 511-20; 587-590 (1983).

The preparation of cyclopropyl-containing renin inhibiting peptides is disclosed in U.S. paten application, Serial No. 023,404, filed 9 March 1987.

## INFORMATION DISCLOSURE

U.S. Patent 4,613,676 discloses certain substituted 5-amino-4-hydroxy valeryl derivatives, having 2 amino acid residues between the hydrogen or acyl N-terminus and the transition state moiety. A broad class of acyl substituents, including hydroxy-substituted alkyl and etherified sugar moieties, are disclosed.

International Application PCT/EP87/00593 (International Publication Number WO 88/02756 dated 21 April 1988) discloses the incorporation of many types of sugars into peptides other than renin inhibitors.

U.S. Patent 4,729,985 discloses renin inhibitors having a protecting group with a molecular weight less than 500, including, e.g., a (tris-hydroxy)-(t-butyluriedo).

U.S. Patent 4,668,770 generically discloses renin inhibitory tripeptides having a poly-hydroxy group at the C-terminus.

U.K. patent application GB 2200115-A (published 27 July 1988) discloses renin inhibitory peptides having a sugar moiety at the N-terminus attached through an ether linkage.

## SUMMARY OF THE INVENTION

The present invention particularly provides:

A renin inhibitory peptide having a noncleavable transition state insert corresponding to the 10,11-position of the renin substrate (angiotensinogen) and a polyhydroxy-substituted alkyl moiety bonded directly through carbon or nitrogen to the N-terminus.

The present invention also provides renin inhibitors having the substituents noted above and at least three amino acid residues between the N-terminus and the transition state insert. When the polyhydroxy substituted moiety on the N-terminus is THAM, the compounds of the present invention have a heterocyclic or heterocyclic N-oxide or another polyhydroxy-substituted alkyl moiety at the C terminus.

A number of specific examples of such polyhydroxy substituted alkyl moieties are exemplified in the EXAMPLES, CHARTS, and CLAIMS below.

For example, the present invention provides the renin inhibitory peptides having a polyhydroxy substituted alkyl substituent and having amino acid residues and transition state inserts as depicted by the following formulas. In these formulas, the variables A, B, C, D, G, H, and I are meant to depict amino acid residues and E-F depicts a stabilized transition state insert. Thus, examples of compounds of the present invention are those having amino acid residues of the formula:

(1) $-B_7-C_8-D_9-E_{10}-F_{11}-G_{12}-$

wherein $B_7$ is Pro or a derivative thereof; $C_8$ is Phe or a derivative thereof; $D_9$ is His, N(Me)His or a derivative thereof; $E_{10}-F_{11}$ is LVA or a derivative thereof; $G_{12}$ is Ile or a derivative thereof;

(2) $-B_7-C_{81}-D_9-E_{101}-F_{111}-G_{121}-$

wherein $B_7$ is Pro or a derivative thereof; $C_{81}$ is Phe, $(OCH_3)$tyr or a derivative thereof; $D_9$ is His, N(Me)His or a derivative thereof; $E_{101}-F_{111}$ is formula XI, LVA, CVA or a derivative thereof; $G_{121}$ is absent Ile or a derivative thereof;

(3) $-B_7-C_8-D_9-E_{102}-F_{112}-$

wherein $B_7$ is Pro or a derivative thereof; $C_8$ is Phe or a derivative thereof; $D_9$ is His, N(Me)His or a derivative thereof; $E_{102}-F_{112}$ is CVA or a derivative thereof;

(4) $-A_6-B_7-C_8-D_9-E_{103}-F_{113}-G_{123}-$

wherein α-Asn, α-N-Boc-Asn, $-C(0)-(CH_2)_2-C(0)-$ or a derivative thereof; $B_7$ is Pro or a derivative thereof; $C_8$ is Phe or a derivative thereof; $D_9$ is His, N(Me)His or a derivative thereof; $E_{103}-F_{113}$ is LVA, -Chaψ[$CH_2NH$]-Phe-$NH_2$ or a derivative thereof; $G_{123}$ is absent, Ile or a derivative thereof.

Additionally, the renin-inhibitory peptides described in European Application 0173481, published 5 March 1986, at pages 2 to 12 (expressly incorporated herein) are particularly suitable for this invention.

These compounds are shown in relation to the human renin substrate as follows:

| | | | | | | | | | | |
|---|---|---|---|---|---|---|---|---|---|---|
| | 6 | 7 | 8 | 9 | 10 | 11 | 12 | 13 | | |
| | -His | Pro | Phe | His | Leu | Val | Ile | His- | | |
| X | $A_6$ | $B_7$ | $C_8$ | $D_9$ | $E_{10}$ | $F_{11}$ | $G_{12}$ | $H_{13}$ | $I_{14}$ | Z, |

By "renin inhibitory peptide" is meant a compound capable of inhibiting the renin enzyme in mammalian metabolism and having three or more amino acid residues linked by peptide or pseudo-peptidic bonds.

By "a non-cleavable transition state insert" is meant a transition state insert which is not cleavable by a hydrolytic enzyme in mammalian metabolism. A variety of such transition state inserts, corresponding to the 10,11-position of the renin substrate, are known in the art. The following references disclose a variety of such inserts and are presented as merely illustrative of, and not as limiting, the transition state inserts encompassed by the present invention:

U.S. Patent 4,424,207 (Szelke); European Patent 104041A (Szelke); European Patent Application 144,290A (Ciba Geigy AG); European Patent 0,156,322 (Merck); European Patent 161-588A (Merck); European Patent 0,172,347 (Abbott); European Patent 172-346-A (Abbot); European Patent 156-318 (Merck); European Patent 157-409 (Merck); European Patent 152-255 (Sankyo); and U.S. Patent 4,548,926 (Sankyo); and

U.S. patent application, Serial No. 904,149, filed 5 September 1986; U.S. patent application, Serial No. 844,716, filed 27 March 1986; PCT application, Serial No. PCT/US86/00713, filed 7 April 1986; U.S. patent application, Serial No. 945,340, filed 22 December 1986; and U.S. patent application, Serial No. 825,250, filed 3 February 1986; and

A. Spaltenstein, P. Carpino, F. Miyake and P.B. Hyskins, Tetrahedron Letters, 27:2095 (1986); D.H. Rich and M.S. Bernatowicz, J. Med. Chem., 25:791 (1982); Roger, J. Med. Chem., 28:1062 (1985); D.M. Glick et al., Biochemistry, 21:3746 (1982); D.H. Rich, Biochemistry, 24:3165 (1985); R.L. Johnson, J. Med. Chem.,

25:605 (1982); R.L. Johnson and K. Verschovor, J. Med. Chem., 26:1457 (1983); R.L. Johnson, J. Med. Chem., 27:1351 (1984); P.A. Bartlett et al., J. Am. Chem. Soc., 106:4282 (1984); and Peptides: Synthesis, Structure and Function (V.J. Hruby; D.H. Rich, eds.) Proc. 8th American Peptide Sym., Pierce Chemical Company, Rockford, Ill., pp. 511-20; 587-590 (1983).

By "Polyhydroxy-substituted-alkyl moiety" is meant a cyclic or acyclic, branched or unbranched alkyl derivative having from three to nine carbon atoms and from two to eight hydroxyl substituents which are attached to the N-terminus of the renin inhibiting peptide by means of a bond or by amide, urea or carbamate linkages. These polyhydroxy-substituted-alkyl moieties include sugars and other linear or branched polyhydroxy-substituted-alkyl derivatives which are coupled to the N-terminal nitrogen of the renin inhibitor peptide by means of an amino, a carboxylic acid or an aldehyde or ketone substituent on the polyhydroxy-substituted-alkyl moiety. The phrase "bonded directly through carbon or nitrogen" is meant to exclude the use of ether linkages between the polyhydroxy substituent and the peptide. Examples of suitable amino-substituted polyhydroxy-substituted-alkyl derivatives include the aminodesoxyaldoses and the aminodesoxyketoses and their cyclic and reduced derivatives, 2,3-dihydroxy-2-(hydroxymethyl)-propylamine, 2-hydroxy-1,1-bis(hydroxymethyl)ethylamine, 3-hydroxy-2,2-bis(hydroxymethyl)propylamine, 3-amino-1,2-propanediol, 2-amino-1,3-propanediol, 4-hydroxy-3,3-bis(hydroxymethyl)butylamine, and 2,3,4-trihydroxybutylamine. Examples of suitable carboxylic acid substituted polyhydroxy-substituted-alkyl derivatives include the onic and uronic acid sugars such as gluconic and glucuronic acids, glyceric acid, 3-hydroxy-2,2-bis(hydroxymethyl)propionic acid, 2-hydroxy-2,2-bis(hydroxymethyl)-acetic acid, 4-hydroxy-3,3-bis(hydroxy)butyric acid, 2,3,4-trihydroxybutyric acid. Examples of suitable aldehyde or ketone substituted sugars include the aldoses and ketoses which can be condensed directly with the N-terminal amine of renin inhibitory peptides means of a reductive alkylation with, for example, sodium cyanoborohydride. In addition, reducing sugars can be condensed with the N-terminal amine by means of the acid catalyzed Amadori rearrangement. Examples of this and other reactions suitable for incorporating sugars onto peptides having a free amine are set forth in WO published PCT application 87/02756.

By "derivatives" of amino acids is meant the well known amino acid derivatives commonly employed in renin inhibitors as set forth in the reference described above.

As is apparent to those of ordinary skill in the art, the renin inhibitor peptides of the present invention can occur in several isomeric forms, depending on the configuration around the asymmetric carbon atoms. All such isomeric forms are included within the scope of the present invention. Preferably, the stereochemistry of the amino acids corresponds to that of the naturally-occurring amino acids. Where the stereochemistry of the amino acid is not indicated, it is preferably in the L-configuration.

Renin inhibitory peptides commonly have protecting groups of at the N-terminus and the C-terminus. Many of these protecting groups are known in the polypeptide art. Examples of such protecting groups are given below.

The renin inhibitory peptides of the present invention have a variety of novel polar end groups at the N-terminus. These polar end groups will increase the water solubility of the peptides, improve the oral absorption characteristics of the peptides and/or slow the metabolic degradation of the peptides, all while retaining the therapeutically useful renin-inhibiting activity of the peptides.

Examples of pharmaceutically acceptable acid addition salts include: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methansulfonate, 2-naphthalenesulfonate, nicotinate, oxalate, palmoate, pectinate, persulfate, 3-phenylpropionate, picrate, pivalate, propionate, succinate, tartrate, thiocyanate, tosylate, and undecanoate.

The carbon atom content of various hydrocarbon-containing moieties is indicated by a prefix designating the minimum and maximum number of carbon atoms in the moiety, i.e., the prefix ($C_i$-$C_j$) indicates a moiety of the integer "i" to the integer "j" carbon atoms, inclusive. Thus ($C_1$-$C_4$)alkyl refers to alkyl of one to 4 carbon atoms, inclusive, or methyl, ethyl, propyl, butyl, and isomeric forms thereof. $C_4$-$C_7$cyclic amino indicates a monocyclic group containing one nitrogen and 4 to 7 carbon atoms.

Examples of ($C_3$-$C_{10}$)cycloalkyl which include alkyl-substituted cycloalkyl containing a total of up to 10 total carbon atoms, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, cyclopentyl, 2,2-dimethylcyclopentyl, cyclohexyl, cycloheptyl, cyclooctyl, cyclononyl, cyclodecyl and isomeric forms thereof.

Examples of aryl include phenyl, naphthyl, (o-, m-, p-)tolyl, (o-, m-, p-)ethylphenyl, 2-ethyl-tolyl, 4-ethyl-o-tolyl, 5-ethyl-m-tolyl, (o-, m-, or p-)propylphenyl, 2-propyl-(o-, m-, or p-)tolyl, 4-isopropyl-2,6-xylyl, 3-propyl-4-ethylphenyl, (2,3,4- 2,3,6-, or 2,4,5-)-trimethylphenyl, (o-, m-, or p-)fluorophenyl, (o-, m-, or p-trifluoromethyl)phenyl, 4-fluoro-2,5-xylyl, (2,4-, 2,5-, 2,6-, 3,4-, or 3,5-)difluorophenyl, (o-, m-, or p-)chlorophenyl, 2-chloro-p-tolyl, (3-, 4-, or 6-)chloro-o-tolyl, 4-chloro-2-propylphenyl, 2-isopropyl-4-chlorophenyl, 4-chloro-3-fluorophenyl, (3- or 4-)chloro2-fluorophenyl, (o-, m-, or p-)trifluoro-methylphenyl, (o-, m-, or p-)ethoxyphenyl, (4- or 5-)chloro-2-methoxy-phenyl, and 2,4-dichloro(5- or 6-)methylphenyl, and the like.

Examples of -Het include: 2-, 3-, or 4-pyridyl, imidazolyl, indolyl, $N^{in}$-formyl-indolyl, $N^{in}$-$C_1$-$C_5$alkyl-C(0)-indolyl, [1,2,4]-triazolyl, 2-, 4-, or 5-pyrimidinyl, 2- or 3-thienyl, piperidinyl, pyrryl, pyrrolinyl, pyrrolidinyl, pyrazolyl, pyrazolinyl, pyrazolidinyl, imidazolinyl, imidazolidinyl, pyrazinyl, piperazinyl, pyridazinyl, oxazolyl, oxazolidinyl, isoxazolyl, isoxazolidinyl, morpholinyl, thiazolyl, thiazolidinyl, isothiazolyl, isothiazolidinyl, quinolinyl, isoquinolinyl, benzimidazolyl, benzothiazolyl, benzoxazolyl, furyl, thienyl, and benzothienyl. Each of these moieties may be substituted as noted above.

As would be generally recognized by those skilled in the art of organic chemistry, a heterocycle as defined herein for -Het would not be bonded through oxygen of sulfur or through nitrogen which is within a ring and part of a double bond.

Halo is halogen (fluoro, chloro, bromo, or iodo) or trifluoromethyl.

Examples of pharmaceutically acceptable cations include: pharmacologically acceptable metal cations, ammonium, amine cations, or quaternary ammonium cations. Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are also within the scope of this invention. Pharmacologically acceptable amine cations are those derived from primary, secondary, or tertiary amines. The novel peptides herein contain both natural and synthetic amino acid residues. These residues are depicted using standard amino acid abbreviations (see, e.g., Eur. J. Biochem., 138, 9 (1984)) unless otherwise indicated.

In addition to the treatment of warm-blood animals such as mice, rats, horses, dogs, cats, etc., the compounds of the invention are effective in the treatment of humans.

The renin inhibitors of this invention are useful for treating any medical condition for which it is beneficial to reduce the levels of active circulating renin. Examples of such conditions include renin-associated hypertension and hyperaldosteronism, hypertension, hypertension under treatment with another antihypertensive and/or a diuretic agent, congestive heart failure, angina, and post-myocardial infarction. The renin-angiotension system play a role in maintenance of intracellular homeostasis: see Clinical and Experimental Hypertension, 86, 1739-1742 (1984) at page 1740 under Discussion. The compounds will also be useful as molecular probes for the diagnosis and study of the physiology of blood pressure regulation or other physiological functions.

Further, the renin inhibitors of this invention may be useful in the treatment of cerebrovascular disorders and disorders of intracellular homeotasis. The possible role of the renin-angiotensin system in the maintenance of intracellular homeostasis is disclosed in Clinical and Experimental Hypertension, 86:1739-1742 (1984). Additionally, the renin inhibitors of this invention potentiate the antithrombotic activity of a thromboxane antagonist (U.S. patent 4,558,037). The antihypertensive effect of the renin inhibitors of this invention are potentiated by combination with a thromboxane synthetase inhibitor.

The compounds of the present invention are preferably orally administered to humans to effect renin inhibition for the purpose of favorably affecting blood pressure. For this purpose, the compounds are administered from 0.1 mg to 1000 mg per kg per dose, administered from 1 to 4 times daily. The compounds of the present invention are preferably orally administered either as the present compound or in the form of pharmacologically acceptable acid addition salts. Preferred pharmacologically acceptable salts for oral administration include the citrate and aspartate salts, although any pharmacologically acceptable salt is useful in this invention, including those listed above. These salts may be in hydrated or solvated form. Other routes of administration include parenteral, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic

acid find use in the preparation of injectibles.

Equivalent dosages for such other routes of administration are thus employed. The exact dose depends on the age, weight, and condition of the patient and on the frequency and route of administration. Such variations are within the skill of the practitioner or can readily be determined.

The compounds of the present invention may be in the form of pharmaceutically acceptable salts both those which can be produced from the free bases by methods well known in the art and those with which acids have pharmacologically acceptable conjugate bases.

Conventional forms and means for administering renin-inhibiting compounds may be employed and are described, e.g., in U.S. Patent No. 4,424,207 which is incorporated by reference herein. Likewise, the amounts disclosed in the U.S. Patent No. 4,424,207 are examples applicable to the compounds of the present invention.

The renin-inhibiting compounds of this invention may be administered in combination with other agents used in antihypertensive therapy such as diuretics, α and/or β-adrenergic blocking agents, CNS-acting agents, adrenergic neuron blocking agents, vasodilators, angiotensin I converting enzyme inhibitors, and the like as described, for example, in published European patent application 156 318.

For example, the compounds of this invention can be given in combination with such compounds or salts or other derivative forms thereof as:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynaten; trimaterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
(((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]-propoxy)-indole;
(carbazolyl-4-oxy-5,2-(2-methoxyphenoxy)-ethylamino-2-propanol);
(1-((1,1-dimethylethyl)amino)-3-((2-methyl ′H-indol-4-yl)oxy)-2-pro panol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[2-indol-3-yl-1,1-dimethylethyl)-amino]propoxy]benzonitrile HCl) (bucindolol;
(α-[tert.butylamino)methyl]-7-ethyl-2-benzofuranmethanol) (bufuralol);
(3-[3-acetyl-4-[3-(tert.butylamino)-2-hydroxypropyl]-phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol)); ((±)-3′-acetyl-4′-(2-hydroxy-3-isopropylaminopropoxy)-acetanilide HCl) (diacetolol);
(methyl-4-[2-hydroxy-3-[(1-methylethyl)aminopropoxyl]]-benzenepropanoate HCl) (esmolol);
(erythro-DL-1-(7-methylindan-4-yloxy)-3-isopropylaminobutan-2-ol); (1-(tert.butylamino)-3-[0-(2-propynyloxy)-phenoxy]-2-propanol (pargolol);
(1-(tert.butylamino)-3-[o-6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)-amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl); (4-[3-(tert.butylamino)-2-hydroxypropoxy]-N-methylisocarbostyril HCL);
((±)-N-2-[4-(2-hydroxy-3-isopropylaminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[(1-methylethyl)amino]propoxyphenyl]-butanamide) (acebutolol);
((±)-4′-[tert-butylamino)-2-hydroxypropoxy]spiro[cyclohexane-1,2′- indan]-1′-one) (spirendolol);
(7-[3-[[2-hydroxy-3-[(2-methylindol-4-)oxylpropyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-tert.butylamino)-2-hydroxy-propoxy)benzonitrile HCl) (bunitrolol);
((±)-2′-[3-(tert.butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbotyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5-6,7,8-tetrahydro-cis-6,7-dihydroxy-1-naphthyl)oxy]-2-propanol) (nadolol);
((S)-1-(2-cyclopentylphenoxy)-3-[1,1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxyisoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-β-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydrcxypropyl]aminoethyl]hydantoin HCl);
(3,4-dihydro-8-(2-hydroxy-3-isopropylaminopropoxy)-3-nitroxy-2H-1-benzopyran) (nipradolol);

Angiotensin I Converting Enzyme Inhibitors:

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[1-(ethoxycarbonyl)-3-phenyl-propyl]amino]-1-oxopropyl]-1,2,3,4-tetrahydro-3-isoquinoline carboxylic acid);
((S)-1-[2-[(1-(ethoxycarbonyl)-3-phenylpropy]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$[1-(S)-carboxy-3-phenylpropyl]-L-lysyl-L-proline (lysinopril);

Other antihypertensive agents include:

aminophylline; cryp tenamine acetates and tannates; deserpidine; meremethoxylline procaine; pargyline; trimethaphan camsylate; and the like, as well as admixtures and combinations thereof.

Typically, the individual daily dosages for these combinations can range from about one-fifth of the minimally recommended clinical dosages to the maximum recommended levels for the entries when they

are given singly. Coadministration is most readily accomplished by combining the active ingredients into a suitable unit dosage form containing the proper dosages of each. Other methods of coadministration are, of course, possible.

The novel peptides of the present invention possess an excellent degree of activity in treating renin-associated hypertension and hyperaldosteronism.

Renin inhibitors have also been disclosed to control the rise in intraocular pressure associated with the use of steroidal antiinflammatory drugs as described in International Application PCT/-US86/02291 (International Publication Number WO 87/02581 dated 7 May 1987).

The compounds of the present invention are prepared as depicted in the charts and as described more fully in the Preparations and Examples.

## Chart A

In Chart A is described the conversion of N-deprotected Boc-Pro-Phe-N(Me)His-LVA-Ile-AMP, i.e., Pro-Phe-N(Me)His-LVA-Ile-AMP into its tris(hydroxymethyl)methylamino (THAM) urea derivative. The preparation of Boc-Pro-Phe-N(Me)His-LVA-Ile-AMP is described in U.S. patent application, Serial No. 147,073, field 20 January 1988, and in published European patent application 0173481, published 5 March 1986, which are hereby incorporated by reference. Referring to Chart A, reaction of the A-1 compound with the A-2 compound yields the A-3 compound. Reduction of the A-3 compound, e.g., with hydrogen yields the A-4 compound. Y. Yokoyama et al., Macromolecules 15:11 (1982). Treatment of the A-4 compound with phenyl chloroformate yields the A-5 phenylcarbamate. This new crystalline, stable intermediate is useful for the conversion of any primary or secondary amine to the corresponding THAM urea. Addition of the A-5 phenyl carbamate to Pro-Phe-N(Me)His-LVA-Ile-AMP affords the A-6 orthoester-protected urea. Using methanolic hydrochloric acid prepared by bubbling hydrogen chloride gas through methanol, the orthoester protecting group is removed and the A-7 deprotected triol is obtained. During some runs of this sequence, compounds of the formula IV may also be obtained.

## CHART B

Removal of the Boc group from the B-1 compound, treatment with carbamate A-5, and oxidation with m-chloroperbenzoic acid gives intermediate B-3. Using methanolic hydrochloric acid prepared by bubbling hydrogen chloride gas through methanol, the orthoester protecting group is removed and the B-4 deprotected triol is obtained. During some runs of this sequence, compounds of the formula V may also be obtained. Oxidation of the B-1 His(Ts) intermediate prepared as described in U.S. patent application, Serial No. 147,073, filed 20 January 1988, with m-chloroperbenzoic acid followed by detosylation gives the B-2 N-oxide. Both modifications - the N-oxide and the THAM urea - are incorporated into the same molecule with the B-4 compound.

## CHART C

In Chart C, the preparation of C-terminal THAM amides is described. The C-2 THAM amide (wherein R is H) of Boc-Ile-OH is prepared via DCC-mediated coupling of the C-1 Boc-Ile-OH and THAM. Acetylation of the C-2 THAM amide ($Ac_2O$, pyridine) affords the corresponding triacetate (wherein R is acetyl). Each of these intermediates is further elaborated separately as indicated in Chart C.

The triacetate is coupled with the protected LVA intermediate to yield the C-3 compound. Subsequent block coupling with the Boc-Pro-Phe-His(Ts)-OH fragment yields the C-4 compound. The tosyl and silyl protecting groups are simultaneously removed from the final product using tetrabutylammonium fluoride in tetrahydrofuran.

## CHART D

In Chart D, N(Me)His THAM amides D-5 (R is acetyl or hydrogen) are prepared in classical stepwise fashion from intermediate C-3 (R is H or acetyl) (Chart C). Removal of N-protecting groups (Boc or Ts) are accomplished using standard procedures. Likewise, the peptide bond formations are done using diethylphosphoryl cyanide, dicyclohexylcarbodiimide or other usual coupling reagents.

## CHART E

In chart E is summarized the preparation of the Boc-Pro-Phe-N(Me)His-LVA-Ile-AMP analog in which an unsymmetrical urea is formed using de-Boc'd Boc-Pro-Phe-N(Me)His-LVA-Ile-AMP, i.e., Pro-Phe-N(Me)His-LVA-Ile-AMP as one amine component and glucosamine as the other. Treatment of D-glucosamine with phenyl chloroformate (following a procedure analogous to that of Chargaff and Bovarnick, J. Biol. Chem., 118:421(1937)) subsequent acetylation affords crystalline carbamate E-2. The E-3 compound (R is acetyl of H) is prepared by reaction of E-2 with Pro-Phe-N(Me)His-LVA-Ile-AMP.

## CHART F

Chart F describes the preparation of renin inhibitory peptides of the present invention having $(R_{120})$-$(R_{124})N(CH_2)_nC(O)$- at the N-terminal of the peptide.

The amines of formula F-1 are either known compounds or are easily prepared by known methods. Conversion of the amines of formula F-1 to the unsymmetrical ureas of formula F-3 can be done by a number of standard methods, a convenient one of which is described in Tetrahedron Letters 1935 (1977).

The formula F-1 amine is treated with phenyl chloroformate to obtain the compound of formula F-2. The formula F-2 compound is joined to a peptide such as $H$-$A_6$-$B_7$-$C_8$-$D_9$-$E_{10}$-$F_{11}$-$G_{12}$-$H_{13}$-$I_{14}$-Z to obtain the unsymmetrical ureas of formula F-3. In formula F-3, "-$NHA_6$-" represents the amino nitrogen from the amino acid $A_6$.

Generally the hydroxy groups in $R_{120}$ and $R_{124}$ are protected during the urea formation steps as non-polar derivatives, for example, as ethers, orthoesters or acylates.

The unsymmetrical urea products of the formula F-3 are stable to both acidic and basic blocking group removal conditions.

## CHART G

Chart G describes the preparation of renin inhibitory peptides of the present invention having $-N(R_{120})(R_{124})$ at the C-terminal of the peptide.

The amines of formula G-1 are either known compounds or are easily prepared by known methods. The amides of formula G-2 are prepared from the amines of formula G-1 by standard peptide-forming reactions. In formula G-2, "$I_{14}C(O)$-" represents the carbonyl group from the amino acid $I_{14}$.

The carboxy and hydroxy groups of $R_{120}$ and $R_{124}$ are best protected as non-polar derivatives during the coupling reactions for example, esters for the carboxy; ethers, orthoesters or acylates for the hydroxys. In Charts F and G, in peptides wherein $R_{120}$ is (c) $R_{123}OOC$-$C((CH_2)_n$-$CO_2R_{123})_2$, this substituent may be prepared from aminocitric acid, a known compound. See A. Dornow, K. Rombusch, Chem. Ber. 88:1334 (1965). In peptides wherein $R_{120}$ is (d) $HO$-$CH_2$-$CH(OH)$-$CH_2$-, this substituent may be prepared from 3-amino-1,2-propanediol (Aldrich Chem. Co.). In peptides wherein $R_{120}$ is the structure in (e), this substituent may be prepared by standard techniques from the substituent in (d).

## CHART H

Renin inhibitory peptides of the present invention include compounds with a polar N-terminal group and utilizing the small C-terminal truncated diol as the transition-state insert. The polar groups are attached to the N-terminal proline of the smallest C-terminal truncated renin inhibitory peptide template.

The aldehyde H-1 shown in Chart H has already been described in PCT patent application, Serial Number PCT/US87/03007, filed 23 November 1987, and is now available in large quantity. Addition of isobutyl magnesium bromide which leads directly to the compound H-4 proceeds in low yield due to competing reduction of the aldehyde to the corresponding alcohol. Addition of 2-methyl-1-propenyl magnesium bromide, however, affords the adducts in very good yield. The two epimers H-2 and H-3 can be separated by chromatography. The major adduct H-3 is then hydrogenated to the saturated compound H-4. The protecting groups are removed with acidic methanol to give the free amine H-5. Appropriate amino acids can then be coupled to give desired peptides.

CHART I

Chart I shows the preparation of the renin inhibitor I-4. The known phenyl urethane I-1 is reacted with the amine I-2 to give the urea I-3. The protecting group is removed with acid to give the desired compound I-4. The corresponding renin inhibitor having-$(0CH_3)$Tyr and -His in place of -Phe and -N(Me)His is prepared in an analogous manner.

CHART J

For the preparation of the compounds of Chart K, the building block J-4 is needed and it is prepared as shown in Chart J. The known urethane J-1 is reacted with proline benzyl ester to give the ester J-3. The ester J-3 is then hydrogenolyzed to the corresponding acid J-4.

CHART K

Chart K shows the preparation of the renin inhibitor K-4. The acid K-1, prepared as the compound J-4 in Chart J, is coupled to the amine K-2 to give the adduct K-3. The protecting group is removed with ammonia in methanol to give the desired compound K-4. The corresponding renin inhibitor having -$(0CH_3)$Tyr and -His in place of -Phe and-N(Me)His is prepared in an analogous manner.

CHART L

The starting material is the $N(CH_3)$His protected compound L-1, prepared as described in U.S. patent application, Serial No. 147,073, filed 20 January 1988, and in European application 0173481, published 5 March 1986. The Boc protective group is removed in the usual way (trifluoroacetic acid) to give L-2 Polar end groups, derived from shikimic acid and 2,2-bis(hydroxymethyl)propionic acid, are then attached to the N-terminus and, finally the tosyl protective group on the $N(CH_3)$His(Ts) unit is removed. The two final products prepared, L-4A and L-4b are obtained.

CHART M

Connecting serinol M-1 to the N-terminus of a peptide through a ureido link creates an isostere that resembles a 2-acyl glyceride. Treatment of serinol M-1 with phenyl chloroformate, followed by acetic anhydride gives the protected phenyl urethane M-2. Coupling of M-2 to de-Boc'd Boc-Pro-Phe-N(Me)His-LVA-Ile-AMP, i.e., Pro-Phe-N(Me)His-LVA-Ile-AMP affords intermediate M-3. Selective aminolysis of M-3 gives the desired product M-4. And, finally, oxidation of M-4 with meta-chloroperoxybenzoic acid yields the corresponding pyridine N-oxide M-5.

## CHARTS N AND O

Chart N illustrates the preparation of the renin inhibitor N-4, which is further described in Example 23 below. Chart O illustrates the preparation of the renin inhibitor 0-4, which is further described in Example 24 below.

## CHART P

Renin inhibitors of generic formula P-4 can be prepared as described in Chart P.

The renin inhibitor residue preferably contains Pro at the $P_4$ site, an aromatic amino acid residue at the $P_3$ site (e.g., Phe, Tyr (0-Me ether, etc.), His or N(Me)His at the $P_2$ site, a transition-state unit occupying the $P_1$-$P_1'$ sites such as LVA, CVA or other transition-state units as described above and may contain other binding units at the $P_2'$ and $P_3'$ sites as described above. An example of this synthetic procedure is the preparation of renin inhibitor N-4 as described in Example 23 below and Chart N above. The stereochemistry of the carbinol centers in the sugar-like residues attached to the amino termini can be R or S.

The starting materials P-1 are commercially available (e.g., glucamine and $HOCH_2$-$CHOH$-$CH_2$-$NH_2$), or are described in the literature. See F. Kagan, M.A. Rebenstorf and R.V. Heinzelman, J. Am. Chem. Soc., 79:3541 (1957); G.W.E. Plant and R.A. Harvey, Methods Enzymol., 18(Issue Pt B):515 (1971); M. Israel and N. Muhammad, J. Heterocycl. Chem., 10:209 (1973); S. Honda, S. Suzuki and K. Kakehi, J. Chromatography, 396:93 (1987); H. Pischel, A. Holy, J. Vesely and G. Wagner, Coll. Czech. Chem. Commun., 52:2061 (1987); J. Davoll and D.D. Evans, J. Chem. Soc., 5041 (1960); or can be prepared from aldoses by reductive amination using procedures described in the above references. Especially preferred starting materials are 1-amino-2,3-propanediol, glucamine or the aldamines derived by reductive amination of D-arabinose, L-arabinose, D-xylose, D-ribose, D-mannose and D-galactose.

## CHART Q

Renin inhibitors of generic formula Q-2 can be prepared as described in Chart Q. The stereochemistry of the carbinol centers in the sugar-like residues attached to the amino termini can be R or S.

The definition of renin inhibitors residue is as defined above. The preferred aldoses are D-arabinose, L-arabinose, D-xylose, D-ribose, D-glucose, D-mannose and D-galactose. The reductive amination is carried out as described earlier for the preparation of renin inhibitors of formula Q-2 wherein n is 3 or 4 and the renin inhibitor residue is -Pro-Phe-N(Me)His-LVA-Ile-AMP in Examples 25 and 26 below. (See also, the reference described above for reductive amination of aldoses to produce aldamines).

Pharmacologically acceptable salts of Q-2 are part of the compounds of the present invention.

Renin inhibitors of generic formula Q-3 can be prepared in several ways. Where Renin Inhibitor Residue is as defined above and the preferred aldonic acid residues are derived from D-arabinose, L-arabinose, D-xylose, D-ribose, D-glucose, D-mannose and D-galactose, the stereochemistry of the chiral carbinol centers in the aldonic acid unit can be R or S. The common aldonic acids or their lactones are known compounds and some are commercially available (e.g., D-glucono-$\delta$-lactone, D-gluconic acid, potassium salt, etc.).

Aldonic acids are readily prepared from the corresponding aldoses by hypoiodite oxidation, bromine oxidation or electrolytic oxidation (see Methods in Carbohydrate Chemistry, Volume II, 11-15 (1963) edited by R.L. Whistler, M. L. Wolfrom and S.N. BeMiller). The corresponding $\alpha$ and/or $\gamma$ lactones can be readily prepared (see reference above. pp 16-18). An ammonolysis reaction is an aldonic acid lactone ($\gamma$ or $\delta$ lactone) and a renin inhibitor having an amino group on the amino terminus leads directly to the desired product Q-3. For example, heating D-glucono-$\delta$-lactone with the desired peptide in dimethylformamide yields the renin inhibitor of formula Q-3 wherein n is 4 and the renin inhibitor residue is -Pro-Phe-N(Me)His-LVA-Ile-AMP as described in Example 27 below. (Method analogous to F. Scholnick and P.E. Pfeffer, J. Dairy Sci., 63:471 (1980); and M.A.M. Nassr, M.A.M. Taha and M.A.E. Shaban, Carbohydrates Res., 121:119 (1983). (See, for example, the method of R.L. Sneath, Jr., J.E. Wright, A.H. Soloway, S.M. O'Keefe, A.S. Dey and W.D. Smolnycki, J. Med. Chem., 19:1290 (1976)).

Another way to obtain products of type Q-3 is to acylate the renin inhibitor having a free amino group on the amino terminus with a fully inhibitor aldonyl chloride and then remove the acetyl protective groups

by ammonolysis with methanolic ammonia (see conversion of P-3 to P-4). The fully acetylated aldonyl chloride is obtained by treating the fully acetylated aldonic acid with phosphorous pentachloride in ether or other suitable solvent (references below). The fully acetylated aldonic acids, in turn, are obtained by acetylation of the aldonic acid salts with acetic anhydride in the presence of hydrogen chloride or by stepwise acetylation of aldonic acid lactones with acetic anhydride and fused zinc chloride. Procedures for preparing fully acetylated aldonyl chlorides are, for example, Methods in Carbohydrate Chemistry, Volume II, 21-26 (1963), edited by R.L. Whistler, M.L. Wolfrom and J.N. BeMiller; C.E. Braun and C.D. Cook, Org. Synth., Coll., Vol 5:887 (1973).

The reaction of the acetylated aldonyl chlorides with the basic renin inhibitor can be carried out in, for example, pyridine, and the various products are purified as described earlier for related structures (i.e., extractions, washings, chromatography, etc.). The acylation of a basic renin inhibitor will occur selectively on the terminal amino group. If the transition-state unit contains an amino group (e.g., in the form of a reduced dipeptide unit) it is not necessary to selectively block the amino group of the transition-state unit. Any acylation that occurs on the imidazole ring of a histidine unit can be reversed by selective hydrolysis of the imidazoyl amide so formed.

## CHARTS R AND S

Charts R and S exemplify the preparation of renin inhibitory peptides obtained by and amide linkage to the N-terminal phenylalanine or N-terminal proline of an alkanedioic acid having attached to its remaining free carboxylic acid moiety an N-linked saccharide, especially monosaccharides.

Examples of alkanedioic acid include succinic acid, L-aspartic acid, D-aspartic acid, N-protected L- and D-aspartic acid and derivatives thereof. Examples of monosaccharides include mannose and 2-amino-2-deoxyglucose and derivatives thereof, including N-acyl derivatives.

In Chart R, commercially available chloride R-1 is converted to the asparagine fragment R-5 via the azide R-2. Hydrogenation of R-2 affords the amine R-3 which is coupled with L-aspartic acid, α-benzylester having conventional chemistry to give R-4. Hydrogenation of R-4 provides R-5. Coupling of R-5 separately with R-6 and R-9 affords R-7 and R-10, respectively. Base hydrolysis of R-7 gives R-8 and base hydrolysis of R-9 provides R-11.

In a similar fashion, the mannose-asparagine fragment is prepared and coupled as outlined in Chart S.

## CHART T

Chart T describes the preparation of transition-state insert Chaψ[CH(0H)$CH_2$]Val(CVA). U.S. Patent Application Serial No. 213,787, filed 30 June 1988, which is hereby incorporated by reference, provides novel processes and intermediates for the synthesis of the hydroxyethylene isostere dipeptide leu-val and similar transition state moieties. Processes and intermediate analogous to those described therein be used to prepare CVA.

## TABLE A

Table A describes additional renin inhibitor compounds that may be prepared having polar end groups at the N-terminus and CVA as the transition state insert.

The compound of formula XX, wherein X is an electron-withdrawing group and anion stabilizing group, for example, $-N0_2$ and -Br; and wherein $R_{11}$, $R_{12}$, and $R_{13}$ are each independently trimethylsilyl pr t-butyldimethylsilyl; is useful as an intermediate in making renin inhibitor peptides, especially useful as an acylating agent. Especially preferred are compounds of formula XX wherein $R_{11}$, $R_{12}$ and $R_{13}$ are each trimethylsilyl or wherein one of $R_{11}$, $R_{12}$ and $R_{13}$ is t-butyldimethylsilyl and the remaining two are trimethylsilyl. The compound of formula XX wherein $R_{10}$ is H and $R_{11}$, $R_{12}$ and $R_{13}$ are each trimethylsilyl is prepared as described in Preparation 49, and is used in the preparation of the renin inhibitor peptide of formula B-4 (Refer to Chart B), Example 46.

Sawyer, et al, Tetrahedron, Vol 44, 1988, pages 661-673, discloses the preparation of the transition

state insert Cha$\psi$-[$CH_2NH$]Phe-$NH_2$.

Generally, the renin inhibiting polypeptides may be prepared by either polymer assisted or solution phase peptide synthetic procedures analogous to those described hereinafter or to those methods known in the art. For example, the carboxylic moiety of $N^{\alpha}$-t-butyloxycarbonyl (Boc)-substituted amino acid derivatives having suitable side chain protecting groups, if necessary, may be condensed with the amino functionality of a suitably protected amino acid, peptide or polymer-bound peptide using a conventional coupling protocol such as dicyclohexylcarbodiimide (DCC) and 1-hydroxybenzotriazole (HOBT) or diethylphosphoryl cyanide (DEPC) and triethylamine ($Et_3N$) in methylene chloride or dimethylformamide. The synthetic procedures used to incorporate the novel moieties herein are analogous to those described, for example, in U.S. patents 4,424,207; 4,470,971; 4,477,440; 4,477,441; 4,478,826; 4,478,827; 4,479,941; and 4,485,099, and copending application Serial No. 753,198, filed 9 July 1985, and copending application Serial No. 825,250, filed 3 February 1986, all of which are expressly incorporated by reference herein. See, also, published European patent applications 45,161; 45,665; 53,017; 77,028; 77,029; 81,783; 104,041; 111,266; 114,993; and 118,223.

Generally, methods of alkylation useful in alkylating histidine for use in the present invention are found in Cheung, S.T. et al, Can. J. Chem., Vol 55, pp. 906-910 (1977). However it is now found that in Cheung, S.T. et al, methods it is critical that the reaction conditions for the alkylation of histidine be anhydrous. Further, it is now found also that during work-up instead of adding water directly to the reaction mixture, it is preferred that a buffered aqueous solution be added to the reaction mixture, for example, aqueous sodium or potassium hydrogen sulfate.

Variations in the above description for starting materials, reactants, reaction conditions and required protecting groups to obtain other such N-alkylated compounds are known to an ordinarily skilled chemist or a readily available in the literature.

These peptides may also be prepared by the standard solid phase techniques of Merrifield. Appropriate protecting groups, reagents, and solvents for both the solution and solid phase methods can be found in "The Peptides: Analysis, Synthesis, and Biology," Vols. 1-5, eds. E. Gross and T. Meienhofer, Academic Press, NY, 1979-1983.

Using the procedures described above, and those well known to one of ordinary skill in the art, all of the compounds of the present invention are prepared.

The compounds of the present invention may be in either free form or in protected form at one or more of the remaining (not previously protected) peptide, carboxyl, amino, hydroxy, or other reactive groups. The protecting groups may be any of those known in the polypeptide art. Examples of nitrogen and oxygen protection groups are set forth in T.W. Greene, Protecting Groups in Organic Synthesis, Wiley, New York, (1981); J.F.W. McOmie, ed. Protective Groups in Organic Chemistry, Plenum Press (1973); and J. Fuhrhop and G. Benzlin, Organic Synthesis, Verlag Chemie (1983). Included among the nitrogen protective groups are t-butoxycarbonyl (Boc), benzyloxycarbonyl, acetyl, allyl, phthalyl, benzyl, benzoyl, trityl and the like.

The preferred compounds of this invention are:

N-[[[2-Hydroxy-1,1-bis(hydroxymethyl)ethyl]amino]carbonyl]-Pro-Phe-N(Me)His-LVA-Ile-AMP, pyridine N-oxide;

N-(Glucosaminocarbonyl)-Pro-Phe-N(Me)His-LVA-Ile-AMP;

(Glucosaminocarbonyl)-Pro-Phe-N(Me)His-LVA-Ile-AMP, N-oxide;

L-Histidinamiude, 1-[[(2-deoxy-L-glucitol-2-yl)amino]carbonyl]-L-prolyl-L-phenylalanyl-N-[2-hydroxy-5-methyl-1-(2-methylpropyl)-4-[[[2-methyl-1-[[(2-pyridinylmethyl)amino]carbonyl]butyl]amino]carbonyl]hexyl]-N.alpha.-methyl-, N-oxide;

L-Histidiniamide, 1-[[2-deoxy-D- glucopyranos-2-yl)amino]carbonyl]-L-prolyl-L-tyrosyl-N-[1-(cyclohexylmethyl)-2,3-dihydroxy-5-methylhexyl]-, [1S-(1R*,2S*,3R*)];

L-Histidiniamide, 1-[[2-deoxy-D- glucopyranos-2-yl)amino]carbonyl]-L-prolyl-L-phenylalanyl-N-[1-(cyclohexylmethyl)-2-hydroxy-5-methyl-4-[[(2-methylbutyl)amino]carbonyl]hexyl]-N.alpha.-methyl-, [1S-[1R*,2R*,4R*(R*)]]-.

[$HOCH_2$]$_2$CHNHCO-Pro-Phe-N[$CH_3$]-His-LVA-Ile-AMP;

[$HOCH_2$]$_2$CHNHCO-Pro-Phe-N[$CH_3$]-His-LVA-Ile-AMP[N-oxide];

N-[Glucaminocarbonyl]-Pro-Phe-N[$CH_3$]-His-LVA-Ile-AMP;

N-[S-Methyl-1-thiolincosaminide]carbonyl-Pro-Phe-N[$CH_3$]-His-LVA-Ile-AMP;

N-[2,3,4,5-Tetrahydroxypentyl]-Pro-Phe-N[$CH_3$]-His-LVA-Ile-AMP;

THAM-C(O)-Pro-Phe-N(Me)His-CVA-(2-hydroxypropyl)amino;

DGLU-C(O)-Pro-Phe-N(Me)His-CVA-(2-hydroxypropyl)amino;

THAM-C(O)-Pro-Phe-N(Me)His-CVA-(2,3-dihydroxypropyl)amino;

DGLU-C(P)-Pro-Phe-N(Me)His-CVA-(2,3-dihydroxypropyl)amino;

DGLU-C(O)-Pro-Phe-N(Me)His-CVA-AMP;
DGLU-C(O)-Pro-Phe-N(Me)His-CVA-AMP-NO;
N-[β-N-AcGlc]-α-N-Boc-Asn-Pro-Phe-N-(Me)His-LVA-Ile-AMP;
N-[β-Man]-α-N-Boc-Asn-Pro-Phe-N-(Me)His-LVA-Ile-AMP;
N-[β-N-AcGlc]-α-N-Boc-Asn-Pro-Phe-N-(Me)His-LVA-Ile-AMP-NO;
N-[β-N-AcGlc]-α-Asn-Pro-Phe-N-(Me)His-LVA-Ile-AMP;
N-[β-N-AcGlc]-α-Asn-Pro-Phe-N-(Me)His-LVA-Ile-AMP-NO;
N-[β-N-AcGlc]-α-N-Boc-Asn-Pro-Phe-N-(Me)His-LVA-Ile-NH-. $C(CH_2OH)_3$;
N-[β-N-AcGlc]-C(O)-$(CH_2)_2$-C(O)-Pro-Phe-N-(Me)His-LVA-Ile-AMP;
N-[β-N-AcGlc]-α-N-Boc-D-Asn-Pro-Phe-N-(Me)His-LVA-Ile-AMP; and
N-[β-N-AcGlc]-α-N-Boc-Asn-Pro-Phe-His-Chaψ[$CH_2$NH]Phe-$NH_2$.

The most preferred compounds of this invention are:

B-[[[2-Hydroxy-1,1-bis(hydroxymethyl)ethyl]amino]carbonyl]-Pro-Phe-N(Me)His-LVA-Ile-AMP, pyridine N-oxide;
N-(Glucosaminocarbonyl)-Pro-Phe-N(Me)His-LVA-Ile-AMP;
(Glucosaminocarbonyl)-Pro-Phe-N(Me)His-LVA-Ile-AMP, N-oxide; L-Histidinamiude, 1-[[(2-deoxy-L- glucitol-2-yl)amino]carbonyl]-L-prolyl-L-phenylalanyl-N-[2-hydroxy-5-methyl-1-(2-methylpropyl)-4-[[[2-methyl-1-[[(2-pyridinylmethyl)amino]carbonyl]butyl]amino]carbonyl]hexyl]-N.alpha.-methyl-, N-oxide;
L-Histidinamide, 1-[[2-deoxy-D-glucopyranos-2-yl)amino]carbonyl]-L-prolyl-L-tyrosyl-N-[1-(cyclohexylmethyl)-2,3-dihydroxy-5-methylhexyl]-, [1S-(1R*,2S*,3R*)];
L-Histidinamide, 1-[[2-deoxy-D-glucopyranos-2-yl)amino]carbonyl]-L-prolyl-L-phenylalanyl-N-[1-(cyclohexylmethyl)-2-dihydroxy-5-methyl-4-[[2-methylbutyl)amino]carbonyl]hexyl]-N.alpha.-methyl-, [1S-[1R*,2S*,4R*(R*)]]-;
THAM-C(O)-Pro-Phe-N(Me)His-CVA-(2,3-dihydroxypropyl)amino;
N-[β-N-AcGlc]-α-N-Boc-Asn-Pro-Phe-N-(Me)His-LVA-Ile-AMP; and
N-[β-Man]-α-N-Boc-Asn-Pro-Phe-N-(Me)His-LVA-Ile-AMP.

The determination of renin inhibitory activity of the compounds of the present invention ($IC_{50}$'s) are well known to one of ordinary skill in the art, as, for example, described in published European application 0173481, published 5 March 1986, pages 103-105.

## DESCRIPTION OF THE PREFERRED EMBODIMENTS

The following Preparations and Examples illustrate the present invention.

In the Preparations and Examples below and throughout this document:

Ac is acetyl;
AMP is 2-(aminomethyl)pyridinyl;
AMP-NO is (2-pyridylmethyl)amino, pyridine N-oxide;
Asn is asparagine;
BOC is t-butoxycarbonyl;
BOM is benzyloxymethyl;
Bz is benzyl;
C is centigrade;
Celite is a filter aid;
CVA is Chaψ[CH(0H)$CH_2$]Val;
DCC is dicyclohexylcarbodiimide;
DEPC is diethyl cyanophosphonate or diethylphosphoryl cyanide;
DGLU is (2-deoxy-D-glucopyranos-2-yl)amino;
DGLUA is (2-deoxy-D-glucopyranos-2-yl)amino tetraacetate ester;
DHPA is (±)-(2,3-dihydroxypropyl)amino;
DMF is dimethylformamide;
Et0Ac is ethyl acetate;
FTrp is $N^{in}$-formyl-indolyl;
g is grams;
GEA is 2-(guanidylethyl)amino;
GMPMA is (3-guanidylmethyl)phenyl)methylamino;
His is histidine;
HPLC is high performance liquid chromatography;

$I_2$ is iodine;
Ile is isoleucine;
(im-Tos)His is $N_{im}$-tosyl-L-histidine;
IR is infra red spectra;
L-Selectride is lithium tri-sec-butylborohydride;
LVA is Leuψ[CH(OH)$CH_2$]Val;
Lys is lysine;
M or mol is mole;
MBA is 2-methylbutylamino (racemic or optically active);
MBAS is 2S-methylbutylamino;
Me is methyl;
min is minute;
ml is milliliter;
MS is mass spectroscopy;
NMHis is Nα-methyl-L-histidine;
NMR is nuclear magnetic resonance;
NOAI is (1-naphthyloxy)acetyl;
p-TSA salt is para-toluene sulfonic acid salt;
Ph is phenyl;
Phe is phenylalanine;
Pro is proline;
POA is phenoxyacetyl;
RIP means a compound having the formula H-Pro-His-Phe-His-Phe-Phe-Val-Tyr-Lys-0H.2($CH_3$C(0)0H).X$H_2$0 which is a known renin-inhibiting peptide.
Skellysolve B is as defined in the Merck Index, 10th edition;
t-BDMS or TBDMS is t-butyldimethylsilyl;
TFA is trifluoroacetic acid;
THAM is [2-hydroxy-1,1-bis(hydroxymethyl)ethyl]amino;
THAMOA is (1-methyl-2,6,7-trioxabicyclo[2.2.2]oct-4-yl)amino;
THF is tetrahydrofuran;
TLC is thin layer chromatography;
Tos or TS is p-toluenesulfonyl;
Tr is trityl (triphenylmethyl);
Tyr is tyrosine;
2HPA is (±)-(2-hydroxypropyl)amino;
2HPA(S) is (S)-(2-hydroxypropyl)amino;
UV is ultraviolet; and
Z is benzyloxycarbonyl.

The wedge-shape line indicates a bond which extends above the plane of the paper relative to the plane of the compound thereon.

The dotted line indicates a bond which extends below the plane of the paper relative to the plane of the compound thereon.

## General Procedures

### Procedure A-Boc group removal.

A 5% solution of the Boc protected amine in an equal volume of methylene chloride and trifluoroacetic acid is allowed to stir at room temperature for 1 h and then concentrate in vacuo. A solution of the residue in methylene chloride is washed once with aqueous sodium bicarbonate. The aqueous wash is backwashed twice with methylene chloride. The combined organic fractions are dried over magnesium sulfate and concentrated in vacuo. The residue is then used as is in the next step without further purification.

### Procedure B-Coupling an acid to an amine using diethylphosphoryl cyanide (DEPC).

To a nitrogen covered 0.04 molar solution of the free amine in methylene chloride is added 1.25 equivalents of the acid followed by 1.25 equivalents of triethylamine and 1.4 equivalents of diethylphosphoryl cyanide. The solution is allowed to stir at room temperature for 2-24 h, diluted with methylene chloride, and washed once with aqueous sodium bicarbonate. The aqueous fraction is backwashed twice with methylene chloride. The organic fractions are combined, dried over magnesium sulfate, and concentrated in vacuo. The residue is then chromatographed over silica gel to yield the coupled product.

Procedure C-Coupling an acid to an amine using diethylphosphoryl cyanide.

To a nitrogen covered 0.04 molar solution of the acid in methylene chloride is added 1.25 equivalents of the amine followed by 1.25 equivalents of triethylamine and 1.4 equivalents of diethylphosphoryl cyanide. The solution is allowed to stir at room temperature for 2-24 h, diluted with methylene chloride and washed once with aqueous sodium bicarbonate. The organic fractions are combined, dried over magnesium sulfate, and concentrated in vacuo. The residue is then chromatographed over silica gel to yield the coupled product.

EXPERIMENTAL SECTION

Preparation 1 1-Methyl-4-nitro-2,6,7-trioxabicyclo[2.2.2]octane (Formula A-3) Refer to Chart A.

Following the general procedure of Yokoyama et al., Macromolecules 15:11 (1982), a solution of 52 g of triethyl orthoacetate in 160 ml of dioctyl phthalate is treated with 48.32 g of 2-(hydroxymethyl)-2-nitro-1,3-propanediol, added with stirring in one portion. The resulting homogeneous mixture is heated at 120 °C for 2.5 h, during which time 27 ml of ethanol is removed by short path distillation. The mixture is then cooled to room temperature, treated with 200 mg of p-toluenesulfonic acid in 80 ml of dioctyl phthalate, and finally divided into two equal portions, each in a 1000 ml round-bottomed flask. Each half is subjected to sublimation in a Kugelrohr apparatus at 150 °C, 0.2 mm for 3 h (most of the material is sublimed during the first two hours). The combined sublimed material is recrystallized from acetone/hexane, thereby affording 17 g of the title product at white crystals with m.p. 124-125 °C. An additional 5-6 g of product would be available, if desired, via chromatography of the mother liquors from the above crystallization.

Physical characteristics are as follows:

NMR ($\delta$, $CDCl_3$, TMS): 4.42, 1.50.

$^{13}C$-NMR ($\delta$, $CDCl_3$, TMS): 110.23, 67.72, 61.22, 22.08.

TLC (Silica Gel GF): $R_f$ 0.81 (1% triethylamine/ethyl acetate); $R_f$ 0.82 [5%(4M ammonia-methanol)/95% chloroform].

Preparation 2 1-Methyl-2,6,7-trioxabicyclo[2.2.2]octan-4-amine (Formula A-4) Refer to Chart A.

A solution of 17 g of the title product of Preparation 1 in 140 ml of dioxane is hydrogenated over 600 mg of platinum oxide at 50 psi for 2.5 h. The mixture is filtered through Celite in an argon atmosphere, and the catalyst is washed with additional dioxane. Following removal of the dioxane on the rotary evaporator, the residue is recrystallized from ethyl acetate. The crystalline title product, after drying in a vacuum desiccator for 18 h, weighs 10.2 g and exhibits m.p. 105-109 °C.

The mother liquors from the crystallization are chromatographed on a column containing 200 g of silica gel. The column is packed and eluted with 25 ml fractions of 5% (4M ammonia-methanol)/95% chloroform. Combination of fractions 15-25 affords an additional 1.72 g of pure title product (total yield, 11.92 g).

Physical characteristics are as follows:

NMR ($\delta$, $CDCl_3$, TMS): 3.89, 1.46.

$^{13}C$-NMR ($\delta$, $CDCl_3$, TMS): 108.26, 72.65, 44.92, 22.87.

Infrared ($\nu_{max}$, Nujol, $cm^{-1}$): 3465, 3373, 1446, 1408, 1392, 1354, 1297, 1124, 1053, 1012, 994, 983, 864, 860, 816, 760.

Mass spectrum (C.I.): Peaks at m/z 146 (base peak, $M+H^+$), 164. E.I. gives no molecular ion, but peaks at m/z 132, 115, 90, 73, 54, 43.

TLC (Silica Gel GF): $R_f$ 0.27 (1% triethylamine/ethyl acetate); $R_f$ 0.36 [5% (4M ammonia-methanol)/95%

chloroform].

Preparation 3 1-Methyl-4-(phenoxycarbonylamino)-2,6,7-trioxabicyclo[2.2.2]octane (Formula A-5) Refer to Chart A.

A 250 ml, 3-necked, round-bottomed flask equipped with an addition funnel and an air stirrer is charged with 100 ml of dry pyridine. With rapid stirring, 6.85 ml (8.55 g) of phenyl chloroformate is added dropwise over 5 min. The resulting thick (but stirrable) suspension is stirred for 10 min, then treated dropwise over 5 min with a solution of 2.0 g of the title product of Preparation 2 in 10 ml of pyridine. After an additional 15 min, the reaction mixture is cooled in an ice bath and treated with 25 ml of water (the first 5 ml of which is added slowly -- gas evolution). The mixture is stirred for 15 min, then poured into half-saturated aqueous sodium bicarbonate and extracted 3 times with 100 ml of 1:1 ethyl acetate/hexane. The extracts are washed with additional aqueous sodium bicarbonate and brine, dried over anhydrous sodium sulfate, and evaporated. A vacuum pump is employed at the end to remove small amounts of pyridine.

The crude product, a white solid, is recrystallized from approximately 25 ml of 30% ethyl acetate/hexane and dried at 25° C for 3 h, thereby affording pure title product, 2.62 g, m.p. 186-188° C.

The mother liquors from this recrystallization are chromatographed on a 200 g column of silica gel. The column is packed and eluted in 18 ml fractions with 35% ethyl acetate/hexane (the material is introduced onto the top of the column with chloroform). Fractions 26-40 yield an additional 665 mg of clean title product (total yield is 3.285 g).

Physical characteristics are as follows:

NMR (δ, $CDCl_3$, TMS): 7.55-7.04, 4.96, 4.22, 1.47.

Mass spectrum (FAB): $[M+H]^+$ observed: 266.1029; other peaks at m/z 224, 190, 172, 148, 95, 77, 43.

TLC (Silica Gel GF): $R_f$ 0.40 (35% ethyl acetate/hexane).

Example 1 N-(1-Methyl-2,6,7-trioxabicyclo[2.2.2. ]oct-4-yl-aminocarbonyl)-Pro-Phe-N(Me)His-LVA-Ile-AMP (Formula A-6) Refer to Chart A.

A. N-Deprotection

A solution containing 3.0 g of Boc-Pro-Phe-N(Me)His-LVA-Ile-AMP, 15 ml of trifluoroacetic acid and 15 ml of methylene chloride is allowed to stand at room temperature for 2.5 h under an atmosphere of argon. The reaction mixture is then added dropwise over about 5-10 min, at room temperature, to a rapidly stirred mixture of 125 ml of water, 18.2 g of solid sodium bicarbonate and 70 ml of methylene chloride. The mixture is then transferred to a separatory funnel and extracted with two additional 100 ml portions of methylene chloride. The combined extracts are dried over anhydrous sodium sulfate and evaporated to dryness. The crude N-deprotected product (3.8 g; apparently retained some solvent) is homogeneous by TLC and is used in subsequent reactions without purification. [$R_f$ 0.13; 8% (4M ammonia-methanol)/92% chloroform; Boc-Pro-Phe-N(Me)His-LVA-Ile-AMP exhibited $R_f$ 0.29 on the same plate].

B. Reaction with the title product of Preparation 3.

The N-deprotected product from Section A is dissolved in 35 ml of dioxane which has been dried immediately prior to use by percolation through 100 g of Woelm activity I alumina. The stirred solution is treated with 0.5 ml of triethylamine and 1.71 g of the title product of Preparation 3, and the resulting homogeneous solution is stirred under an argon atmosphere at 50° C for 20 h. Following removal of the dioxane on a rotary evaporator (30° C bath temperature) the residue is diluted with 150 ml of chloroform and washed with 70 ml of saturated aqueous sodium bicarbonate. The aqueous layer is then extracted with three 50 ml portions of chloroform. The extracts are finally dried over anhydrous sodium sulfate and concentrated in vacuo.

The crude product is chromatographed on a column containing 400 g of 40-60 µl silica gel. The column is packed and eluted in 25 ml fractions with 4.5% [4M ammonia-methanol)/95% chloroform. Fractions 132-160, homogeneous by TLC, are combined and afford 2.40 g of pure urea derivative A-6 (Refer to Chart A). In this case, the purified product is to be deprotected in a subsequent step and is therefore allowed to

remain a colorless foam (after drying 1 h at 30° C). In an earlier small scale run, the chromatographically pure product is precipitated by dripping a concentrated methylene chloride solution into an excess of hexane with stirring. Filtration and drying afford an amorphous, free-flowing white solid.

Physical characteristics are as follows:

NMR (δ, $CDCl_3$, TMS): 4.18, 1.49. Diagnostic for the tricyclic orthoester portion. The rest of the spectrum closely resembles that of Boc-Pro-Phe-N(Me)His-LVA-Ile-AMP.

Mass spectrum (FAB): $[M+H]^+$ observed at m/z 1001.586; other ions observed at m/z 567, 539, 388, 269, 241, 172, 124, 109, 86, 70.

TLC (Silica Gel GF): $R_f$ 0.29 [10% (4M ammonia-methanol)/90% chloroform; Boc-Pro-Phe-N(Me)His-LVA-Ile-AMP exhibited $R_f$ 0.40 on the same plate].

Example 2 N-[[[2-Hydroxy-1,1-bis(hydroxymethyl)ethyl]amino]carbonyl]-Pro-Phe-N(Me)His-LVA-Ile-AMP (Formula A-7) Refer to Chart A.

A 0.1 M methanolic hydrochloric acid solution is prepared by adding 0.78 ml of acetyl chloride, with stirring, to 100 ml of dry methanol at 0° C. This solution is used to dissolve 2.40 g of the title product of Example 1, and the resulting mixture is stirred for 2 h at 25° C. TLC analysis of the reaction mixture (spotted directly without dilution or workup) shows clean and complete conversion to the title product. The mixture is treated with 12 ml of 1 M aqueous potassium hydroxide (final pH 13.2) and then stirred 30 min longer. The desired title product is then isolated by extraction with chloroform (after first removing the methanol in vacuo).

The crude title product is chromatographed on a column containing 180 g of silica gel. The column is packed and eluted in 18 ml fractions with 12% 4 M ammonia-methanol/88% chloroform. Fractions 38-60 are combined and evaporated to dryness. The residue is dissolved in 30 ml of warm methylene chloride, and the solution is dripped into 100 ml of rapidly stirred hexane. Filtration and drying (0.1 mm, 25° C, 2 h) affords 2.036 g of pure title product as a white, easily-handled, amorphous solid.

Physical characteristics are as follows:

NMR (δ, DMSO, TMS): 3.51 ppm; otherwise essentially the same as de-Boc'd Boc-Pro-Phe-N(Me)His-LVA-Ile-AMP.

Mass spectrum (FAB): $[M+H]^+$ observed at m/z 977.5827. Other ions at m/z 586, 543, 245, 222, 124, 109, 70.

TLC (Silica Gel GF): $R_f$ 0.26 [15% (4 M ammonia-methanol)/85% chloroform.

During some runs of this sequence, smaller amounts of N-[4,4-Bis(hydroxymethyl)-4,5-dihydrooxazol-2-yl]-Pro-Phe-N(Me)His-LVA-Ile-AMP, the compound of formula IV, were also isolated.

Physical characteristics for IV:

Mass spectrum (FAB): $[M+H]^+$ observed at 959; other ions at m/z 542, 497, 374, 346, 227, 199, 167, 151, 124, 109, 86, 70, 45.

TLC (Silica Gel GF): $R_f$ 0.33 [16% (7M ammonia-methanol)/84% chloroform]; title compound of Example 2 has $R_f$ 0.27 on the same plate.

$IC_{50}$: $1.0 \times 10^{-9}$ M.

Example 3 Boc-Pro-Phe-N(Me)His-LVA-Ile-AMP, pyridine N-oxide (Formula B-2) Refer to Chart B.

A stirred solution of 3.0 g of the B-1 compound (Refer to Chart B), prepared as described in U.S. patent application, Serial No. 825,250, field 3 February 1986, in 90 ml of Burdick and Jackson chloroform is treated with 725 mg of m-chloroperbenzoic acid (assumed 80% purity; therefore 580 mg), and the resulting clear solution is stirred in the dark for 4 h at 25° C. The reaction mixture is then poured into 100 ml of brine/100 ml of saturated sodium bicarbonate and extracted with five 100 ml portions of chloroform. (The first two extractions should be taken only gently to avoid emulsion problems). The extracts are washed with additional aqueous sodium bicarbonate and brine, dried over anhydrous sodium sulfate and evaporated.

The crude product (4.5 g) is dissolved in 60 ml of methanol and treated with 2.2 g of 1-hydroxybenzotriazole hydrate, and the resulting clear solution is stirred for 12 h at room temperature under argon. The mixture is then evaporated to dryness and the residue is chromatographed immediately on a column containing 450 g of silica gel, packed and eluted once with 1000 ml 6% (4 M ammonia-methanol)/94% chloroform, then 25 ml fractions.

Fractions 88-120 are homogeneous by TLC and are combined. Following evaporation of the solvent, the

residue is dissolved in 8 ml of methylene chloride, and that solution is added dropwise to 50 ml of rapidly stirred hexane. After filtration and drying (0.1 mm, 25° C, 2 h), the resulting white solid, clean title product weighs 2.25 g.

Physical characteristics are as follows:

NMR (δ, $CDCl_3$, TMS): 8.3-8.2.

Mass spectrum (FAB): $[M+H]^+$ observed at m/z 946.5756; other ions at m/z 930, 822, 440, 365, 217, 124.

TLC (Silica Gel GF): $R_f$ 0.33 [12% (4 M ammonia-methanol)/88% chloroform.

Example 4 N-[[[2-Hydroxy-1,1-bis(hydroxymethyl)ethyl]amino]carbonyl]-Pro-Phe-N(Me)His-LVA-Ile-AMP, pyridine N-oxide (Formula B-4) Refer to Chart B.

A. Boc Removal

A mixture of 5.0 g of the B-1 compound (Refer to Chart B) in 15 ml of trifluoroacetic acid and 15 ml of methylene chloride is allowed to stand at 25° C for 4 h under nitrogen. The reaction mixture is then added dropwise over 5 min to a stirred mixture of 125 ml of water, 18.2 g of sodium bicarbonate and 70 ml of methylene chloride. The mixture is then transferred to a separatory funnel, and the aqueous layer is extracted with two additional 100 ml portions of methylene chloride. The extracts are dried over sodium sulfate and concentrated in vacuo, thereby affording the de-Boc'd product which is completely clean by TLC. ($R_f$ 0.36 (10% 4 M ammonia-methanol)/90% chloroform).

B. Formation of the B-3 Compound (Refer to Chart B)

A solution of the crude product from Section A above in 20 ml of dioxane is treated with 0.7 ml of triethylamine and 2.44 g of phenoxyurethane A-5, and the resulting mixture is stirred under nitrogen at 50° C for 18 h. (At 6 h, the reaction mixture is homogeneous and yellow; by 18 h, a thick precipitate has appeared). Following removal of the dioxane on the rotary evaporator, the residue is partitioned between aqueous sodium bicarbonate and chloroform. The organic extracts are washed with brine, dried over anhydrous sodium sulfate, and concentrated. The crude product is used directly in Section C without purification. ($R_f$ 0.14 (3% 4 M ammonia-methanol)/97% chloroform).

C. MCPBA oxidation

A solution of the crude product from Section B (above in 125 ml of Burdick and Jackson chloroform is treated with 1.25 g of m-chloroperbenzoic acid (80% purity, 1.0 g), and the resulting clear solution is stirred for 1.5 h at 25° C. The reaction mixture is then poured into aqueous sodium bicarbonate and extracted with chloroform. The extracts are washed with aqueous sodium bicarbonate and brine, dried over anhydrous sodium sulfate, and evaporated to dryness.

The crude product is chromatographed on a 400 g column of silica gel, packed and eluted once with 700 ml 4% (4 M ammonia-methanol)/96% chloroform, then 25 ml fractions. Fractions 72-78 contains 450 mg of intermediate Compound B-3 (Refer to Chart B), contaminated with 5% of a less polar impurity. Fractions 79-125 yield 3.5 g of clean B-3 compound, while fractions 126-140 gives 800 mg of a more polar material. (For the major product, the 3.5 g above, $R_f$ 0.28; 6% (4 M ammonia-methanol)/94% chloroform).

D. Detosylation, orthoester hydrolysis

A solution of 3.5 g of intermediate Compound B-3 (Refer to Chart B) from Section C above in 100 ml of 0.1 M methanolic hydrochloric acid is allowed to stand at 25° C for 4 h. Then 5 ml of water is added, followed by 12 ml of aqueous 1 M potassium hydroxide, and the methanol is removed on the rotary evaporator. Solid sodium chloride is added until the aqueous residue is completely saturated, and the product is isolated via extraction seven times with chloroform (100 ml). Following drying and evaporation of the extracts, the crude product (4.2 g) is chromatographed on a column containing 400 g of silica gel, packed and eluted once with 750 ml of 12% (4 M ammonia-methanol)/88% chloroform, then 25 ml fractions.

Fractions 92-180 are combined and concentrated in vacuo. A 100 ml portion of chloroform is added and evaporated to ensure complete removal of the methanol. The resulting white foam is dissolved in 20 ml of hot chloroform and added dropwise to 70 ml of rapidly stirred hexane. After 20 min. the solids are filtered off, washed with additional 20/70 chloroform/hexane and dried (25° C, 0.05 mm, 16 h), thereby affording 2.14 g of pure THAM urea, the title product.

The side-cut fractions from Section C above (1.25 g) are carried separately through the same Section D hydrolysis procedure and afford an additional 560 mg of pure title product, identical by TLC, NMR and RP-HPLC to the 2.14 g from the preceding paragraph. Thus, the total overall yield of pure title product is 2.70 g.

Physical characteristics are as follows:

NMR (δ, $CD_3OD$, TMS): 8.40-8.33, 3.75.

Mass spectrum (FAB): $[M+H]^+$ observed at m/z 993.5823; other ions at m/z 977, 869, 543, 365, 299, 245, 217, 124, 70.

TLC (Silica Gel GF): $R_f$ 0.19 (15% (4 M ammonia-methanol)/85% chloroform).

HPLC: retention time 17.3 min; 10 x 250 mm, Altex Ultrasphere ODS 5 µl solumn, 2 ml/min, detector 254 nm, solvent 90 methanol/10 pH 3 phosphate buffer. Injected 2 µl of a 10 mg/ml solution with detector range 0.05 aufs.

During some runs of the Example 4 sequence, smaller amounts of N-[4,4-Bis(hydroxymethyl)-4,5-dihydrooxazol-2-yl]-Pro-Phe-N(Me)His-LVA-Ile-AMP, pyridine N-oxide, the compound of formula V, were also isolated.

Physical characteristics of V:

Mass spectrum (FAB): $[M+H]^+$ observed at m/z 975; other ions at 541, 497, 346, 227, 199, 167, 124, 70.

TLC (Silica Gel GF): $R_f$ 0.25 [16% (7M ammonia-methanol)/84% chloroform]; title compound of Example 4 has $R_f$ 0.19 on the same plate.

$IC_{50}$: 1.1 x $10^{-9}$ M.

Preparation 4 Boc-Ile, THAM amide (Formula C-2) Refer to Chart C.

A mixture containing 12 g of Boc-Ile (hemihydrate), 7.0 g of tris(hydroxymethyl)aminomethane (THAM), 7.1 g of 1-hydroxybenzotria zole hydrate, 10.85 g of dicyclohexylcarbodiimide, and 15 g of crushed 3A molecular sieves in 500 ml of methylene chloride is stirred vigorously (air stirrer) over a 64 h weekend at 25° C. The suspension is then cooled to 0° C and filtered through a medium-porosity sintered glass funnel, and the filtrate is concentrated in vacuo. The residue (21 g) is taken up in chloroform and chromatographed on a column containing 1.2 kg of silica gel. The column is packed and eluted once with 1800 ml of 4% methanol/chloroform, then 250 ml fractions.

Fractions 13-15 yields 6.0 g of a 2:1 adduct of Boc-Ile-OH and THAM - i.e., from ester formation between one of the hydroxyls of the title product and a second molecule of Boc-Ile-OH [Mass spectrum (FAB): $(M+H)^+$ observed at m/z 548.3629].

Fractions 27-37 from the above chromatogram affords 3.08 g of the desired THAM amide title product as a white solid. The amide is homogeneous by TLC and is used in subsequent steps without recrystallization.

Physical characteristics are as follows:

NMR (δ, $CDCl_3$, TMS): 7.1, 5.10-5.05, 4.10, 3.90, 3.70, 1.46.

Mass spectrum (FAB): $[M+H]^+$ observed at m/z 335.2157; other ions at m/z 317, 279, 261, 222, 122, 86, 57.

TLC (Silica Gel GF): $R_f$ 0.31 (8% methanol/chloroform).

Recrystallization of a 10 mg sample from chloroform affords material with m.p. 126-128° C.

Preparation 5 Boc-Leuψ[CH(OTBS)$CH_2$]Val-Ile, THAM amide (Formula C-3: R is H) refer to Chart C.

A solution of 80 mg of THAM amide (the title product of Preparation 4) in 5 ml of 1:1 trifluoroacetic acid and methylene chloride is allowed to stand for 1 h at 25° C. Following evaporation to dryness on the rotary evaporator, the material is divided in half and each dissolved in 2 ml of dry methylene chloride.

To one-half of the de-Boc'd product is added 45 mg of Boc-Leuψ[CH(OTBS)$CH_2$]Val-OH, followed by 15 µl of triethylamine and 16 µl of diethylphosphoryl cyanide. After 18 h at 25° C, the reaction mixture is poured into aqueous sodium bicarbonate and extracted with chloroform. The extracts are washed with brine,

dried over anhydrous sodium sulfate and evaporated to dryness.

The other half of the de-Boc'd product (in 2 ml of methylene chloride) is treated with 45 mg of the silylated LVA intermediate, 22 mg of dicyclohexylcarbodiimide and 14.2 mg of 1-hydroxybenzotriazole hydrate. The resulting solution is stirred for 18 h at 25 °C and then filtered to remove the dicyclohexylurea. The filtrate is diluted with more methylene chloride and washed with aqueous sodium bicarbonate. The extracts are dried (sodium sulfate) and concentrated to an oil.

TLC analysis of each of these reactions (6% methanol/chloroform) show that both form the same major product, with the DEPC (diethylphosphoryl cyanide) reaction being cleaner. Since neither reaction contains difficult-to-remove impurities, they are combined and chromatographed on 22 g of silica gel. The column is packed and eluted once with 22 ml of 5% methanol/chloroform, then 1.5-2.0 ml fractions.

Fractions 36-58 are combined and yield 64 mg of clean title product.

Physical characteristics are as follows:

NMR ($\delta$, $CDCl_3$, TMS): 6.95, 6.1-6.0, 4.57-4.47, 4.25-3.50, 1.44, 0.90, 0.09.

Mass spectrum (FAB): $[M+H]^+$ observed at m/z 662.4790; other ions at m/z 562, 475, 328, 231, 196, 112, 86, 73, 57.

TLC (Silica Gel GF): $R_f$ 0.41 (8% (4 M ammonia-methanol)/92% chloroform).

Example 5 Boc-Pro-Phe-His-LVA-Ile, THAM amide (Formula C-4: R is H) Refer to Chart C.

A. Boc Removal

A solution of 60 mg of amide triol intermediate (the title product of Preparation 5) in 5 ml of 1:1 trifluoroacetic acid and methylene chloride is allowed to stand at 25 °C for 1 h. The mixture is then concentrated in vacuo, and the residue is placed under high vacuum for 15 min.

B. Coupling with Boc-Pro-Phe-His(Ts)-OH

The residue from Section A above is dissolved in 1 ml of methylene chloride and added to a suspension of 69 mg of Boc-Pro-Phe-His(Ts)-OH in 1 ml of methylene chloride. The stirred suspension is treated with 20 $\mu$l of triethylamine, followed by 18 $\mu$l of diethylphosphoryl cyanide, and the resulting homogeneous solution is stirred for 20 h at 25 °C. The mixture is then diluted with methylene chloride, poured into aqueous sodium bicarbonate, and extracted with three 80 ml portions of methylene chloride. The extracts are dried (sodium sulfate) and evaporated, and the crude product is chromatographed on a 21 g column of silica gel. The column is packed and eluted once with 25 ml of 5% methanol/chloroform, then 1-2 ml fractions.

Fractions 32-46 are combined and yield 36 mg of pure product, $R_f$ 0.33 (8% (4 M ammonia-methanol)-/92% chloroform).

C. Desilylation/Detosylation

A solution of 36 mg of the Section B product in 0.67 ml of tetrahydrofuran is treated with 0.33 ml of 1 M tetrabutylammonium fluoride in tetrahydrofuran. After 6 h at 25 °C, and additional 0.33 ml of tetrabutylammonium fluoride solution is added. After a total reaction time of 12 h, the mixture is diluted with chloroform, poured into brine containing a little sodium bicarbonate and extracted three times with chloroform (80 ml). The organic layers are dried (sodium sulfate) and evaporated, and the crude product (600 mg) is chromatographed on a 22 g column of silica gel. The column is packed with 8% (4 M ammonia-methanol)-/92% chloroform and eluted with 250 ml of the same solvent (2.5 ml fractions 1-100), followed by 100 ml of 10% (4 M ammonia-methanol)/90% chloroform (2 ml fractions, 100-150) and finally 250 ml of 13% (4 M ammonia-methanol)/87% chloroform (5 ml fractions, 151-200). Fractions 170-195 are combined and yield 35 mg of an oil, containing the title product as well as some reagent-derived garbage which streaks on the TLC plate.

The partially purified product is subjected to preparative TCL on a single 500 $\mu$l 8x8 inch plate. The material is spotted onto the plate in chloroform, and the plate is developed with 10% (4 M ammonia-methanol)/90% chloroform and visualized under UV light. The product is recovered by stirring the scraped-

off silica band with 20 ml of 20% (4 M ammonia-methanol)/80% chloroform. Filtration and evaporation of the solvent yield a white foam weighing 7 mg. The product is dissolved in 100 µl of methylene chloride and precipitated by addition of 2 ml of hexane. Filtration and drying afford 6 mg of an amorphous white powder, the desired THAM amide title product, completely clean by TLC.

Physical characteristics are as follows:
Mass spectrum (FAB): $[M+H]^+$ observed at m/z 929.5674; other ions at 911, 808, 568, 499, 242, 184, 142, 120, 110, 86, 70, 57.
TLC (Silica Gel GF): $R_f$ 0.33 (15% (4 M ammonia-methanol)/85% chloroform).

Example 6 Boc-Leuψ[CH(OTBS)$CH_2$]Val-Ile, THAM amide, triacetate (Formula C-3: R is acetyl) Refer to Chart C.

A. Acetylation

A stirred 5° C solution of 2.59 g of the title product of Preparation 4 in 15 ml of pyridine is treated with 7.5 ml of acetic anhydride and 80 mg of 4-dimethylaminopyridine, and the resulting homogeneous mixture is stirred for 1 h at 25° C under argon atmosphere. The reaction mixture is then cooled to 0° C, treated with 7 ml of methanol, and stirred for 30 min at 0° C and 30 min at 25° C. The mixture is poured into 1:1 brine/water and extracted with ethyl acetate. The extracts are washed with cold 0.5 M hydrochlorid acid, water, sodium bicarbonate and brine, dried over sodium sulfate and evaporated. The crude product (3.6 g) is completely clean by TLC and is used in the next step without purification. ($R_f$ 0.45 (1:1 ethyl acetate/hexane).

B. Boc Removal

The crude Section A product is allowed to stand in 10 ml of methylene chloride and 7 ml of trifluoroacetic acid for 2 h. The mixture is then evaporated to dryness on the rotary evaporator. Two 50 ml portions of chloroform are added and evaporated to co-distill the last traces of trifluoroacetic acid. The crude amine is used directly in Section C below.

C. Coupling with LVA Intermediate

A solution of the Section B product in 60 ml of methylene chloride is cooled to 0° C and treated with 3.45 g of Boc-Leuψ-[CH(OTBS)$CH_2$]Val-OH, followed by 1.13 ml of triethylamine and 1.24 ml of diethylphosphoryl cyanide, the latter reagent being added dropwise over about 5 min. The clear reaction mixture is stirred at 0° C for 1 h and 25° C for 18 h. It is then poured into aqueous sodium bicarbonate, and the product is isolated by extraction with methylene chloride. The extracts are dried (sodium sulfate) and evaporated, and the crude product (8.1 g) is chromatographed on 700 g of silica gel. The column is packed and eluted once with 800 ml of 45% ethyl acetate/hexane, then 25 ml fractions.

Fractions 52-115 are combined and yield 5.0 g of pure triacetate title product.

Physical characteristics are as follows:
NMR (δ, $CDCl_3$, TMS): 6.30, 6.06-5.96, 4.55-4.10, 3.80-3.45, 2.10, 1.45.
Mass spectrum (FAB): $[M+H]^+$ observed at m/z 788.5117; other ions at m/z 714, 688, 601, 444, 328, 248, 213, 196, 112, 86, 73, 57.
TLC (Silica Gel GF): $R_f$ 0.25 (10% acetone/methylene chloride); $R_f$ 0.50 (12% iPrOH/hexane); $R_f$ 0.42 (30% acetone/hexane); $R_f$ 0.53 (50% ethyl acetate/hexane).

Example 6 Boc-Pro-Phe-His-LVA-Ile, THAM amide, triacetate (Formula C-4: R is acetyl) Refer to Chart C.

A 105 mg sample of triacetate intermediate (the title product of Preparation 6) is de-Boc'd exactly as described earlier in Example 5 using 5 ml of 1:1 trifluoroacetic acid/methylene chloride.

The crude amine is coupled with Boc-Pro-Phe-His(Ts)OH (92 mg) using 22 µl of triethylamine and 22 µl of diethylphosphoryl cyanide in 2 ml of methylene chloride, exactly as described earlier in Example 5.

The crude product (242 mg) is chromatographed on a column containing 21 g of silica gel. The column is packed once with 25 ml of 67% ethyl acetate/hexane and eluted with 150 ml of the same solvent, then 1.5 ml fractions, followed by 200 ml of 75% ethyl acetate/hexane and 100 ml of pure ethyl acetate. Fractions 170-242 are combined and yield 94 mg of the pure His(TS)-silyl derivative of the title product. ($R_f$ 0.53 (80% ethyl acetate/hexane)).

The purified product from the preceding paragraph is dissolved in 1.5 ml of tetrahydrofuran and treated with 0. ml of 1 M tetrabutylammonium fluoride in tetrahydrofuran (Aldrich). After 6 h, another 0.8 ml of tetrabutylammonium fluoride is added, and stirring is continued for an additional 6 h. The mixture is then poured into brine containing a little sodium bicarbonate and extracted three times with chloroform (80 ml). The extracts are washed with brine, dried over sodium sulfate and concentrated. The crude product (780 mg) is chromatographed on 70 g of silica gel. The column is packed and eluted in 6 ml fractions with 15% methanol/ethyl acetate. Fractions 42-56 are combined and evaporated to dryness, affording 35 mg of a white foam. The foam is dissolved in a minimal amount of methylene chloride and reprecipitated by adding 2 ml of hexane. Filtration and drying (18 h, 25° C, 0.05 mm) affords 25 mg of pure THAM triacetate, the title product, as an amorphous white powder.

Physical characteristics are as follows:

NMR (δ, $CDCl_3$, TMS): 2.10, 1.45.

Mass spectrum (FAB): $[M+H-COCH_3]^+$ observed at m/z 1013; other ions at m/z 995, 499, 351, 196, 120, 110, 86, 70, 57.

TLC (Silica Gel GF): $R_f$ 0.29 [8% (4 M ammonia-methanol)/92% chloroform]; $R_f$ 0.45 (15/85 methanol/ethyl acetate).

Preparation 7 Boc-N(Me)His(Ts)-Leuψ[CH(OTBS)$CH_2$]Val-Ile, THAM amide, triacetate (Formula D-2: R is acetyl) Refer to Chart D.

Removal of the Boc from the title product of Preparation 6 (5.0 g) is accomplished as described in earlier experiments using 10 ml og trifluoroacetic acid and 10 ml of methylene chloride at 25° C for 2.5 h. Following the usual sodium bicarbonate workup, the crude amine is dissolved in 60 ml of methylene chloride and treated with 4.03 g of Boc-N(Me)His(Ts)OH . dicyclohexylamine salt, 0.93 ml of triethylamine and 1.01 ml of diethylphosphoryl cyanide, the latter reagent is added to the stirred reaction mixture at 25° C via syringe over 15 min. TLC analysis of an aliquot 30 min after the addition is complete indicates that the coupling is finished. The mixture is diluted with methylene chloride, washed with aqueous sodium bicarbonate, dried (magnesium sulfate), and concentrated in vacuo.

The crude product (8.1 g) is chromatographed on a column containing 700 g of silica gel. The column is packed with 65% ethyl acetate/hexane and eluted with 4000 ml of the same solvent, (one 450 ml portion, then 50 ml fractions), followed by 3000 ml of pure ethyl acetate.

Fractions 28-54 are combined and give 5.5 g of pure title product ($R_f$ 0.74 (ethyl acetate); $R_f$ 0.20 (65% ethyl acetate/hexane).

Fractions of 95-130 affords 0.95 g of the product resulting from desilylation of the LVA hydroxyl group during the Boc removal (i.e., the title product with Leuψ[CH(OH)$CH_2$]Val-. ($R_f$ 0.30 (ethyl acetate); $R_f$ 0.02 (65% ethyl acetate/hexane)).

Preparation 8 Boc-Phe-N(Me)His(Ts)-LVA-Ile, THAM amide, triacetate (Formula D-3) Refer to Chart D.

The silyl derivative (the title product of Preparation 7 (R is acetyl)) is de-Boc'd (and simultaneously desilylated) using 25 ml of trifluoroacetic acid and 20 ml of methylene chloride at 25° C for 4.5 h. In similar fashion, the alcohol, the title product of Preparation 7 (R is H), (0.95 g, also isolated in the preceding experiment) is treated with 5 ml of trifluoroacetic acid and 5 ml of methylene chloride at 25° C for 4.5 h. Since both of these deprotection reactions affords the same product (the de-Boc'd amine corresponding to LVA intermediate (the title product of Preparation 7 (R is acetyl)), they are combined and added slowly to a stirred mixture of 252 ml of water, 36 g of sodium bicarbonate and 132 ml of methylene chloride. Further extractions with methylene chloride, drying the extracts and concentration in vacuo affords the crude amine, which is about 90% pure by TLC with ethyl acetate.

The crude amine is dissolved in 60 ml of methylene chloride and treated with 1.67 ml of triethylamine, 3.18 g of Boc-Phe, and 1.82 ml of diethylphosphoryl cyanide added dropwise at 0° C. The mixture is stirred for 1 h at 0° C, then over a weekend at 25° C. Standard methylene chloride/sodium bicarbonate workup

affords a crude product (10.1 g), which is chromatographed on a 700 g column of silica gel. The column is packed and eluted once with 800 ml of 90/10 ethyl acetate/hexane, then 50 ml fractions.

Fractions 45-70 are combined and afford 3.0 g of the title product, completely homogeneous by TLC ($R_f$ 0.50 (ethyl acetate)).

Preparation 9 Boc-Pro-Phe-N(Me)His(Ts)-LVA-Ile, THAM amide, triacetate (Formula D-4) Refer to Chart D.

Deprotection of the title product of Preparation 8 is accomplished using the standard procedure (3.0 g of the title product of Preparation 8, 10 ml of trifluoroacetic acid, 10 ml of methylene chloride at 25° C for 3 h).

The crude amine is dissolved in 30 ml of methylene chloride and treated with 572 mg of Boc-Pro, 0.37 ml of triethylamine and 0.4 ml of diethylphosphoryl cyanide. After 1 h at 25° C, TLC indicates that about 20% of the unreacted amine remains, and an additional 172 mg of Boc-Pro, 0.11 ml of triethylamine and 0.12 ml of diethylhosphoryl cyanide are added. After 16 h at 25° C, the reaction mixture is poured into aqueous sodium bicarbonate and extracted with methylene chloride. The extracts are dried over magnesium sulfate and evaporated, and the crude product (4.5 g) is chromatographed on 450 g of silica gel. The column is packed and eluted in 35 ml fractions with 45/55 acetone/hexane. Fractions 50-68 are combined and afford 2.30 g of pure title product ($R_f$ 0.24 (45/55 acetone/hexane)).

Example 7 Boc-Pro-Phe-N(Me)His-LVA-Ile, THAM amide, triacetate (Formula D-5: R is acetyl) Refer to Chart D.

A solution of 400 mg of the title product of Preparation 9 and 200 mg of 1-hydroxybenzotriazole hydrate in 10 ml of methanol is stirred at 25° C for 1 h. The methanol is removed on the rotary evaporator, and the crude product is chromatographed on an 80 g column of silica gel. The column is packed and eluted with 4 ml fractions of 4.5% (0.4 M ammonia-methanol)/95.5% chloroform.

Fractions 66-118 are combined (using chloroform, not the chromatography solvent), and the residue after evaporation is dissolved in 1 ml of methylene chloride and dripped slowly into 25 ml of stirred hexane. Filtration and drying (25° C, 0.1 mm, 64 h) affords 250 mg of pure title product as an amorphous white solid.

Physical characteristics are as follows:

NMR (δ, $CDCl_3$, TMS): 4.50-4.30, 2.08, 1.46.

Mass spectrum (FAB): $[M+H]^+$ observed at m/z 10.69.623; other ions at m/z 1053, 1027, 496, 365, 124, 95, 86, 70, 57, 43.

TLC (Silica Gel GF): $R_f$ 0.46 (8% 4 M ammonia-methanol)/92% chloroform).

Example 8 Boc-Pro-Phe-N(Me)His-LVA-Ile, THAM amide, (Formula D-5: R is H) Refer to Chart D.

A stirred solution of 1.9 g of triacetate (the title product of Preparation 9) in 50 ml of methanol is treated with 10 ml of water and 10 ml of 1 M aqueous potassium hydroxide, and the resulting clear solution is stirred at 25° C for 1 h under nitrogen. By TLC, the reaction is complete at this time. Most of the methanol is removed in vacuo, and the aqueous residue is saturated with solid sodium chloride and extracted five times with methylene chloride (100 ml) and two times with chloroform (100 ml). The combined organic layer is dried over magnesium sulfate and concentrated.

The crude product is chromatographed on 180 g of silica gel. The column is packed and eluted with 15 ml fractions with 12% (4 M ammonia-methanol)/88% chloroform. Fractions 33-60 are combined and evaporation. The residue is dissolved in 5 ml of methylene chloride, and the solution is added dropwise to 70 ml of stirred hexane. Filtration and drying (0.1 mm, 25° C, 14 h) affords 1.27 g of pure THAM amide, the title product, as an easily-handled amorphous white solid.

Physical characteristics are as follows:

NMR (δ, $CDCl_3$, TMS): 3.80-3.60, 1.45.

Mass spectrum (FAB): $[M+H]^+$ observed at m/z 943.5856; other ions at m/z 365, 217, 124, 95, 86, 70, 57.

TLC (Silica Gel GF): $R_f$ 0.28 [15% (4 M ammonia-methanol)/85% chloroform].

Preparation 10 N-(Phenoxycarbonyl)-D-glucosamine, tetra-O-acetate (Formula E-2) Refer to Chart E.

Following a procedure based on that of Chargoff and Bovarnick, J. Biol. Chem., 118:421 (1937), 5.25 g of solid sodium bicarbonate is added over 2 min (carbon dioxide evolution) to a stirred solution of 5.4 g of D-glucosamine hydrochloride (Aldrich) in 25 ml of water. To this stirred mixture is added 4.7 ml of phenyl chloroformate (about 0.4 ml every five min). By the eighth addition, the mixture is very difficult to stir and control of foaming also becomes a problem. At this point the reaction mixture is diluted with 75 ml of water and transferred to a 400 ml beaker. The use of an air stirrer then solves both the stirring and foam control problems. The suspension is stirred at 25° C for 10 min after the last of the phenyl chloroformate has been added. The solids are isolated by filtration through a medium porosity sintered glass funnel and washed two times with acetone (10 ml) and methanol (10 ml). The crude phenyl carbamate, after drying for 16 h at 25° C and 0.1 mm, weighs 6.0 g, and is used immediately in the next step without further purification or characterization.

A solution of 5.32 g of the crude phenyl carbamate in 30 ml of pyridine and 10 ml of acetic anhydride is stirred at 25° C for 3 h. The mixture is then cooled in an ice bath, treated with 5 ml of methanol, and stirred for 1 h at 0° C and 1 h at 25° C. The mixture is poured into ice and water containing 52 g of sodium bisulfate and extracted with 1:1 ethyl acetate/hexane. The extracts are washed with water, aqueous sodium bicarbonate and brine, dried (sodium sulfate), and evaporated.

The crude title product (10.4 g) is chromatographed on a 700 g column of silica gel, packed and eluted once with 2000 ml of 50% ethyl acetate/hexane, then 230 ml fractions. Fractions 4-12 are combined, and upon evaporation of the solvent, the product is readily crystallized. The product is triturated with 25 ml of ether, then diluted with 15 ml of pentane and filtered. The solids are washed with 10 ml of 1:1 ether/pentane and dried at 25° C, 0.05 mm for 3 h, thereby affording 7.48 g of pure phenyl carbamate tetracetate, the title product, with m.p. 146-148° C.

Physical characteristics are as follows:

NMR (δ, $CDCl_3$, TMS): 7.45-7.08, 6.35, 5.45-5.13, 4.40-3.80, 2.30-2.10.

Infrared: $\nu_{max}$ (mull, $cm^{-1}$) 3314, 1753, 1712, 1542, 1450, 1224, 1204, 1137, 1046, 1016, 1006, 922.

Mass spectrum (FAB): $[M+H]^+$ observed at m/z 468; other ions at m/z 408, 366, 288, 246, 228, 216, 194, 152, 141, 110, 95, 43.

Anal. Found: C, 53.89; H, 5.41; N, 2.85.

Example 9 N-(Glucosaminocarbonyl)-Pro-Phe-N(Me)His-LVA-Ile-AMP (Formula E-3: R is H) Refer to Chart E.

A solution of 0.215 mmole of de-Boc'd Boc-Pro-Phe-N(Me)His-LVA-Ile-AMP (prepared as described in Example 1) in 2.5 ml of dry dioxane is treated with 200 mg of the title product of Preparation 10 and 0.04 ml of triethylamine. After the reaction mixture has been stirred for 18 h at 50° C, TLC analysis (10% (4 M ammonia-methanol)/90% chloroform) indicates that the reaction is proceeding cleanly to a single spot faster moving than de-Boc'd Boc-Pro-Phe-N(Me)His-LVA-Ile-AMP, but that the reaction is only about 40% finished. An additional 100 mg of the title product of Preparation 10 is added, and stirring at 50° C is continued for another 24 h. The reaction mixture is then poured into aqueous sodium bicarbonate and the product is isolated via extraction with chloroform.

The crude product is chromatographed on a column containing 20 g of silica gel, packed and eluted in 2.5 ml fractions with 6% (4 M ammonia-methanol)/94% chloroform. Fractions 38-66 are combined and yield 210 mg of clean tetraacetate, the title product (R is acetyl). ($R_f$ 0.36; 10% (4 M ammonia-methanol)/90% chloroform).

The tetraacetate (210 mg) is dissolved in 5 ml of methanol and treated with 1 ml of 4 M ammonia in methanol, and the resulting yellow solution is stirred under nitrogen for 18 h at 25° C. The reaction mixture is then evaporated to dryness and the crude product (160 mg; >95% pure by TLC) is chromatographed on 21 g of silica gel. The column is packed with 15% (4 M ammonia-methanol)/85% chloroform and eluted with 100 ml of 20%, followed by 200 ml of 25% (4 M ammonia-methanol)/75% chloroform (1 x 25 ml, then 2.5 ml fractions).

Fractions 77-105, homogeneous by TLC, are combined and evaporated to dryness. The residue is dissolved in 1 ml of 10% methanol/chloroform, and the solution is dripped slowly into 25 ml of stirred hexane. The solids are filtered and dried (0.1 mm, 25° C, 3 h), thereby affording 95 mg of pure glucosamine urea derivative, the title product (R is H), as an amorphous white powder.

Physical characteristics are as follows:

Mass spectrum (FAB): $[M+H]^+$ observed at m/z 1035.583; other ions at m/z 1017, 586, 365, 243, 222, 124, 109, 95, 86, 70, 55.
TLC (Silica Gel GF): $R_f$ 0.37 (30% (4 M ammonia-methanol)/70% chloroform).
$IC_{50}$: 4.9 x $10^{-10}$ M.

Example 10 (Glucosaminocarbonyl)-Pro-Phe-N(Me)His-LVA-Ile-AMP, N-oxide.

A. BOC Removal:

A solution of 3.2 of Boc-Pro-Phe-N(Me)His-LVA-Ile-AMP, prepared as described in U.S. patent application, Serial No. 147,073 filed 20 January 1988, and in published European patent application 0173481, in 10 ml of methylene chloride is treated with 5.5 ml of trifluoroacetic acid, and the resulting, clear, light yellow solution is stirred at 25° C for 1 h. The reaction mixture is then diluted with 125 ml of methylene chloride and added dropwise over 30 min to a vigorously stirred mixture of 500 ml of water, 8 g of solid sodium bicarbonate and 200 ml of methylene chloride. The aqueous layer is separated and extracted with eight 100 ml portions of methylene chloride. The combined organic layers are dried over anhydrous sodium sulfate and evaporated to dryness. The resulting colorless, amorphous solid (2.9 g) is 95-97% pure by TLC and is used immediately in part B below.

Physical characteristics are as follows:
TLC Silica Gel GF: $Rf_f$ 0.31 (10% 4M ammonia-methanol/90% chloroform; starting material exhibited $R_f$ 0.54 on the same plate).

B. Activated glucosamine coupling:

A stirred solution of the part A product, 3.21 g of N-phenoxycarbonyl-D-glucosamine, tetra-0-acetate and 0.7 ml of triethylamine in 40 ml of dioxane is heated at 50° C for 64 h under nitrogen. The dioxane is then removed at reduced pressure (vacuum pump), and the residue is partitioned between aqueous sodium bicarbonate and chloroform. The aqueous layer is extracted with two additional 100 ml portions of chloroform. Removal of the chloroform at reduced pressure affords the coupled product, containing no unreacted starting peptide and contaminated only by the excess reagent.

Physical characteristics are as follows:
TLC Silica Gel GF: $R_f$ 0.54 (10% 4M ammonia-methanol/90% chloroform: starting material exhibited $R_f$ 0.31 and the tetra-acetate reagent, $R_f$ 0.85 on the same plate).

C. Pyridine N-oxide formation:

A solution of the part B product in 75 ml of chloroform is treated with 853 mg of 80-85% pure m-chloroperbenzoic acid (approximately 15% excess) added in one portion. After 1 h at 25° C, the reaction mixture is treated with excess 10% aqueous sodium sulfite, transferred to a separatory funnel, and extracted with several 100 ml portions of chloroform. The extracts are dried over anhydrous sodium sulfate and evaporated, and the crude product (7.1 g) is chromatographed on a 450 g column of silica gel, packed and eluted (25 ml fractions) with 8% 4M ammonia-methanol/92% chloroform. Fractions 76-94 are combined and yield 2.2 g of the tetra-0-acetate corresponding to the title product. By TLC, the material collected from the chromatogram is slightly more polar than the material that is put onto the column. The post-chromatography material may be either an anomeric mixture or, more likely, the result of partial deacylation (especially at the primary alcohol) by the ammonia and methanol in the chromatography solvent.

D. Acetate removal:

A solution of 2.2 g of the part C product in 50 ml of methanol is treated with 10 ml of 4M ammonia-methanol, and the resulting solution is stirred for 18 h at 25 V. (The solution darkens considerably during this time). Removal of the methanol in vacuo affords a dark residue (2.5 g) which is chromatographed on 200 g of silica gel. The column is packed and eluted (20 ml fractions) with 25% 4M ammonia-methanol/75%

chloroform.

Based on their TLC homogeneity, fractions 58-105 are combined and evaporated to dryness. The residue is dissolved in 6 ml of 10% methanol/chloroform and reprecipitated by the rapid addition of 150 ml of pentane. (In subsequent runs, the methanol/chloroform solution should be added to the pentane. This avoids the formation of large aggregates and yields a more uniform, finely-divided product). The solids are filtered and dried in a vacuum desiccator (25°, 6 h, 0.05 mm), thereby affording 1.15 g of the title product. (A 50 mg portion of the product that was subjected to more vigorous drying -- 48 h, 70°, 0.05 mm -- developed four less polar impurities totaling 1-5%).

Physical characteristics are as follows:

Mass spectrum (FAB): $[M+H]^+$ observed at m/z 1051.584; other ions at m/z 846, 602, 365, 124.

TLC (Silica Gel GF): $R_f$ 0.14 (25% 10M ammonia-methanol/75% chloroform); the title product of Example 9 exhibited $R_f$ 0.20 on the same plate.

$IC_{50}$: 1.1 x $10^{-9}$ M.

Example 11 L-Histidinamide, 1-[[(2-deoxy-L-glucitol-2-yl)amino]carbonyl]-L-prolyl-L-phenylalanyl-N-[2-hydroxy-5-methyl-1-(2-methylpropyl)-4-[[[2-methyl-1-[[(2-pyridinylmethyl)amino]carbonyl]butyl]amino]-carbonyl]hexyl]-N.alpha.-methyl-, N-oxide.

A solution of 110 mg of the title product of Example 10 in 8 ml of absolute ethanol is treated with 110 mg (large excess) of sodium borohydride, added in one portion. The mixture is stirred in a nitrogen atmosphere for 12 h at 25° C. The mixture is then cooled to 0° C and acidified carefully with 1M methanolic hydrochloric acid. The mixture is concentrated to dryness on the rotary evaporator, and the residue is taken up in methanol. Ammonia-methanol (approximately 8M) is added until the mixture is basic. The mixture is filtered, and the solids (which by TLC were shown to contain essentially none of the desired product) are discarded. Evaporation of the filtrate affords 215 mg of an amorphous solid.

Attempted chromatographic purification of this material on silica gel was unsuccessful. Even with a very polar eluting solvent (50% 8M ammonia-methanol/50% chloroform, the material elutes in a very broad band.

The recovered material from the silica chromatography is purified on a 10 g column of 50-100 µ $C_{18}$ reverse phase silica gel (Waters). The column is eluted with 65/35 methanol/water (75 drop fractions). Fractions 28-56 are combined and lyophilized (after first removing the methanol on the rotary evaporator), thereby affording 37 mg of the title product as an amorphous solid.

Physical characteristics are as follows:

Mass Spectrum (FAB): $[M+H]^+$ observed at m/e 1053.5942. Other ions observed at m/e 929, 603, 527, 478, 452, 365, 305, 196, 124, 109, 86, 70.

TLC (Silica Gel GF): $R_f$ 0.35 (30% 7M ammonia-methanol/70% chloroform; starting material exhibited $R_f$ 0.42 on the same plate).

TLC ($C_{18}$ RP Silica Gel): $R_f$ 0.34 (65/35 methanol/water).

Preparation 11 1R and 1S-[3-tert-Butyloxycarbonyl-4S-cyclohexylmethyl-2,2-dimethyl-5R-oxoazolidinyl]-3-methyl-2-butene-1-ol (Formula H-2 and H-3) Refer to Chart H.

To a stirred mixture of 0.54 g of magnesium turnings and a crystal of iodine in 10 ml of dry tetrahydrofuran under argon in an oil bath at 80° C is slowly added a solution of 2.84 g of 1-bromo-2-methylpropene in 5 ml of tetrahydrofuran. After 3 h, the reaction mixture is allowed to cool and then is stirred in an ice bath. A solution of 4.6 of 3-tert-butyloxycarbonyl-4S-cyclohexylmethyl-2,2′-dimethyl-5R-oxazolidinyl carboxaldehyde (H-1) in 10 ml of tetrahydrofuran is slowly added. After 1 h, the reaction mixture is allowed to warm to room temperature and stirred overnight. The mixture is treated with saturated aqueous ammonium chloride and then extracted with dichloromethane. The organic phase is dried (magnesium sulfate) and then concentrated. The residue is flash chromatographed on silica gel with 10%-15% ethyl acetate in hexane to give 1.34 g of the title product H-2 and 2.88 g of the title product H-3.

Physical characteristics of H-2 are as follows:

$^1H$-NMR: δ 1.47, 1.55, 1.60, 1.74, 1.75, 3.65, 4.25, 5.05.

IR (mull): 3469, 1702 $cm^{-1}$.

$[\alpha]_D$ = +2° (c = 1.022, chloroform).

MS: Found, 381.2888.

Anal. Found: C, 69.27; H, 10.34; N, 3.95.

Physical characteristics of H-3 are as follows:
$^1$H-NMR: δ 1.48, 1.57, 1.62, 1.73, 1.78, 3.8, 4.0, 4.3, 5.3.
IR (neat): 3480, 1700, 1683 $cm^{-1}$.
$[\alpha]_D$ = +11° (c = 0.843, chloroform).
MS: Found, 381.2863.
Anal. Found: C, 69.07; H, 9.96; N, 4.08.

Preparation 12 1S-[3-tert-Butyloxycarbonyl-4S-cyclohexylmethyl-2,2-dimethyl-5R-oxazolidinyl]-3-methyl-1-butanol (Formula H-4) Refer to Chart H.

To a solution of 2.88 g of compound H-3 of Preparation 11 in 20 ml of ethyl acetate is added 0.15 g of 5% platinum on charcoal. This mixture is shaken on a Parr shaker under 50 psi of hydrogen for 5 h. The resulting mixture is filtered through Celite with ethyl acetate washings and filtrate is concentrated to give 2.5 g of the title product.

Physical characteristics are as follows:
$^1$H-NMR: δ 0.92, 0.97, 1.48, 1.52, 1.55, 3.6, 4.05.
IR (mull): 3498, 1680 $cm^{-1}$.
$[\alpha]_D$ = +1° (c = 0.978, chloroform).
MS: Found, 383.3017.
Anal. Found: C, 68.52; H, 10.73; N, 3.91.

Preparation 13 2S-Amino-1-cyclohexyl-3R,4S-dihydroxy-6-methylheptane (Formula H-5) Refer to Chart H.

A solution of 2.5 g of compound H-4 of Preparation 12 in 15 ml of 1M hydrochloric acid in methanol is allowed to stir at room temperature for 3 h. Small portions of 2.5 g of solid sodium bicarbonate is slowly added. After stirring for 15 min, the mixture is diluted with dichloromethane and then filtered through Celite with dichloromethane washings. The filtrate is concentrated and the resulting residue is flash chormatographed on silica gel with 5%-10% methanol (saturated with gaseous ammonia) in dichloromethane to give 1.3 g of the title product.

Physical characteristics are as follows:
$^1$H-NMR: δ 0.92, 0.97, 3.05, 3.25, 3.8.
IR (mull): 3349, 3324, 3282 $cm^{-1}$.
$[\alpha]_D$ = -23° (c = 0.891, chloroform).
MS: Found, 244.2268.
Anal. Found: C, 69.45; H, 12.15; N, 5.99.

Preparation 14 N-(1-Methyl-2,6,7-trioxabicyclo[2.2.2]oct-4-yl-aminocarbonyl)-L-prolyl-L-phenylalanyl-$N^{\alpha}$-methyl-$N^{im}$-tosyl-L-histidyl-2S-amino-1-cyclohexyl-3R,4S-dihydroxy-6-methylheptane. Refer to Chart I (Formula I-3)

A solution of 133 mg of the free amine I-2 and 83 mg of the urethane I-1 with 0.06 ml of diisopropylethylamine in 0.4 ml of dioxane is heated in an oil bath at 50°C for 15 h. The concentrated reaction mixture is chromatographed on silica gel with 3%-5% methanol in dichloromethane to give 148 mg of the title product.

Physical characteristics are as follows:
$^1$H-NMR spectrum of which was consistent with the structure.

Example 12 L-Histidinamide, 1-[[[2-hydroxy-1,1-bis(hydroxymethyl)ethyl]amino]carbonyl]-L-prolyl-L-phenylalanyl-N-[1(cyclohexylmethyl)-2,3-dihydroxy-5-methylhexyl]-N.alpha.-methyl-, [1S-(1R*,2S*,3R*)-]- or THAM-Pro-Phe-N-MeHis-CVG (Formula I-4) Refer to Chart I.

To a stirred solution of 148 mg of compound I-3 of Preparation 14 in 2.7 ml of methanol is added 0.3 ml of 1M hydrochloric acid in methanol. After stirring for 3 h, 0.45 ml of 1M aqueous sodium hydroxide is added. After a few min, 1 ml of saturated aqueous sodium chloride is added and methanol is removed on a

rotary evaporator. The residue is partitioned between aqueous sodium chloride and dichloromethane. The organic phase is dried (magnesium sulfate) and then concentrated. The residue is chromatographed on silica gel with 10%-15% methanol (saturated with ammonia) in dichloromethane to give 85 mg of the title product.

Physical characteristics are as follows:

$IC_{50}$ (nM): 0.26.

$k'$ (partition ratio) (90:10 = methanol: Aqueous pH3 phosphate buffer, 1.5 ml/min) 5.55.

FAB-MS: Found, 786.4772.

Preparation 15 N-(1,3,4,6-Tetra-0-acetyl-D-glucosaminocarbonyl)-L-proline, benzylester. Refer to Chart J (Formula J-3).

A solution of 0.935 g of compound J-1, 0.484 g of proline benzyl ester hydrochloride and 0.7 ml of diisopropylethylamine in 2 ml of dioxane is stirred in an oil bath at 70 °C for 3 h. The reaction mixture is chromatographed on silica gel with 50%-70% ethyl acetate in hexane to give 0.84 g of the title product.

Physical characteristics are as follows:

$^1H$-NMR spectrum of which was consistent with the structure.

Preparation 16 N-(1,3,4,6-Tetra-0-acetyl-D-glucosaminocarbonyl)- L-proline. Refer to Chart J (Formula J-4).

A mixture of 0.55 g of compound J-3 and 50 mg of 5% palladium on charcoal in 10 ml of ethyl acetate is shaken under 50 psi of hydrogen for 3 h. The mixture is then filtered through Celite with ethyl acetate washings. The filtrate is concentrated to give 0.34 g of the title product.

Physical characteristics are as follows:

$^1H$-NMR spectrum of which was consistent with the structure.

Preparation 17 N-(1,3,4,6-Tetra-0-acetyl-D-glucosaminocarbonyl)-L-prolyl-L-phenylalanyl-N-α-methyl-$N^{im}$-tosyl-L-histidyl-2S-amino-1-cyclohexyl-3R,4S-dihydroxy-6-methylheptane. Refer to Chart K (Formula K-3).

To a stirred solution of 52.3 mg of the amine K-2 and 48 mg of the acid K-1 in 1 ml of dichloromethane is added 0.035 ml of diisopropylethylamine, followed by 0.015 ml of diethylphosphoryl cyanide. After stirring overnight, the concentrated reaction mixture is chromatographed on silica gel with 2%-4% methanol in dichloromethane to give 85 mg of two anomers of the title product.

Example 13 L-Histadinamide, 1-[[(2-deoxy-D-glucopyranos-2-yl)amino]carbonyl]-L-prolyl-L-phenylalanyl-N-[1-(cyclohexylmethyl)-2,3-dihydroxy-5-methylhexyl]-N.alpha.-methyl-, [1S-(1R*,2S*,3R(*)]]- or Glu-Pro-Phe-N-MeHis-CVG (Formula K-4) Refer to Chart K.

A solution of 85 mg of compound K-3 of Preparation 17 in 2 ml of methanol (saturated with ammonia) is allowed to stand overnight. The concentrated reaction mixture is then chromatographed on silica gel ith 5%-20% methanol (saturated with ammonia) in dichloromethane to give 56 mg of the title product.

Physical characteristics are as follows:

$k'$ (partition ratio) (90:10 = Methanol:Aqueous pH3 phosphate buffer, 1.5 ml/min): 4.60, 5.0.

FAB-MS Found: 844.4819.

$IC_{50}$ (nM): 2.4.

Example 14 L-Histidinamide, 1-[[[2-hydroxy-1,1-bis(hydroxymethyl)ethyl]amino]carbonyl]-L-prolyl-0-methyl-L-tyrosyl-N-[1-(cyclohexylmethyl)-2,3-dihydroxy-5-methylhexyl]-, [1S-(1R*,2S*,3R*)]- or THAM-Pro- Tyr-($OCH_3$)-His-CVG.

This compound is prepared using procedures analogous to those for the title product of Example 12.

Physical characteristics are as follows:

$k'$ (partition ratio)(90:10 = Methanol:Aqueous pH3 phosphate buffer, 1.5 ml/min): 5.53.

FAB-MS Found: 802.4706.
$IC_{50}$ (nM): 0.21.

Example 15 L-Histadinamde, 1-[[(2-deoxy-D-glucopyranos-2-yl)amino]carbonyl]-L-prolyl-L-tyrosyl-N-[1-(cyclohexylmethyl)-2,3-dihydroxy-5-methylhexyl]-, [1S-(1R*,2S*,3R*)]- or Glu-Pro-Tyr($OCH_3$)-His-CVG.

This compound is prepared using procedures analogous to those described for the title product of Example 13.

Physical characteristics are as follows:
k′ (partition ratio)(90:10 = Methanol:Aqueous pH3 phosphate buffer, 1.5 ml/min): 4.55, 5.04.
FAB-MS Found: 860.4778.
$IC_{50}$ (nM): 2.3.

Example 16 L-Histidinamide, N-[[[2-hydroxy-1,1-bis(hydroxymethyl)ethyl]amino]carbonyl]-L-phenylalanyl-N-[2-hydroxy-5-methyl-1-(2-methylpropyl)-4-[[[2-methyl-1-[[(2-pyridinylmethyl)amino]carbonyl]butyl]amino]-carbonyl]hexyl]-N.alpha.-methyl-, [1S-[1R*,2R*,4R*(1R*,2R*)]]-.

A solution of 40 mg of N-(1-methyl-2,6,7-trioxabicyclo[-2.2.2]oct-4-yl-aminocarbonyl)-0-phenyl, prepared as described in Preparation 3 (Formula A-5), 60 mg of H-Phe-N(Me)-His-LVA-Ile-AMP and 0.03 ml of diisopropylethylamine in 0.3 ml of dioxane is heated at 50 °C for 20 h. The concentrated mixture is then chromatographed on silica gel with 5% methanol (saturated with ammonia) in dichloromethane to give 30 mg of a solid. This is stirred in 0.1M hydrochloric acid in methanol for 1 h, and then neutralized with ammonia. Passage through silica gel with 20% methanol (saturated with ammonia) in dichloromethane gives 10 mg of the title product.

Physical characteristics are as follows:
HPLC: Retention time 9.1 min (90:10 = Methanol:Aqueous phosphate pH3 detector 225 nm).
HRMS Found: 902.5098.
$IC_{50}$: 1.8 x $10^{-8}$ M.

Example 17 L-Histidinamide, 1-[[(2-deoxy-D-glucopyranos-2-yl)amino]carbonyl]-L-prolyl-L-phenylalanyl-N-[1-(cyclohexylmethyl)-2-hydroxy-5-methyl-4-[[(2-methylbutyl)amino]carbonyl]hexyl]-N.alpha.-methyl-, [1S-[1R*,2R*,4R*(R*)]]- or glu-Pro-Phe-N-MeHis-CVA-Mba.

To a stirred solution of 165 mg of N-(1,3,4,6-Tetra-0-acetyl-D-glucosaminocarbonyl)-L-proline prepared as described in Preparation 16 (Formula J-4) and 243 mg of H-Phe-N(Me)-His(Ts)-CVA-Mba in 2 ml of dichloromethane is added 0.06 ml of diisopropylethylamine and 0.05 ml of diethylphosphoryl cyanide. After stirring overnight, the mixture is chromatographed on silica gel with 3% methanol in dichloromethane to give 350 mg. This material is allowed to stand in 3 ml of methanol (saturated with ammonia) overnight. The concentrated mixture is chromatographed on silica gel with 20% methanol (saturated with ammonia) in dichloromethane to give 240 mg of the title product.

Physical characteristics are as follows:
HRMS Found: 941.5727.
$IC_{50}$: 1.8 x $10^{-9}$ M.

Example 18 L-Histidinamide, 1-[[(2-deoxy-D-glucopyranos-2-yl)amino]carbonyl]-L-prolyl-L-phenylalanyl-N-[4-[[[5-amino-1-[[(4-piperidinylmethyl)amino]carbonyl]pentyl]amino]carbonyl]-2-hydroxy-5-methyl-1-(2-methylpropyl)hexyl]-N.alpha.-methyl-, [1S-[1R*,2R*,4R*(R*)]]-, triacetate (salt) or Glu-Pro-Phe-N-MeHis-LVA-Lys-Ampip.

To a stirred solution of 52 mg of N-(1,3,4,6-Tetra-0-acetyl-D-glucosaminocarbonyl)-L-proline, prepared as described in Preparation 16 (Formula J-4) and 72 mg of H-Phe-N(Me)-His(Ts)-LVA-Lys(Z)-NH-$CH_2$-piperidinyl-Z, wherein Z is a protecting group, in 1 ml of dichloromethane is added 0.035 ml of diisopropylethylamine and 0.018 ml of diethylphosphoryl cyanide. After stirring overnight, the mixture is chromatographed on silica gel with 5% methanol in dichloromethane to give 90 mg. This material is allowed

to stand in 2 ml of methanol (saturated with ammonia) overnight. The concentrated residue is stirred in 1 ml of dimethylformamide with 30 mg of 5% palladium on charcoal and 65 mg of ammonium formate overnight. The concentrated mixture is dissolved in acetic acid/dichloromethane and then precipitated with ether to give 30 mg of the title product.

Physical characteristics are as follows:
HPLC: Retention time 11.7 min (aqueous phosphate pH 3, detector 254 nm).
HRMS Found: 1056.647.
$IC_{50}$ (M): 7.0 x $10^{-7}$ M.

Preparation 18 Phe-N[$CH_3$)-His[Ts]-LVA-Ile-AMP (Formula L-2) Refer to Chart L.

To 0.405 g of Boc-Phe-N($CH_3$)-His(Ts)-LVA-Ile-AMP is added 8 ml of 1:1, trifluoroacetic acid : methylene chloride. The resulting solution is stirred at room temperature for 1.75 h and then concentrated under reduced pressure. The residue is diluted with methylene chloride and washed with saturated aqueous sodium bicarbonate. The aqueous wash is reextracted two times with methylene chloride and all of the organic extracts are combined, dried (magnesium sulfate), filtered and concentrated to give the title product. This material is used in Preparation 19 without additional purification.

Preparation 19 [3,4,5-Trihydroxy-1-cyclohexene-1-]-CO-Phe-N[$CH_3$]-His[Ts]-LVA-Ile-AMP (Formula L-3a) Refer to Chart L.

A solution of the white solid prepared in Preparation 18 in 5 ml of methylene chloride and 1 ml of dimethylformamide (DMF) is treated with 90 µl of diisopropylethylamine (DIEA), 86 µl of diethyl cyanophosphonate (DEPC) and 88 mg of shikimic acid (Aldrich). The resulting milky suspension is allowed to stir under a nitrogen atmosphere at room temperature for 24 h (the mixture becomes homogeneous after approximately 45 min). The solution is diluted with methanol and then concentrated under reduced pressure to give the crude product. This material is chromatographed over 50 g of silica gel (63-200µ), eluting with 10% methanol/methylene chloride while collecting 6 ml fractions. Fractions 30-108 are combined and concentrated to give 0.2609 g of the title product.

Physical characteristics are as follows:
Mass spectrum (FAB) found m/e 1043.525; other ions at m/e 889, 609, 519, 278, 124, 109, 91, 86.
TLC $R_f$ = 0.26 in 10% methanol/methylene chloride (visualized using phosphomolybdic acid). The TLC's in the following examples 19-28 and Preparations 20-27 were visualized using the reagent in parentheses.

Example 19 [3,4,5-Trihydroxy-1-cyclohexene-1-]-CO-Phe-N[$CH_3$]-His-LVA-Ile-AMP (Formula L-4a) Refer to Chart L.

A solution of 0.2609 g of the title product of Preparation 19 in 17 ml of tetrahydrofuran (THF) and 3 mL of dimethylformamide (DMF) is treated with 0.34 g of 1-hydroxybenzotriazole (1-HOBt) and the resulting solution is allowed to stir under a nitrogen atmosphere at room temperature for 24 h. The solution is concentrated under reduced pressure to give the crude product. This material is chromatographed over 50 g of silica gel (63-200µ), eluting with 20% methanol/methylene chloride containing 0.15% ammonium hydroxide while collecting 6 mL fractions. Fractions 11-98 are combined and concentrated to give the crude product contaminated with excess 1-HOBt. This material is rechromatographed over 20 g of silica gel (63-200µ), eluting with 20% (4M ammonia/methanol)/chloroform while collecting 6 ml fractions. Fractions 12-28 are combined and concentrated to give 0.1577 g of the title product.

Physical characteristics are as follows:
Mass spectrum (FAB) found m/e 889.5218; other ions at m/e 781, 455, 365, 276, 222, 196, 124, 109.
TLC $R_f$ = 0.32 in 20% (4M ammonia/methanol)/chloroform (sulfuric acid).
$IC_{50}$ (M): 3.9 x $10^{-9}$.

Preparation 20 Phe-N[$CH_3$]-His[Ts]-LVA-Ile-AMP (Formula L-2) Refer to Chart L.

The preparation of this material is repeated as decribed above (same amounts of reagents, with a

reaction time of 3.5 h and following the same workup). This material is used in Preparation 21 without additional purification.

Preparation $[HOCH_2]_2C[CH_3]CO$-Phe-N$[CH_3]$-His[Ts]-LVA-Ile-AMP (Formula L-3b) Refer to Chart L.

A solution of the white solid prepared in Preparation 20 in 5 ml of methylene chloride and 1 ml of dimethylformamide (DMF) is treated with 90 µl of diisopropylethylamine (DIEA), 86 µl of diethyl cyano phosphonate (DEPC) and 68 mg of 2,2-bis(hydroxymethyl)propionic acid (Aldrich). The resulting pale yellow solution is allowed to stir under a nitrogen atmosphere at room temperature for 25 h. The solution is diluted with methanol and then concentrated under reduced pressure to give the crude product. This material is chromatographed over 50 g of silica gel (63-200µ), eluting with 6% methanol/methylene chloride containing 0.5% acetic acid while collecting 6 ml fractions. Fractions 65-100 are combined and concentrated to give 0.0613 g (an additional 0.0287 g of material is later obtained from rechromatography of some prior mixed fractions) of the title product.

Physical characteristics are as follows:

Mass spectrum (FAB), m/e 1003 $[M+H]^+$, 985, 925, 849, 523, 407, 278, 271, 253, 155, 154, 124, 120, 99, 86.

TLC $R_f$ = 0.12 in 6% methanol/methylene chloride with acetic acid present (UV).

Example 20 $[HOCH_2]_2C[CH_3]CO$-Phe-N$[CH_3]$-His-LVA-Ile-AMP (Formula L-4b) Refer to Chart L.

A solution of 0.0613 g of the title product of Preparation 21 in 4 mL of tetrahydrofuran (THF) and 1 mL of dimethylformamide (DMF) is treated with 0.0836 g of 1-hydroxybenzotriazole (1-HOBt) and the resulting solution is allowed to stir under a nitrogen atmosphere at room temperature for 25 h. The solution is concentrated under reduced pressure to give the crude product. This material is chromatographed over 20 g of silica gel (63-200µ), eluting with 12% (4M ammonia/methanol)/chloroform while collecting 6 ml fractions. Fractions 9-17 are combined and concentrated to give 0.0202 g of the title product.

Physical characteristics are as follows:

Mass spectrum (FAB) found m/e 849.5214; other ions at m/e 741, 586, 415, 365, 236, 222, 196, 124, 120, 109.

TLC $R_f$ = 0.33 in 12% (4M ammonia/methanol)/chloroform (sulfuric acid).

$IC_{50}$ (M): 4.6 x $10^{-9}$.

Preparation 22 PhenylOCONHCH$[CH_2OAc]_2$ (Formula M-2) Refer to Chart M.

A 250 mL three-necked round bottom flask, equipped with mechanical stirrer, is charged with a solution of 2.16 g of serinol hydrochloride (Aldrich) dissolved in 50 ml of water. To this vigorously stirred solution is added 3.5 g of solid sodium bicarbonate portion-wise followed by the addition of 3.2 ml of phenylchloroformate (Aldrich) over approximately 3 min via syringe. Continued stirring for approximately 20 min affords a two phase mixture which is then diluted with methanol and concentrated under reduced pressure to give a white solid. This material is suspended in 16 ml of pyridine and 8.0 mL of acetic anhydride and the resulting mixture is stirred at room temperature for 4.5 h. The reaction is quenched with methanol and concentrated under reduced pressure. The residue is then diluted with water, acidified to pH between 3 and 4 (concentrated hydrochloric acid) and extracted two times with ethyl acetate. The organic extracts are washed with 1N hydrochloric acid, saturated aqueous sodium bicarbonate and brine, dried (magnesium sulfate), filtered and concentrated to give 3.52 g of the crude product as a yellow oil. This material is flash chromatographed over a 35 mm (OD) x 26 mm silica gel (40-63µ) column, eluting with 30% ethyl acetate/skellysolve B and collecting 140 mL fractions. Fractions 5-9 are combined and concentrated to give 2.34 g of the title product.

Physical characteristics are as follows:

$^1H$ NMR (300 MHz; $CDCl_3$) δ 7.42-7.10, 5.42, 4.32-4.15, 2.11.

Mass spectrum found m/e 295.1044; other ions at m/e 94, 43.

TLC $R_f$ = 0.28 in 30% ethyl acetate/hexane (phosphomolybdic acid).

Preparation 23 $[AcOCH_2]_2CHNHCO$-Pro-Phe-N$[CH_3]$-His-LVA-Ile-AMP (Formula M-3) Refer to Chart M.

A solution of 0.219 g of Pro-Phe-N$(CH_3)$-His-LVA-Ile-AMP, 0.156 g of the title product of Preparation 22 and 51 µl of triethylamine (Aldrich) all in 5 ml of dimethylformamide (DMF) is stirred at 50° C under a nitrogen atmosphere for 22.5 h at which time TLC analysis indicates the displacement reaction is complete. The solution is concentrated under reduced pressure to remove solvents to give the crude product as a colorless oil.

Physical characteristics are as follows:

TLC $R_f$ = 0.46 in 20% (4M ammonia/methanol)/chloroform (phosphomolybdic acid). This material is used in the next experiment without additional purification.

Example 21 $[HOCH_2]_2CHNHCO$-Pro-Phe-N$[CH_3]$-His-LVA-Ile-AMP (Formula M-4) Refer to Chart M.

The title product of Preparation 23 is dissolved in 5 ml of methanol, treated with 1 ml of 4M ammonia/methanol and stirred at room temperature for 18 h. Solvents are removed under reduced pressure to give the crude product as a colorless oil. This material is chromatographed over 50 g of silica gel (63-200µ), eluting with 15% (4M ammonia/methanol)/chloroform while collecting 6 ml fractions. Fractions 30-58 are combined and concentrated to give 0.2140 g of the desired product.

Physical characteristics are as follows:

Mass spectrum found m/e 947.5743; other ions at m/e 513, 365, 215, 187, 124, 109, 86, 70.

TLC $R_f$ = 0.14 and 0.43 in 10 and 15% (4M ammonia/methanol)/-chloroform, respectively (phosphomolybdic acid).

$IC_{50}$ (M): $3.6 \times 10^{-10}$.

Example 22 $[HOCH_2]_2CHNHCO$-Pro-Phe-N$[CH_3]$-His-LVA-Ile-AMP[N-oxide] (Formula M-5) Refer to Chart M.

The title product of Example 21 (0.100 g) and 0.27 g of metachloroperoxybenzoic acid (MCPBA; Aldrich) all in 5 ml of chloroform is stirred at room temperature under a nitrogen atmosphere for 2.5 h. Because TLC analysis still indicates unreacted starting material, another 0.27 g of MCPBA is added and the solution is stirred an additional 1 h. Solvents are removed under reduced pressure and the residue is chromatographed over 50 g of silica gel (63-200µ), eluting with 15% (4M ammonia/methanol)/chloroform while collecting 6 ml fractions. Fractions 56-115 are combined and concentrated to give 0.0558 g of the title product (fractions 50-55 affords 6.3 mg of impure material).

Physical characteristics are as follows:

Mass spectrum found m/e 963.5683; other ions at m/e 947, 513, 365, 215, 124, 86, 70.

TLC $R_f$ = 0.31 in 15% (4M ammonia/methanol)/chloroform (phosphomolybdic acid).

$IC_{50}$ (M): $8.8 \times 10^{-10}$.

Preparation 24 N-(Phenoxycarbonyl)-D-glucamine-penta-O-acetate (Formula N-2) Refer to Chart N.

A solution of 3.0 g of D-glucamine (Formula N-1) (Huls) and 1.4 g of sodium bicarbonate all in 100 ml of water is cooled to ice bath temperature. To this mechanically stirred solution is added via syringe 3.1 ml of phenylchloroformate (Aldrich) dropwise over 5 min. The ice bath is removed and the resulting turbid solution is vigorously stirred while gradually warming to room temperature over 30 min. Methanol is added to destroy excess chloroformate and the solution is concentrated under reduced pressure to give the crude product [N-(phenoxycarbonyl)-D-glucamine] as a white residue. This solid is suspended in 12 ml of acetic anhydride and 24 ml of pyridine and the resulting mixture is stirred at room temperature under a nitrogen atmosphere for 20 h. The reaction is quenched with methanol and concentrated under reduced pressure. The concentrate is diluted with water, acidified with concentrated hydrochloric acid and extracted two times with ethyl acetate. The organic extracts are washed with 1N hydrochloric acid, saturated aqueous sodium bicarbonate and brine, dried (magnesium sulfate), filtered and concentrated under reduced pressure to give 7.73 g the crude title product as a yellow oil. This material is flash chromatographed over a 50 mm (OD) x 24 cm silica gel (40-63µ) column, eluting with 50% ethyl acetate/hexane and collecting 140 ml fractions. Fractions 7-12 are combined and concentrated to give 6.86 g of the title product.

Physical characteristics are as follows:
$^1H$ NMR (300 MHz; $CDCl_3$) δ 7.41-7.08, 5.57-5.49, 5.47-5.35, 5.23-5.13, 5.10-5.02, 4.34-4.25, 4.17-4.06, 3.66-3.52, 3.50-3.39, 2.14, 2.12, 2.10, 2.05, 2.03.
Mass spectrum found m/e 512.1779; other ions at m/e 452, 410, 392, 376, 358, 316, 274, 230, 136, 95, 94.
TLC $R_f$ = 0.56 in 50% ethyl acetate/hexane (sulfuric acid).

Preparation 25 N-[Penta-O-acetylglucaminocarbonyl]-Pro-Phe-N[$CH_3$]-His-LVA-Ile-AMP (Formula N-3) Refer to Chart N.

A solution of 0.214 g of Pro-Phe-N($CH_3$)-His-LVA-Ile-AMP, 0.195 g of the title product of Preparation 24 and 250 μl of triethylamine (Aldrich) all in 5 ml of dimethylformamide (DMF) is stirred at 50° C under a nitrogen atmosphere for 4 h at which time TLC analysis indicates the displacement reaction is complete. Heating is discontinued and the solution is let to cool and stir an additional 13 h. The solution is then concentrated under reduced pressure to remove DMF to give the crude product as a colorless oil.

Physical characteristics are as follows:
TLC $R_f$ = 0.46 in 10% (4M ammonia/methanol/chloroform (sulfuric acid). This material is used in Example 23 without purification.

Example 23 N-[Glucaminocarbonyl]-Pro-Phe-N[$CH_3$]-His-LVA-Ile-AMP (Formula N-4) Refer to Chart N.

The title product of Preparation 25 is dissolved in 10 ml of methanol, treated with 2 ml of 4M ammonia/methanol and stirred at room temperature for 29 h. Solvents are removed under reduced pressure to give the crude product as a pale yellow oil. This material is chromatographed over 53 g of silica gel (63-200μ), eluting with 70:25:5, chloroform/methanol/ammonium hydroxide while collecting 6 ml fractions. Fractions 24-42 are combined and concentrated to give 0.2282 g of the title product.

Physical characteristics are as follows:
Mass spectrum found: m/e 1037.602; other ions at m/e 603, 586, 452, 365, 305, 124, 109, 86, 70.
TLC $R_f$ = 0.45 in 70:25:5, chloroform/methanol/ammonium hydroxide (sulfuric acid).
HPLC purity = 96%.

Preparation 26 N-(Phenoxycarbonyl)-S-methyl-1-thiolincosaminide-tetra-O-acetate (Formula 0-2) Refer to Chart 0.

A milky suspension of 0.50 g of S-methyl-1-thiolincosaminide (Formula 0-1) (Upjohn) and 0.17 g of sodium bicarbonate, all in 20 ml of water is cooled to ice bath temperature and then treated with phenylchloroformate (0.37 ml) dropwise over 5 min. The resulting mixture is stirred for 15 min, the ice bath is removed and after an additional 20 min of stirring a clear colorless solution has formed. The solution is concentrated under reduced pressure to give the crude N-[phenoxycarbonyl]-S-methyl-1-thiolincosaminide. This material is suspended in 1.5 ml of acetic anhydride and 2.6 ml of pyridine and the resulting mixture is stirred at room temperature under a nitrogen atmosphere for 19 h. The reaction is quenched with methanol and concentrated under reduced pressure. The concentrate is diluted with water, acidified with concentrated hydrochloric acid and extracted two times with ethyl acetate. The organic extracts are washed with IN hydrochloric acid, saturated aqueous sodium bicarbonate and brine, dried (magnesium sulfate), filtered and concentrated under reduced pressure to give 1.03 g of the crude title product as a white foam. This material is flash chromatographed over a 25 mm (OD) x 26 cm silica gel (40-63μ) column, eluting with 30% ethyl acetate/hexane and collecting 100 ml fractions. Fractions 2-6 are combined and concentrated to give 0.78 g of the title product.

Physical characteristics are as follows:
$^1H$ NMR (300 MHz; $CDCl_3$) δ 7.40-7.05, 5.68-5.59, 5.33-5.12, 4.46-4.23, 2.16-2.00, 1.27.
Mass spectrum found m/e 542.1692; other ions at m/e 494, 482, 452, 392, 332, 272, 176, 95.
TLC $R_f$ = 0.69 in 50% ethyl acetate/hexane (sulfuric acid).

Preparation 27 N-[S-Methyl-1-thiolincosaminide-tetra-O-acetate]-carbonyl-Pro-Phe-N[$CH_3$]-His-LVA-Ile-AMP (Formula 0-3) Refer to Chart O.

A solution of 0.200 g of Pro-Phe-N($CH_3$)-His-LVA-Ile-AMP, 0.203 g of the title product of Preparation 26 and 50 μl of triethylamine (Aldrich), all in 5 ml of dimethylformamide (DMF) is stirred at 50° C under a nitrogen atmosphere of 18 h at which time TLC analysis indicates no reaction has occurred. Approximately 0.5 ml of additional triethylamine is added, the temperature is increased to 75° C and stirring is continued for 24 h. After this time TLC analysis indicates no unreacted starting material and the solution is concentrated under reduced pressure to give the crude title product as a colorless oil.

Physical characteristics are as follows:

TLC $R_f$ = 0.49 in 10% (4M ammonia/methanol)/chloroform (sulfuric acid). This material is used in Example 24 without purification.

Example 24 N-[S-Methyl -1- thiolincosaminide]carbonyl-Pro-Phe-N[$CH_3$]-His-LVA-Ile-AMP (Formula 0-4) Refer to Chart 0.

The title product of Preparation 27 is dissolved in 10 ml of methanol, treated with 2 ml of 4M ammonia/methanol and stirred at room temperature for 20 h. Solvents are removed under reduced pressure to give the crude product as a pale yellow oil. This material is chromotographed over 50 g of silica gel (63-200μ), eluting with 13% (4M ammonia/methanol)/chloroform while collecting 5 ml fractions. Fractions 22-38 are combined and concentrated to give 0.1671 g of what appears to be a monoacetylated analog of the desired product. Fractions 56-140 give 0.0997 g of the title product.

Physical characteristics are as follows:

Mass spectrum found: m/e 1109.608; other ions at m/e 675, 377, 365, 329, 124, 109, 86, 70.

TLC $R_f$ = 0.25 in 15% (4M ammonia/methanol)/chloroform (sulfuric acid).

Example 25 N-[2,3,4,5-Tetrahydroxypentyl]-Pro-Phe-N[$CH_3$]-His-LVA-Ile-AMP (Formula Q-2: n is 3; Renin Inhibitor Residue is -Pro-Phe-N(Me)His-LVA-Ile-AMP) Refer to Chart Q.

A solution of 0.200 g of [Pro-Phe-N[$CH_3$]-His-LVA-Ile-AMP], 0.053 g of D-ribose (Aldrich) and 0.31 g of sodium cyanobrorohydride (Aldrich), all in 5 ml of methanol is stirred at room temperature under a nitrogen for 24 h. Concentrated hydrochloric acid is then added (2-3 drops) until the pH is lowered to approximately 2. The resulting solution is stirred for 10 min and then concentrated under reduced pressure to give the crude product as a white solid. This material chromatographed over 50 g of silica gel (63-200μ), eluting with 15% (4M ammonia/methanol)/chloroform) while collecting 6 ml fractions. Fractions 43-103 are combined and concentrated to give.0.1689 g of the title peptide.

Physical characteristics are as follows:

Mass spectrum (FAB) found: m/e 964.5855; other ions at m/e 586, 530, 379, 365, 351, 204, 124, 109.

TLC $R_f$ = 0.25 and 0.41 in 15 and 20% (4M ammonia/methanol)/-chloroform, respectively (sulfuric acid).

HPLC purity = 93%.

Example 26 N-[2,3,4,5,6-Pentahydroxyhexyl]-Pro-Phe-N[$CH_3$]-His-LVA-Ile-AMP (Formula Q-2: n is 4; Renin Inhibitor Residue is Pro-Phe-N(Me)His-LVA-Ile-AMP) Refer to Chart Q.

A solution of 0.200 g of [Pro-Phe-N[$CH_3$]-His-LVA-Ile-AMP], 0.067 g of D-glucose (Aldrich) and 0.31 g of sodium cyanoborohydride (Aldrich), all in 5 ml of methanol is stirred at room temperature under a nitrogen atmosphere for 24 h. Concentrated hydrochloric acid is then added (2-3 drops) until the pH is lowered to approximately 2. The resulting solution is stirred for 10 min and then concentrated under reduced pressure to give the crude product as a white solid. This material is chromatographed over 50 g of silica gel (63-200μ), eluting with 25% (4M ammonia/methanol)/chloroform) while collecting 6 ml fractions. Fractions 23-80 are combined and concentrated to give 0.1891 g of the title peptide.

Physical characteristics are as follows:

TLC $R_f$ = 0.46 elongated spot) in 25% (4M ammonia/methanol)/-chloroform (sulfuric acid).

Example 27 N-D-Gluconoyl-Pro-Phe-N[$CH_3$]-His-LVA-Ile-AMP (Formula Q-3: n is 4; Renin Inhibitor Residue is Pro-Phe-N(Me)His-LVA-Ile-AMP) Refer to Chart Q.

A solution of 0.202 g of Pro-Phe-N[$CH_3$]-His-LVA-Ile-AMP and 0.0567 g of D-glucono-δ-lactone (Sigma), all in 10 ml of methanol is stirred at reflux under a nitrogen atmosphere for 24 h. TLC analysis indicates a substantial amount of unreacted starting peptide. Methanol is removed under reduced pressure and the concentrate is redissolved in 5 ml of DMF. This solution is then stirred at 65° C for two h at which time another 52 mg of starting lactone is added. Stirring is continued for an additional 2 h at 65° C and then at room temperature for 39 h. Another 54 mg of lactone is added and the resulting solution was again heated at 65° C for 4.5 h. This solution is concentrated under reduced pressure to give the crude product as a colorless oil. This material is chromatographed over 50 g of silica gel (63-200μ), eluting with 35% (4M ammonia/methanol)/chloroform) while collecting 6 ml fractions. Fractions 37-55 are combined and concentrated to give the title peptide.

Physical characteristics are as follows:

TLC $R_f$ = 0.37% (70:25:5 chloroform:methanol:conc. ammonium hydroxide) (sulfuric acid).

Example 28 [1S-[1R*,2R*,4R*(R*)]]-1-[[[2-Hydroxy-1,1-bis(hydroxy-methyl)ethyl]imino]carbonyl]-L-prolyl-L-phenylalanyl-N-[1-(cyclohexylmethyl)-2-hydroxy-4-[[(2-hydroxypropyl)imino]carbonyl]-5-methylhexyl]-Nα-methyl-L-histidinamide; or THAM (C(O)ProPheNMHisCVA2HPA(S).

A. Boc(0TBDMS)CVA2HPA(S)

By the general coupling procedure C, described before the Experimental Section, 0.10 g of the acid Boc(0TBDMS)CVA prepared as described in Chart T and Preparations 41-48 is coupled with S-(+)-1-amino-2-propanol and chromatographed over 150 ml of silica gel (elution with 2.5% methanol/methylene chloride) to yield 0.113 g of the coupled product.

Physical characteristics are as follows:

FAB mass spectrum: Found: $[m^\bullet + H]^+$ at m/z 543.

B. Boc(im-Tos)NMHis(0TBDMS)CVA2HPA(S)

To a nitrogen covered ice bath, cooled solution of 2.323 g of the Boc amine prepared as in Part A in 16 ml of methylene chloride is added 16 ml of trifluoroacetic acid dropwise over 11 min. After stirring at room temperature for 1 h, the reaction mixture is added dropwise over 8.5 min to a mixture of 19.2 g of sodium bicarbonate in 130 ml of water and 75 ml of methylene chloride. After mixing well, the aqueous layer is separated and extracted twice with methylene chloride. The combined organic fractions are dried over magnesium sulfate and concentrated in vacuo to yield 2.15 g of the amine free base. The amine is then coupled (coupling procedure B described before the Experimental Section) with Nα-t-butoxycarbonyl-Nα-methyl-Nim-tosyl-L-histidine and chromatographed over a 400 ml silica gel column (elution with 2.5% methanol/methylene chloride containing 0.25% ammonium hydroxide) to yield 2.32 g of the coupled product.

Physical characteristics are as follows:

FAB mass spectrum: Found: $[m^\bullet + H]^+$ at m/z 848.

C. BocProPhe(im-Tos)NMHisCVA-2HPA(S)

By the general procedure A for Boc group removal described before the Experimental Section (reaction time is 2 h), 0.501 g of the Boc amine of Part B yields 0.4484 g of the amine free base. The amine is then coupled (coupling procedure B described before the Experimental Section) with Nα-t-butoxycarbonyl-L-prolyl-L-phenylalamine from Example 29b and chromatographed over a 150 ml silica gel column (elution with 3.5% methanol/methylene chloride containing 0.35% ammonium hydroxide) to yield 0.399 g of the coupled product.

Physical characteristics are as follows:

FAB mass spectrum: Found: $[M^\bullet + H]^+$ at m/z 978.

D. THAMOAC(0)ProPhe(im-Tos)NMHisCVA2HPA(S)

By the general procedure A for Boc group removal described before the Experimental Section (reaction time is 2.5 h), 0.20 g of the Boc amine of Part C yields 0.18 g of the amine free base. To a nitrogen covered solution of the amine in 1.0 ml of dioxane is added 0.031 ml of triethylamine followed by 0.109 g of the title product of Preparation 3. After heating in an oil bath at 50° for 22 h, the solution is allowed to cool and concentrated in vacuo. a solution of the residue in methylene chloride is washed once with aqueous sodium bicarbonate. The aqueous wash is backwashed twice with methylene chloride. The combined organic fractions are dried over magnesium sulfate and concentrated in vacuo. The residue is chromatographed over a 150 ml silica gel column (elution with 4% methanol/methylene chloride containing 0.4% ammonium hydroxide) to yield 0.213 g of the urea.

Physical characteristics are as follows:
FAB mass spectrum: Found: $[m^{\bullet}+H]^{+}$ at m/z 1049.

E. THAMC(O)ProPheNMHisCVA2HPA(S)

To a nitrogen covered solution of 0.1213 g of the protected peptide of Part D is 3.7 ml of methanol is added 0.26 ml of 1.46 M hydrochloric acid in diethyl ether. After stirring for 4 h at room temperature, the solution is concentrated in vacuo. The residue is then concentrated two additional times from methylene chloride and allowed to remain under vacuum for 1 h. To a nitrogen covered solution of the residue in 2.8 ml of methanol is added 0.76 ml of 5.45 M ammonia in methanol. After stirring for 19.5 h at room temperature, the solution is concentrated in vacuo. The residue is chromatographed over a 50 ml silica gel column (elution with 15% methanol/methylene chloride containing 0.5% ammonium hydroxide) to yield first 0.0632 g of the title product, followed by 0.038 g of additional product mixed with some more polar byproduct.

Physical characteristics are as follows:
FAB mass spectrum: Found: $[m^{\bullet}H]^{+}$ at 871.
Measured = 871.5298.
$IC_{50}$: 0.41 X$10^{-9}$ M.

Example 29 [1S-[1R*,2R*,4R*(R*)]]-1-[[(2-Deoxy-D-glucopyranos-2-yl)amino]carbonyl]-L-Prolyl-L-phenylalaryl-N-[1-(cyclohexylmethyl)-2-hydroxy-4-[[(2-hydroxypropyl)-amino]carbonyl]-5-methylhexyl]-Nα-methyl-L-histidinamide; or DGLUC(O)ProPheNMHisCVA2HPA(S).

A. DGLUAC(0)ProPhe(im-Tos)NMHisCVA2HPA(S)

By the general procedure A for Boc group removal described before the Experimental Section (reaction time is 2.2 h), 0.1937 g of the Boc amine (BocProPhe(im-Tos)NMHisCVA2HPA(S) of Part C of Example 28 yields 0.1758 g of the amine free base. To a nitrogen covered solution of 0.1659 of the amine in 3.3 ml of dimethylformamide is added 0.032 ml of triethylamine followed by 0.163 gof the title product of Preparation 10. After stirring in an oil bath at 50° for 17 h, the solution is allowed to cool and concentrated in vacuo at vacuum pump pressure. The residue is chromatographed over a 150 ml silica gel column (elution with 3.5% methanol/methylene chloride containing 0.35% ammonium hydroxide) to yield 0.1675 g of the urea.

Physical characteristics are as follows:
FAB mass spectrum: Found: $[m^{\bullet}+H]^{+}$ at m/z 1251.

B. DGLUC(O)ProPheNMHisCVA2HPA(S)

To a nitrogen covered solution of 0.1675 g of the protected peptide of Part A in 4.3 ml of methanol is added 0.93 ml of 5.45 M ammonia in methanol. After stirring for 18.75 h, the solution is concentrated in vacuo. The residue is chromatographed over a 100 ml silica gel column (elution with 20% methanol/methylene chloride containing 0.5% ammonium hydroxide) to yield 0.0547 g of the title product.

Physical characteristics are as follows:
FAB mass spectrum: Found: $[m^{\bullet}+H]^{+}$ at m/z 929.
Measured = 929.5380.
$IC_{50}$: 1.5 x $10^{-9}$ M.

Example 29a Preparation of 2,3-Bis(tert-Butyldimethylsilyloxy)-propylamine.

A. $ZNHCH_2CH(OH)CH_2OH$

To a nitrogen covered mechanically stirred solution of 5.0 g of 3-amino-1,2-propanediol in 40 ml of water is added 11.63 g of sodium carbonate. The mixture is cooled in an ice bath causing a heavy precipitate to come out of solution. There is then added a solution of 7.83 ml of benzyl chloroformate in 100 ml of methylene chloride over 40 min. After stirring for 3 h, the mixture is allowed to warm to room temperature and there is added 75 ml of water and sufficient methylene chloride to bring the total volume to 450 ml. The aqueous layer is separated and extracted twice with methylene chloride. The organic fractions are combined, dried over magnesium sulfate, and concentrated in vacuo to yield a crystalline residue. Recrystallization from ethyl acetate/hexane yields 3.55 g (m.p. 70.5°-72°), 4.20 g (m.p. 68.5°-71.5°) and 1.06 g (m.p. 67°-70°) of the title product.

B. $ZNHCH_2CH(0TBDMS)CH_20TBDMS$

To a nitrogen covered solution of 1.0 g of the diol of Part A in 25 ml of dimethylformamide is added 1.8 g of imidazole followed by 2.01 g of t-butyldimethylsilyl chloride. After stirring at room temperature for 24 h, the reaction mixture is concentrated in vacuo and the residue partitioned between water and hexane. The aqueous layer is separated and extracted with hexane. The combined organic fractions are washed once with water and brine, dried over magnesium sulfate, and concentrated in vacuo to yield 1.98 g of the di-silyl ether as a gummy residue.

C. $H_2NCH_2CH(0TBDMS)CH_20TBDMS$

A mixture of 1.98 g of the product of Part B and 0.20 g of 10% palladium on carbon catalyst in 150 ml of absolute ethanol is hydrogenated on a Parr hydrogenerator for 2 h. The catalyst is removed by filtration through Celite and the filtrate is concentrated in vacuo. The residue is distilled at 5 mm pressure to yield 1.150 g of the title product.

Physical characteristics are as follows:

B.P.: 114°-116°.

Example 29b Preparation of Nα-t-Butoxycarbonyl-L-prolyl-L-phenyl-alanine.

A. BocPro(OBz)Phe

A mixture of L-phenylalanine, benzyl ester, tosylate (50.2 g) and aqueous sodium bicarbonate is extracted with diethyl ether. The extracts are washed with water, dried with magnesium sulfate and concentrated to give 29.7 g of the free base. A stirred solution of this material in 519 ml of methylene chloride, under nitrogen, is treated sequentially with Boc-L-proline (29.7 g), methylene chloride (519 ml), triethylamine (21.1 ml) and diethylphosphoryl cyanide (23 ml). The mixture is kept at ambient temperature for 1.5 h, mixed with a saturated sodium bicarbonate solution and extracted with methylene chloride. The extracts are washed with water, dried over magnesium sulfate and concentrated to give 55.6 g of the product.

B. BocProPhe

A mixture of the benzyl ester (54.7 g) from Part A, methanol (800 ml) and 10% palladium-on-carbon catalyst (6.08 g) is hydrogen-ated at an initial pressure of 50 psi for 2 h. The mixture is filtered and the filtrate concentrated. A solution of the residue in diethyl ether (500 ml) containing 10 drops of methanol is boiled in the steam bath while adding hexane to keep the volume constant. The product crystallizes; it is collected by filtration and dried in vacuo to give 34.9 g of the product.

Physical characteristics are as follows:
Anal Found: C, 62.63; H, 7.42; N, 7.64.

Example 30 [1R*,2R*,4R*(R*)]-1-[[[2-Hydroxy-1,1-bis(hydroxymethyl)ethyl]amino]carbonyl]-L-prolyl-L-phenylalanyl-N-[1-(cyclohexylmethyl)-4-[[(2,3-dihydroxypropyl)amino]carbonyl]-2-hydroxy-5-methylhexyl]-Nα-methyl-L-histidinamide (Isomer A) THAMC(O)ProPheNMHisCVADHPA (Isomer A).

A. Boc(0TBDMS)CVANHCH$_2$ $\overset{*}{C}$H(0TBDMS)CH$_2$0TBDMS (Isomers A and B)

By the general coupling procedure C described before the Experimental Section, 2.0 g of (Boc-(0TBDMS)CVA) prepared as described in Chart T and Preparations 41-48 is coupled with the product from Preparation 29a and chromatographed over a 400 ml silica gel column (elution with 7% ethyl acetate/hexane) to yield first 0.7693 g of isomer A followed by 1.6336 g of a mixture (AB) and then 0.5513 g of isomer B. The mixture AB is rechromatographed over a 400 ml silica gel column (elution with 7% ethyl acetate/hexane) to yield an additional 0.3698 g of isomer A, 0.6414 g of a mixture and 0.4980 g os isomer B.

Physical characteristics of isomer A are as follows:
FAB mass spectrum: Found: $[m^{\bullet}+H]^{+}$ at m/z 787.

Physical characteristics of isomer B are as follows:
FAB mass spectrum: Found: $[m^{\bullet}+H]^{+}$ at m/z 787.

B. Boc(im-Tos)NMHis(0TBDMS)CVANHCH$_2$ $\overset{*}{C}$H(0H)CH$_2$0H (Isomer A)

By the general procedure A described before the Experimental Section (reaction time is 2.25 h) for Boc group removal, 0.7693 g of the Boc amine of Part A (Isomer A) yields 0.6122 g of the amine free base. The amine is then coupled (coupling procedure B described before the Experimental Section) with Nα-t-butyoxycarbonyl-Nα-methyl-Nim-tosyl-L-histidine and chromatographed over a 200 ml silica gel column (elution with 3.5% methanol/methylene chloride containing 0.35% ammonium hydroxide) to yield four products. The first three (0.117 g, 0.080 g and 0.2212 g) are yet unidentified. The fourth product eluted yields 0.3186 g of the title product.

Physical characteristics are as follows:
FAB mass spectrum: Found: $[m^{\bullet}+H]^{+}$ at m/z 864.

Boc(im-Tos)NMHis(0TBDMS)CVANHCH$_2$ $\overset{*}{C}$H(0Ac)CH$_2$0Ac (Isomer A)

To a nitrogen covered ice bath cooled solution of 0.3186 g of the peptide glycol of Part B in 0.7 ml of pyridine is added 0.36 ml of acetic anhydride dropwise over 30 sec followed by 0.004 g of 4-dimethylaminopyridine. The cooled bath is removed and the solution is stirred at room temperature for 1.5 h and then cooled again in an ice bath. After stirring in the cold for 30 min, the ice bath is removed and the reaction is stirred at room temperature for 1.25 h and then poured into 5 ml of a 1:1 mixture of water and brine. The mixture is extracted three times with ethyl acetate and the combined extracts are washed in turn with cold 0.5 N hydrochloric acid, and then water, aqueous sodium bicarbonate and brine at ambient temperature, dried over magnesium sulfate and concentrated in vacuo. The residue is chromatographed over a 150 ml silica gel column (elution with 2% methanol/methylene chloride containing 0.2% ammonium hydroxide) to yield 0.2617 g of the acylated product.

Physical characteristics are as follows:
FAB mass spectrum: Found: $[m^{\bullet}+H]^{+}$ at m/z 948.

D. BocProPhe(im-Tos)LMHisCVANHCH$_2$ $\overset{*}{C}$H(0Ac)CH$_2$0Ac (Isomer A)

By the general procedure A for Boc group removal described before the Experimental Section (reaction time is 2.5 h), 0.2617 g of the Boc amine of Part C (Isomer A) yields 0.2163 g of the amine free base. The amine is then coupled (coupling procedure B described before the Experimental Section) with the product

from Example 29b and chromatographed over a 150 ml silica gel column (elution with 3% methanol/methylene chloride containing 0.3% ammonium hydroxide) to yield a 0.2483 g of coupled product.

Physical characteristics are as follows:

FAB mass spectrum: Found: $[m^{\bullet}+H]^{+}$ at m/z 1078.

E. THAMOAC(0)ProPhe(im-Tos)NMHisCVANHCH$_2$C*H(0Ac)CH$_2$0Ac (Isomer A)

By the general procedure A for Boc group removal described before the Experimental Section (reaction time is 2 h), 0.2430 g of the Boc amine of Part D (Isomer A) yields 0.210 g of the amine free base. To a nitrogen covered solution of 0.1022 g, the latter amine in 0.4 ml of dioxane is added 0.017 ml of triethylamine followed by 0.058 g of the title product from Preparation 3. After heating in an oil bath at 50° for 16.5 h, the reaction mixture is allowed to cool and is concentrated in vacuo. A solution of the residue in methylene chloride is washed with aqueous sodium bicarbonate. The aqueous wash is backwashed two times with methylene chloride. The combined organic fractions are dried over magnesium sulfate and concentrated in vacuo. The residue is chromatographed over a 50 ml silica gel column (elution with 3% methanol/methylene chloride containing 0.3% ammonium hydroxide) to yield 0.0703 g of the product.

Physical characteristics are as follows:

FAB mass spectrum: Found: $[m^{\bullet}+H]^{+}$ at m/z 1149.

F. THAMC(O)ProPheNMHisCVADHPA (Isomer A)

To a nitrogen covered solution of 0.0703 g of the protected peptide of Part E in 2.0 ml of methanol is added 0.14 ml of 1.46 M hydrochloric acid in diethyl ether. After stirring at room temperature for 2.25 h, the reaction mixture is concentrated in vacuo to yield 0.0687 g of a solid residue. To a nitrogen covered solution of this residue in 1.5 ml of methanol is added 0.4 ml of 5.45 M ammonia in methanol. After stirring at room temperature for 21.5 h, the reaction mixture is concentrated in vacuo. The residue is chromatographed over a 150 ml silica gel column (elution with 20% methanol/methylene chloride containing 0.5% ammonium hydroxide) to yield 0.0307 g of the title product.

Physical characteristics are as follows:

FAB mass spectrum: Found: $[m^{\bullet}+H]^{+}$ at m/z 887.

Measured = 887.5251.

$IC_{50}$: 0.36 x $10^{-9}$ M.

Example 31 [1R*,2R*,4R*(R*)]-1-[[(2-Deoxy-D-glycopyranos-2-yl)amino]carbonyl]-L-prolyl-L-phenylalanyl-N-[1-(cyclohexylmethyl)-4-[[(2,3-dihydroxypropyl)amino]carbonyl]-2-hydroxy-5-methylhexyl]-Nα-methyl-L-histidinamide (Isomer A) DGLUC(O)ProPheNMHisCVADHPA (Isomer A).

A. DGLUAC(O)ProPhe(im-Tos)NMHisCVANHCH$_2$ C*H(0Ac)CH$_2$0Ac (Isomer A)

To a nitrogen covered solution of 0.1081 g of the amine free base ProPhe(im-Tos)-NMHisCVANHCH$_2$ C*H(0Ac)CH$_2$0Ac (Isomer A), prepared from the product of Example 30, part D as in Example 30, part E, in 2 ml of dioxane is added 0.109 g of the title product from Preparation 10 followed by 0.021 g of triethylamine. After heating in an oil bath at 50° for 43.5 h, the reaction mixture is allowed to cool and then concentrated in vacuo. A solution of the residue in methylene chloride is washed once with aqueous sodium bicarbonate. The aqueous wash is backwashed twice with methylene chloride. The combined organic fractions are dried over magnesium sulfate and concentrated in vacuo. The residue is chromatographed over a 100 ml silica gel column (elution first with 4% methanol/methylene chloride containing 0.4% ammonium hydroxide and then 5% methanol/methylene chloride containing 0.5% ammonium hydroxide) to yield first 0.0581 g of the desired urea followed by 0.0225 g of recovered starting material.

Physical characteristics of the desired urea are as follows:

FAB mass spectrum: Found: $[m^{\bullet}+H]^{+}$ at m/z 1351.

B. DGLUC(O)ProPheNMHisCVADHPA (Isomer A)

To a nitrogen covered solution of 0.0581 g of the protected peptide of Part A in 1.4 ml of methanol is added 0.3 ml of 5.45 M ammonia in methanol. After stirring at room temperature for 18.5 h, the reaction mixture is concentrated in vacuo. The residue is chromatographed over a 50 ml silica gel column (elution with 30% methanol/methylene chloride containing 0.5% ammonium hydroxide) to yield 0.0115 g of the title product.

Physical characteristics are as follows:

FAB mass spectrum: Found: $[m^{\bullet}+H]^{+}$ at m/z 945.

Measured = 945.5308.

$IC_{50}$: 1.5 x $10^{-9}$ M.

Example 32 [1S-[1R*,2R*,4R*(R*)]]-1-[[[2-Hydroxy-1,1-bis(hydroxymethyl)ethyl]amino]carbonyl]-L-prolyl-L-phenylalaryl-N-[1-(cyclohexylmethyl)-2-hydroxy-4-[[[4-methoxy-4-oxo-1-[[(2-pyridinylmethyl)amino]carbonyl]-butyl]-amino]carbonyl]-5-methyl-hexyl]-Nα-methyl-L-histidinamide; or THAMC(O)ProPheNMHisCVA(OMe)-GluAmp

A. Boc(0Bz)GluAmp

By the general coupling procedure C described before the Experimental Section, 1.0 g of Nα-t-butoxycarbonyl-L-glutamic acid, α-benzyl ester is coupled with 2-(aminomethyl) pyridine and chromatographed over 200 ml of silica gel (elution with 2% methanol/methylene chloride containing 0.2% ammonium hydroxide) to yield 1.27 g of coupled product as a crystalline solid.

Physical characteristics are as follows:

M.P. 84°-86°.

B. Boc(0TBDMS)CVA(0Bz)GluAmp

By the general procedure A for Boc group removal described before the Experimental Section, 0.33 g of the Boc amine of Part A yields 0.256 g of the amine free base. The amine is then coupled (coupling procedure C described before the Experimental Section) with Boc (OTBDMS) CVA, prepared as described in Chart T and Preparations 41-48 and chromatographed over a 150 ml silica gel column (elution with 2% methanol/methylene chloride containing 0.2% ammonium hydroxide) to yield 0.51 g of coupled product.

Physical characteristics are as follows:

FAB mass spectrum: Found: $[m^{\bullet}+H]^{+}$ at m/z 795.

C. Boc(im-Tos)NMHis(0TBDMS)CVA(0Bz)GluAmp

By the general procedure A for Boc group removal described before the Experimental Section, 0.1469 g of the Boc amine of Part B yields 0.137 g of the amine free base. The amine is then coupled (coupling procedure B described before the Experimental Section) with Nα-t-butoxycarbonyl-Nα-methyl-Nim-tosyl-L-histidine and chromatographed over a 150 ml silica gel column (elution with 2.5% methanol/-methylene chloride containing 0.25% ammonium hydroxide) to yield 0.1344 g of the coupled product.

Physical characteristics are as follows:

FAB mass spectrum: Found: $[m^{\bullet}+H]^{+}$ at m/z 1100.

D. BocProPhe(im-Tos)NMHisCVA(0Bz)GluAmp and (N-Me)(im-Tos)NMHisCVA-(0Bz)GluAmp

By the general procedure A for Boc group removal described before the Experimental Section (reaction time is 4.8 h), 0.1344 g of the Boc amine of Part C yields 0.1235 g of the amine free base. The amine is then coupled (coupling procedure B) described before the Experimental Section with Nα-t-butoxycarbonyl-L-prolyl-phenylal-anime and chromatographed over a 150 ml silica gel column (elution first with 2.5% methanol/methylene chloride containing 0.25% ammonium hydroxide followed by 3.5% methanol/methylene

chloride containing 0.35% ammonium hydroxide) to yield first 0.0758 g of the desired coupled product (BocProPhe(ImTos)NMHisCVA(OBz)GluAmp followed by 0.017 g of the starting peptide having the Boc and TBDMS groups removed.

Physical characteristics of the coupled product are as follows:
FAB mass spectrum: Found: $[m^{\bullet}+H]^{+}$ at m/z 1230.

Physical characteristics of the starting peptide with the Boc and TBDMS groups removed are as follows:
FAB mass spectrum: Found: $[m^{\bullet}+H]^{+}$ at m/z 886.

E. THAMOAC(0)ProPhe(im-Tos)NMHisCVA(0Bz)GluAmp

By the general procedure A for Boc group removal described before the experimental Section (reaction time is 1.5 h), 0.071 g of the Boc amine of Part D yields 0.0638 g of the amine free base. To a nitrogen covered solution of the amine in 0.3 ml of dioxane is added 0.009 ml of triethylamine and 0.031 g of the title product from Preparation 3. After stirring in an oil bath at 50° for 18 h, the solution is concentrated in vacuo. A solution of the residue in methylene chloride is washed once with aqueous sodium bicarbonate. The aqueous wash is backwashed twice with methylene chloride. The combined organic layers are dried over magnesium sulfate and concentrated in vacuo. The residue is chromatographed over a 100 ml silica gel column (elution with 3.75% methanol/methylene chloride containing 0.38% ammonium hydroxide) to yield a 0.0495 g of the urea.

Physical characteristics are as follows:
FAB mass spectrum: Found: $[m^{\bullet}H]^{+}$ at m/z 1301.

F. THAMC(O)ProPheNMHisCVA(OMe)GluAmp

To a nitrogen covered solution of 0.049 g of the protected peptide of Part E in 1.2 ml of methanol is added 0.08 ml of 1.46 M hydrochloric acid in diethyl ether. After stirring at room temperature for 1.5 h, the solution is concentrated in vacuo and allowed to remain under vacuum for 30 min. To a nitrogen covered solution of the residue in 0.9 ml of methanol is added 0.25 ml of 5.45 M ammonia in methanol. After stirring for 20.75 h, the solution is concentrated in vacuo. The residue is chromatographed over a 50 ml silica gel column (elution with 10% methanol/methylene chloride containing 0.5% ammonium hydroxide) to yield first 0.0098 g of deprotected benzyl ester followed by 0.0152 g of the title product.

Physical characteristics of the peptide benzyl ester are as follows:
FAB mass spectrum: Found: $[m^{\bullet}H]^{+}$ at m/z 1123.

Physical characteristics of the title product are as follows:
FAB mass spectrum: Found: $[m^{\bullet}H]^{+}$ at m/z 1047.
Measured = 1047.588.
$IC_{50}$: 0.30 x $10^{-9}$ M.

Example 33 [1R*,2R*,4R*(R*)]-1-[[[2-Hydroxy-1,1-bis(hydroxymethyl)ethyl]amino]carbonyl]-L-prolyl-L-phenylalanyl-N-[1-(cyclohexylmethyl)-2-hydroxy-5-methyl-4-[[2-pyridinylmethyl)amino]carbonyl]hexyl]-Nα-methyl-L-histidinamide, pyridine N-oxide; or THAMC(O)ProPheNMHisCVAAmpNO.

A. Boc(0TBDMS)CVA-Amp

By the general coupling procedure C described before the Experimental Section, 2.0 g of Boc-(OTBDMS) CVA, prepared as described in Chart T and Preparations 41-48, is coupled with 2-(aminomethyl)pyridine and chromatographed over a 400 ml silica gel column (elution with 2% methanol/methylene chloride containing 0.2% ammonium hydroxide) to yield 2.49 g of coupled product.

Physical characteristics are as follows:
FAB mass spectrum: Found: $[m^{\bullet}H]^{+}$ at m/z 576.

B. Boc(im-Tos)NMHis(0TBDMS)CVA-Amp

To a nitrogen covered, ice bath cooled solution of 2.486 g of the Boc amine of Part A in 16 ml of methylene chloride is added 16 ml of trifluoroacetic acid dropwise over 13 min. The ice bath is removed and, after stirring for 1 h at room temperature, the solution is added dropwise over 8 min to a vigorously stirred mixture of 19.2 g of sodium bicarbonate in 130 ml of water and 75 ml of methylene chloride. after mixing well, the aqueous layer is separated and washed twice with methylene chloride. The combined organic fractions are dried over magnesium sulfate and concentrated in vacuo to yield 2.319 g of the amine free base. The amine is then coupled (coupling procedure B described before the Experimental Section) with Nα-t-butoxycarbonyl-Nα-methyl-Nim-tosyl-L-histidine and chromatographed over a 500 ml silica gel column (elution with 2.5% methanol/methylene chloride containing 0.25% ammonium hydroxide) to yield 2.49 g of coupled product.

Physical characteristics are as follows:
FAB mass spectrum: Found: $[m\cdot H]^+$ at m/z 881.

C. BocProPhe(imTos)NMHisCVAAmp

To a nitrogen covered, ice bath cooled solution of 2.495 g of the Boc amine of Part B in 12.1 ml of methylene chloride is added dropwise 12.1 ml of trifluoroacetic acid over 11 min. The ice bath is removed and after 4.75 h, the solution is added dropwise over 5 min to a vigorously stirred mixture of 14.5 g of sodium bicarbonate in 100 ml of water and 60 ml of methylene chloride. After mixing well, the aqueous layer is separated and washed twice with methylene chloride. The combined organic fractions are dried over magnesium sulfate and concentrated in vacuo to yield 2.11 g of the amine free base. A portion (0.50 g) of the crude amine is coupled (coupling procedure B described before the Experimental Section) with Nα-t-butoxycarbonyl-L-prolyl-L-phenylalanine and chromatographed over a 200 ml silica gel column (elution first with 1.5% methanol/methylene chloride containing 0.15% ammonium hydroxide and then 2.5% methanol/methylene chloride contianing 0.25% ammonium hydroxide) to yield in order compound A (0.2618 g), compound B (0.0513 g), and finally the desired coupled product C (0.2445 g).

Physical characteristics of compound A are as follows:
FAB mass spectrum: Found: $[m\cdot H]^+$ at m/z 903.

Physical characteristics of compound B are as follows:
FAB mass spectrum: Found: $[m\cdot H]^+$ at m/z 559.

Physical characteristics of product C are as follows:
FAB mass spectrum: Found: $[m\cdot H]^+$ at m/z 1011.

D. THAMOAC(O)ProPhe(im-Tos)NMHisCVAAmp

The Boc amine product C of Part C (0.1212 g) is dissolved in methylene chloride (1.0 ml), covered with nitrogen and cooled in an ice bath. Trifluoracetic acid (1.0 ml) is added dropwise from a pipette over 1 min. Stirring the solution in the cold is continued, and, after 1 h in the cold, a small aliquot is removed and diluted with about 5 drops of methylene chloride. It is then washed with 2 drops of aqueous sodium bicarbonate. The organic fraction is analyzed by TLC (elution with 5% methanol/methylene chloride containing 0.5% ammonium hydroxide). When deprotection is completed, there is a single product spot. After 1 h, 20 min, the reaction mixture is concentrated on a rotary evaporator. The residue is dissolved in methylene chloride and washed one time with aqueous sodium bicarbonate, and backwashed twice with methylene chloride. The pooled organic fractions are dried over magnesium sulfate and concentrated on a rotary evaporator to form a rigid foamy residue which amounts to 0.106 g. This reaction is repeated with 0.360 g of Boc amine which is dissolved in methylene chloride (3.0 ml), covered with nitrogen and cooled in an ice bath. Trifluoracetic acid (3.0 ml) is added dropwise from an addition funnel over 4-5 min. Stirring is continued in the cold for 1 h. The mixture is analyzed by TLC (as stated above) and deprotection is completed. After 1 h, 25 min, reaction mixture is concentrated on a rotary evaporator. Residue is dissolved in methylene chloride and washed once with aqueous sodium bicarbonate and backwashed twice with methylene chloride. Pooled organic fractions are dried over magnesium sulfate and concentrated on rotary evaporator to form a hard foamy residue which amounts to 0.3304 g.

The product (0.436 g) from the above reactions is combined and dissolved in dioxane (2.3 ml). The mixture is covered with nitrogen and treated with triethylamine (0.053 g) followed by the title product from Preparation 3 (0.25 g). The solution is heated in a oil bath at 50°. After heating for 20 h, 15 min, the oil bath is removed and the reaction mixture is allowed to cool. It is analyzed by TLC (elution with 5%

methanol/methylene chloride contianing 0.5% ammonium hydroxide); the reaction is complete. The reaction mixture is concentrated on a rotary evaporator. The residue is dissolved in methylene chloride and washed once with aqueous sodium bicarbonate. The aqueous wash is backwashed twice with methylene chloride. The pooled organic fractions are dried over magnesium sulfate and concentrated on rotary evaporator to a gummy residue which amounts to 0.7861 g. This material is absorbed onto silica gel and placed on a 150 ml silica gel column and settled in 3% methanol/methylene chloride contianing 0.3% ammonium hydroxide. The column is eluted with the same solvent to fraction 181 and then with 3.5% methanol/methylene chloride containing 0.35% ammonium hydroxide; 5.4 ml fractions are collected and fractions 216-302 are combined. The product amounts to 0.2978 g.

Physical characteristics are as follows:
FAB mass spectrum: Found: $[m \cdot H]^+$ at m/z 1082.

E. THAMOAC(O)ProPhe(im-Tos)NMHisCVAAmpNO

The peptide of Part D (0.1728 g) is dissolved in chloroform (4.5 ml), covered with nitrogen and m-chloroperoxybenzoic acid (0.044 g) is added. The clear solution is stirred at room temperature for 1.5 h. The mixture is analyzed by TLC (elution with 5% methanol/methylene chloride containing 0.5% ammonium hydroxide. The reaction mixture is poured by pipette into aqueous sodium bicarbonate. After mixing well, the aqueous layer is separated and extracted twice with chloroform. The combined organic fractions are washed once with aqueous sodium bicarbonate and once with dilute brine. The aqueous fractions are backwashed with chloroform. Pooled organic fractions are dried over magnesium sulfate and concentrated on a rotary evaporator to a solid residue which amounts to 0.186 g. This material is adsorbed onto silica gel and placed on a 150 ml silica gel column settled in 5% methanol/methylene chloride contianing 0.5% ammonium hydroxide. The residue is eluted with the same solvent and 5.6 ml fractions are collected. The fractions are combined as follows:

| | |
|---|---|
| Fractions 128-191 | (B) - 0.0334 g. |
| Fractions 192-245 | (C) - 0.0640 g. |
| Fractions 246-272 | (D) - 0.0400 g. |
| Fractions 273-306 | (E) - 0.0113 g. |

Physical characteristics of B are as follows:
FAB mass spectrum Found: $[m \cdot H]^+$ at m/z 1098.
Physical characteristics of C are as follows:
FAB mass spectrum Found: $[m \cdot H]^+$ at m/z 1098.
Physical characteristics of E are as follows:
FAB mass spectrum Found: $[m \cdot H]^+$ at m/z 1116.

F. THAMC(O)ProPheNMHisCVAAmpNO

Product D (0.040 g) of Part E which by TLC is a mixture of the titled product (B, C) and the partially hydrolyzed ortho ester product E is dissolved in 1.2 ml of methanol, covered with nitrogen, and 1.46 N hydrochloric acid in diethyl ether (0.08 ml) is added. The solution is stirred at room temperature for about 50 min. Two drops of the reaction mixture is removed and made basic with a couple of drops of 5.4 M ammonia in methanol. It is analyzed by TLC (elution with 7% methanol/methylene chloride containing 0.5% ammonium hydroxide) and shows the starting mixture is gone. After 1 h, the reaction mixture is concentrated on a rotary evaporator and is allowed to remain under vacuum for about 45 min. A gummy residue remains. This is dissolved in methanol (0.9 ml), covered with nitrogen and 5.45 M ammonia in methanol (0.24 ml) is added. Solution is stirred at room temperature for about 19 h and analyzed by TLC (elution with 20% methanol/methylene chloride containing 0.5% ammonium hydroxide). There is a heavy polar product spot present. The reaction mixture is placed on rotary evaporator and concentrated to a semisolid residue which is adsorbed onto silica gel and placed on a 50 ml silica gel column, settled in 15% methanol/methylene chloride containing 0.5% ammonium hydroxide. The residue is eluted with the same solvent and 300 drop (4.6 ml) fractions are collected

The following fractions are combined:

| | |
|---|---|
| Fractions 54-86 | (A) - 0.0082 g. |
| Fractions 87-160 | (B) - 0.0225 g. |

Physical characteristics of B are as follows:
HR FAB mass spectrum Found: $[m \cdot H]^+$ at m/z 920.
Measured: 920.4259.

Example 34 [1R*,2R*,4R*(R*)]-1-[[(2-Deoxy-D-glucopyranos-2-yl)amino]carbonyl]-L-prolyl-L-phenylalanyl-N-[1-(cyclohexylmethyl)-2-hydroxy-5-methyl-4-[[(2-pyridylmethyl)amino]carbonyl]hexyl]-Nα-methyl-L-histidinamide; or DGLUC(O)ProPheNMHisCVAAmp.

A. DGLUAC(O)ProPhe(im-Tos)NMHisCVAAmp

The BocProPhe(imTos)NMHisCVAAmp prepared as in Part C of Example 33 (0.4959 g) is dissolved in methylene chloride (2.1 ml), covered with nitrogen, and cooled in an ice bath. Trifluoroacetic acid (2.1 ml) is added dropwise from an addition funnel over 2.5 min. Stirring is continued in the cold for 1 h, 15 min. The reaction mixture is concentrated on a rotary evaporator and the residue is dissolved in methylene chloride and washed once with aqueous sodium bicarbonate. The aqueous wash is backwashed twice with methylene chloride. The pooled organic fractions are dried over magnesium sulfate and concentrated on a rotary evaporator to a rigid foamy residue which amounts to 0.4298 g. This material is dissolved in dimethylformamide (9.2 ml), covered with nitrogen and treated with triethylamine (0.065 g) followed by the title product from Preparation 10 (0.46 g). The residue is heated in an oil bath at 50°. After 5.75 h, it is analyzed by TLC (elution with 5% methanol/methylene chloride containing 0.5% ammonium hydroxide). The reaction is complete and the reaction mixture is concentrated on a rotary evaporator at vacuum pump pressure to yield a gummy residue that amount to 1.0628 g. This material is adsorbed onto silica gel and placed on a 150 ml silica gel column, settled in 3.75 % methanol/methylene chloride containing 0.38% ammonium hydroxide. The residue is eluted with the same solvent and 5.3 ml fractions are collected. Fractions 66-126 are combined to give 0.5395 g. of the titled product.
Physical characteristics are as follows:
FAB mass spectrum Found: $[m \cdot H]^+$ at m/z 1284.

B. DGLUC(O)ProPheNMHisCVAAmp

The protected peptide of Part A (0.2761 g) is dissolved in methanol (6.9 ml), covered with nitrogen, and 5.45 M ammonia in methanol (1.5 ml) is added. After stirring at room temperature for 17 h, 40 min, the reaction is a brownish color. The mixture is analyzed by TLC (elution with 20% methanol/methylene chloride containing 0.5% ammonium hydroxide). The reaction is complete and a polar product is present. The reaction mizture is concentrated on a rotary evaporator to an amorphous solid residue that amounts to 0.2454 g. This material is adsorbed onto silica gel and placed on a 150 ml silica gel column, settled in 20% methanol/methylene chloride containing 0.5% ammonium hydroxide. The mixture is eluted with the same solvent and 5.6 ml fractions are collected. The following fractions are combined:

| | | | |
|---|---|---|---|
| Fractions 156-270 | (B) - 0.0862 g., | HPLC: | 88.9%. |
| Fractions 271-330 | (C) - 0.0164 g., | HPLC: | 50.7%. |

Sample B is dissolved in a small amount of methanol, mixed with 10 ml of water and lyophilized to give 84 mg of the titled product.
Physical characteristics are as follows:
HR FAB mass spectrum: Found: $[m \cdot H]^+$ at m/z 962.
Measured = 962.5338.

Example 35 [1R*,2R*,4R*(R*)]-1-[[(2-Deoxy-D-glucopyranos-2-yl)amino]carbonyl]-L-prolyl-L-phenylalaryl-N-[1-(cyclohexylmethyl)-2-hydroxy-5-methyl-4-[[(2-pyridylmethyl)amino]carbonyl]hexyl]-Nα-methyl-L-histidinamide, pyridine N-oxide; or DGLUC(O)ProPheNMHisCVAAmpNO.

A. DGLUAC(O)ProPhe(im-Tos)NMHisCVAAmpNO

The DGLUAC(O)ProPhe(im-Tos)NMHisCVAAmp, prepared in Part A of Example 34 (0.2553 g) is dissolved in chloroform (4.3 ml), covered with nitrogen, and m-chloroperbenzoic acid (0.049 g) added. The solution is stirred at room temperature for 1 h, 5 min. The mixture is analyzed by TLC (elution with 5% methanol/methylene chloride containing 0.5% ammonium hydroxide). The reaction is complete and is treated with 5.0 ml of a 10% (10 g/100 ml) aqueous solution of sodium sulfite. It is mixed well and the aqueous fraction is separated and washed three times with chloroform. The pooled organic fractions are dried over magnesium sulfate and concentrated on a rotary evaporator to an amorphous solid that amounts to 0.2661 g. This material is adsorbed onto silica gel and placed on a 150 ml silica gel column, settled in 5% methanol/methylene chloride containing 0.5% ammonium hydroxide. The mixture is eluted with the same solvent and 5.4 ml fractions are collected. Fractions 94-180 are combined to give 0.2309 g. of the product.

Physical characteristics are as follows:
FAB mass spectrum Found: $[m \cdot H]^+$ at m/z 1300.

B. DGLUC(O)ProPheNMHisCVAAmpNO

The protected peptide of Part A (0.2309 g) is dissolved in methanol (5.7 ml), covered with nitrogen, and 5.45 M ammonia in methanol (1.2 ml) is added. After stirring at room temperature for 17 h, 15 min, the reaction takes on a light brown color. It is analyzed by TLC (elution with 20% methanol/methylene chloride containing 0.5% ammonium hydroxide) showing the reaction is complete. One major polar product plus several less polar materials remain, with no starting peptide. The reaction mixture is concentrated on a rotary evaporator to about 1/3 volume. A small amount of silica gel is added and the mixture is concentrated further to a free flowing solid residue. The residue is placed on a 150 ml silica gel column, settled in 2.5% methanol/methylene chloride contianing 0.5% ammonium hydroxide. The mixture is eluted with the same solvent and 5.2 ml fractions are collected. The following fractions are combined:

| | | | |
|---|---|---|---|
| Fractions 176-268 | (B) - 0.0525 g, | HPLC: | 86.4%. |
| Fractions 269-340 | (C) - 0.0232 g, | HPLC: | 61.4%. |

Sample B is solubilized in a small amount of methanol, mixed with 10 ml of water and lyophilized to give 51.5 mg. of the titled product.

Physical characteristics of are as follows:
HR FAB mass spectrum Found: $[m \cdot H]^+$ at m/z 978.
Measured = 978.5307.

Preparation 28 (Formula R-2) Refer to Chart R

A solution of R-1 (0.984 g,) in formamide (10 ml) is treated at ambient temperature under nitrogen with sodium azide (0.497 g). The resulting mixture (yellow) is stirred for 3 h at 85 ± 5 °C, cooled to room temperature, partitioned between ice water (100 ml) and chloro form (100 ml). The layers are separated, and the aqueous layer is extracted twice with 100 ml of chloroform. The chloroform extracts are combined. The organics are dried over sodium sulfate, filtered and concentrated to give 0.72 g of the title product as a tan solid.

Physical characteristics are as follows:
NMR($CDCl_3$,TMS)δ: 1.99, 2.04, 2.11, 3.75-3.79, 3.92, 4.15-4.31, 4.75, 5.08-5.28, 5.57.
Mass Spec (FAB): $[M+H]^+$ at 373.150. Other m/z 331, 330, 210, 168, 150, 108.
TLC (Silica Gel GF): $R_f = 0.13$ in 1:1 ethyl acetate-hexane.

Preparation 29 (Formula R-3) Refer to Chart R.

A suspension of azide R-2 of Preparation 28 (0.64 g) and 5% palladium/calcium carbonate (0.27 g) in absolute ethanol (50 ml) is hydrogenated at atmospheric pressure for 2.25 hours, filtered through Celite, and the filtercake is washed with ethanol. The combined filtrate is concentrated in vacuo to give 0.59 g of crude title product as a greenish-brown solid.

Physical characteristics are as follows:

NMR($CDCl_3$,TMS)$\delta$: 1.98, 2.04, 2.05, 2.11, 3.61-3.68, 3.97-4.25, 5.00-5.12, 5.72.

Mass Spec (FAB): [M + H]+ at 347.1463. Other m/z 330, 210, 168, 150, 126.

TLC (Silica Gel GF): $R_f$ is 0.18 in 20% ethanol/ethyl acetate.

Preparation 30 (Formula R-4) Refer to Chart R.

A solution of R-3 of Preparation 29 (0.236 g) in methylene chloride (17 ml) is treated at ambient temperature under argon with L-aspartic acid, $\alpha$-benzyl ester (0.275 g) followed by triethylamine (0.12 ml) and diethylphosphoryl cyanide (0.144 ml). The resulting yellow solution is stirred at room temperature under argon for 3 hours, partitioned between methylene chloride (100 ml) and cold, saturated sodium bicarbonate (100 ml). The layers are separated and the aqueous layer is extracted with methylene chloride (100 ml). The organics are dried over sodium sulfate, filtered and concentrated to give 0.44 g of crude title product as a brown solid.

The crude product is chromatographed on flash column -1 3/8" x 8" of HPLC grade silica gel in 20% acetone/methylene chloride while collecting 50 ml fractions. Fractions #9-15 are combined and concentrated to give 0.291 g, title product as a waxy brown solid.

Physical characteristics are as follows:

NMR ($CDCl_3$,TMS)$\delta$: 1.42, 1.94, 2.05, 2.07, 2.09, 2.18, 2.69, 2.83, 3.72, 4.03-4.11, 4.26-4.32, 4.54-4.63, 4.93-5.25, 5.71-5.18, 7.20-7.36.

Mass Spec (FAB): $[M+H]^+$ at 652.2738. Other m/z, 596, 552, 492, 330, 223, 210, 168, 150, 126, 108, 91.

TLC (Silica Gel GF): $R_f$ is 0.42 in 20% acetone/methylene chloride.

Preparation 31 (Formula R-5) Refer to Chart R.

A suspension of R-4 of Preparation 30 (0.292 g) and 10% palladium/charcoal (0.065) in methanol (25 ml) is hydrogenated at 50 p.s.i. for 4 h, filtered through Celite and the filtercake is washed with methanol (25 ml). The combined filtrte is concentrated in vacuo to give 0.239 g of crude title product. Crude title product is taken into further reaction.

Physical characteristics are as follows:

NMR ($CDCl_3$,TMS)$\delta$: 1.42, 1.97, 2.03, 2.05, 2.08, 2.61-2.98, 3.85-4.55, 5.10-5.25, 5.92-5.98.

Mass Spec (FAB): $[M+H]^+$ at 562.2243. Other m/z, 506, 488, 462, 368, 330, 210, 168, 150, 126.

TLC (Silica Gel GF): $R_f$ is 0.13 in 20% of 4M ammonia/methanol in methylene chloride.

Preparation 32 (Formula R-6) Refer to Chart R.

A solution of Boc-Pro-Phe-N-MeHis(Ts)-Leu[CH(0H)$CH_2$]-Val-Ile-AMP (0.33 g) in methylene chloride (2 ml) and trifluoroacetic acid (2 ml) is stirred at ambient temperature under argon for 2.5 hours, transferred dropwise into a rapidly stirred suspension of sodium bicarbonate (2.42 g) in water (17 ml) and methylene chloride (30 ml). The layers are separated and the aqueous layer is extracted twice with 20 ml of methylene chloride each time. The organics are combined, dried over sodium sulfate, filtered and concentrated to give 0.30 g of the title product as a more polar foam.

The crude product is taken into further reaction immediately.

Physical characteristics are as follows:

TLC (Silica Gel GF): $R_f$ is 0.13 in 5% of 9:1 methanol-ammonium hydroxide in methylene chloride. $R_f$ is 0.47 in 10% of 9:1-methanol-ammonium hydroxide in methylene chloride.

Preparation 33 (Formula R-7) Refer to Chart R.

A solution of R-6 of Preparation 32 (0.335 g) and R-5 of Preparation 31 (0.239 g) in methylene chloride (8.5 ml) is treated at room temperature under argon with triethylamine (61 µl) and diethylphosphoryl cyanide (72 µl). The reaction is stirred overnight at room temperature under argon. The reaction is partitioned between saturated sodium bicarbonate (50 ml) and methylene chloride (50 ml). The layers are separated, and the aqueous layer is extracted twice with 50 ml of methylene chloride each time. The organics are dried over sodium sulfate, filtered and concentrated to a foam.

The crude product is chromatographed on flash column -1 3/8" x 8" of grade silica gel in 5% methanol/methylene chloride while collecting 50 ml fractions. Fractions #11-21 are combined and concentrated to give 0.32 g the title product as an off-white solid.

Physical characteristics are as follows:

Mass Spec (FAB): $[M+H]^+$ at 1527.7. Other m/z 1469.8, 1427.6, 1373.7, 1093.4, 688.3, 586.4, 519.3, 488.2.

TLC (Silica Gel GF): $R_f$ is 0.44 in 10% methanol/methylene chloride.

Example 36 β-N-AcGLc-α-N-Boc-Asn-Pro-Phe-N-MeHis-Leu[CH(0H)$CH_2$]-Val-Ile-AMP (Formula R-8) Refer to Chart R.

A solution of R-7 of Preparation 33 (0.32 g) in methanol (12 ml) is treated at room temperature under argon with water (2.4 ml) followed by dropwise addition of 1 N 2.4 ml of potassium hydroxide. The resulting solution is stirred for 1 h at ambient temperature, concentrated in vacuo, diluted with cold water (250 ml) and a little methanol. The aqueous suspension is extracted three times with 200 ml of chloroform each time, then three times with 200 ml of ethyl acetate each time. The organics are dried over sodium sulfate, filtered and concentrated to a pale yellow solid.

The crude product is chromatographed on flash column -1 3/8" x 10" of HPLC grade silica gel in 20% of 4 M ammonia/methanol in methylene chloride while collecting 50 ml fractions. Fractions #21-38 are combined and concentrated to give 0.151 g of the title product.

Physical characteristics are as follows:

Mass Spec (FAB): $[M+H]^+$ at 1247.706. Other m/z 1147, 586, 510, 365, 342, 222, 204, 124, 109.

TLC (Silica Gel GF): $R_f$ is 0.14 in 20% of 5 M ammonia/methanol in methylene chloride.

$IC_{50}$: 4.1 x $10^{-10}$ M.

Preparation 34 (Formula R-9) Refer to Chart R.

A solution of Boc-Phe-N-MeHis(Ts)-Leu[CH(0H)$CH_2$]-Val-Ile-AMP (0.24 g) in methylene chloride (2 ml) and trifluoroacetic acid (2 ml) is stirred at ambient temperature under argon for 3 hours, partitioned between saturated sodium bicarbonate (40 ml) and methylene chloride (40 ml). The layers are separated, and the aqueous layer is extracted twice with 40 ml of methylene chloride each time. The combined methylene chloride extracts are dried over sodium sulfate, filtered and concentrated to a quantitative yield of the title product as a light yellow solid.

The crude product is taken into further reaction immediately.

Physical characteristics are as follows:

TLC (Silica Gel GF): $R_f$ is 0.28 in 5% methanol/methylene chloride.

Preparation 35 (Formula R-10) Refer to Chart R.

A solution of R-9 of Preparation 34 (0.214 g) and R-5 of Preparation 31 (0.17 g) in dry methylene chloride (6 ml) is treated at ambient temperature under argon with triethylamine (42 µl) and diethylphosphoryl cyanide (52 µl). The solution is stirred for 3 h, poured into ice-cold saturated sodium bicarbonate (40 ml) and extracted three times with 40 ml of methylene chloride each time. The organics are dried over sodium sulfate, filtered and concentrated to a solid.

The crude product is chromatographed on flash column -1 3/8" x 8" of HPLC grade silica gel-in 4% methanol/methylene chloride while collecting 50 ml fractions. Fractions #22-30 are concentrated in vacuo to give 0.225 g of the title product.

Physical characteristics are as follows:

Mass Spec (FAB): $[M+H]^+$ 1431. Other m/z 1277, 997, 741, 587, 519, 488, 453, 365, 330.

TLC (Silica Gel GF): $R_f$ is 0.49 in 10% methanol/methylene chloride.

Example 37 β-N-AcGlc-α-N-Boc-Asn-Phe-N-MeHis-Leu[CH(0H)$CH_2$]-Val-Ile-AMP (Formula R-11) Refer to Chart R.

A solution of R-10 of Preparation 35 (0.219 g) in methanol (7.5 ml) is treated at ambient temperature under argon with water (1.5 ml) followed by 1N potassium hydroxide (1.5 ml). The solution is stirred at room temperature for 2 h, concentrated in vacuo and diluted with ice-cold brine (60 ml) and methylene chloride (60 ml). The resulting suspension is centrifuged to give an aqueous layer, insoluble white residue and methylene chloride layer. The white solid is dissolved in methanol and concentrated in vacuo to give a white solid (0.164 g) which is homogeneous by TLC.

Physical characteristics are as follows:
Mass Spec (FAB):$[M+H]^+$ 1150. Other m/z 586, 365, 222, 204, 177, 138, 124, 109.
TLC (Silica Gel GF): $R_f$ is 0.16 in 20% of 4M ammonia/methanol in methylene chloride.

Preparation 36 (Formula S-2) Refer to Chart S.

A solution of S-1 (1.0 g) and sodium azide (0.52 g) in formamide (10 ml) is stirred at 80 ± 5° under argon for 3.5 h, cooled to room temperature, diluted with ice water (100 ml) and extracted three times with 100 ml of chloroform each time, then three times with 100 ml of ethyl acetate each time. The organics are dried over sodium sulfate, filtered and concentrated to a yellow oil.

The crude product is chromatographed on flash column -1 3/8" x 13" of HPLC grade silica gel in 30% ethyl acetate/hexane while collecting 50 ml fractions. Fractions #17-21 are combined and concentrated to give 0.55 g of the title product.

Physical characteristics are as follows:
NMR ($CDCl_3$,TMS)δ: 2.00, 2.05, 2.12, 2.17, 4.09-4.18, 4.29-4.38, 5.16, 5.26-5.35, 5.40.
Mass Spec (CI): $[M+NH_4]^+$ at 391; m/z 363, 336, 303, 276, 245, 168.
TLC (Silica Gel GF): $R_f$ is 0.55 in 1:1 ethyl acetate-hexane.
$IC_{50}$: 3.6 x $10^{-8}$ M.

Preparation 37 (Formula S-3) Refer to Chart S.

A suspension of azide S-2 of Preparation 36 (0.55 g) and 5% palladium/calcium carbonate (0.22 g) in absolute ethanol (17 ml) is hydrogenated at atmospheric pressure for 2.5 h at room temperature. The suspension is filtered through Celite, and the filtercake is washed with absolute ethanol. The combined filtrate is concentrated in vacuo to give 0.427 g of off-white solid. The crude product is taken into further reaction.

Physical characteristics are as follows:
NMR ($CDCl_3$,TMS)δ: 1.99, 2.05, 2.11, 2.21, 3.61-3.70, 4.05-4.32, 4.47, 5.03-5.22, 5.39-5.44.
Mass Spec (FAB): $[M+H]^+$ at 348.1286. Other m/z 331, 288, 271, 228, 169, 149, 127, 109.
TLC (Silica Gel GF): $R_f$ is 0.22 in 5% methanol/methylene chloride.

Preparation 38 (Formula S-4) Refer to Chart S.

A solution of S-3 at Preparation 37 (0.423 g) in methylene chloride (28 ml) is treated at ambient temperature under argon with L-aspartic, α-benzyl ester (0.468 g), triethylamine (0.2 ml), followed by diethylphosphoryl cyanide (0.24 ml). The reaction is stirred for 20 h, poured into ice-cold, saturated sodium bicarbonate (150 ml) and methylene chloride (150 ml). The suspension is partitioned, and the layers are separated. The aqueous layer is extracted with methylene chloride (150 ml) and the organics are dried over sodium sulfate, filtered and concentrated to a brown semi-solid.

The crude product is chromatographed on flash column -1 3/8" x 12" of HPLC grade silica gel in 10% acetone/methylene chloride while collecting 50 ml fractions. Fractions #9-13 are combined and concentrated to give 0.292 g of the title product.

Physical characteristics are as follows:
NMR ($CDCl_3$,TMS)δ: 1.42, 1.99, 2.05, 2.09, 2.25, 2.68-2.94, 3.72-3.81, 4.03-4.18, 4.30, 4.52-4.58, 5.05-5.33, 5.43, 5.65, 6.55, 7.35-7.36.
Mass Spec (FAB): $[M+H]^+$ at 653.2556; other m/z 597, 553, 417, 391, 331, 223, 169, 127, 109, 91.

TLC (Silica Gel GF): $R_f$ is 0.30 in 10% acetone/methylene chloride.

Preparation 39 (Formula S-5) Refer to Chart S.

A suspension of S-4 of Preparation 38 (0.25 g) and 10% palladium on charcoal (0.062 g) in absolute methanol (20 ml) is hydrogenated at 50 p.s.i. for 4 h, filtered through Celite and the filtercake is washed with methanol. The combined filtrate is concentrated in vacuo to give 0.22 g of crude title product.

The crude produce is taken into further reaction without purification.

Physical characteristics are as follows:

NMR ($CDCl_3$,TMS)δ: 1.44, 1.98, 2.05, 2.10, 2.23, 2.67-3.93, 3.78-3.86, 4.04-4.48, 5.08-5.30, 5.24, 5.38, 5.53, 5.87.

Mass Spec (FAB): $[M+H]^+$ at 563.2108. Other m/z 517, 507, 489, 463, 417, 331, 301, 169, 57.

TLC (Silica Gel GF): $R_f$ is 0.25 in 20% of 4M ammonia/methanol in methylene chloride.

Preparation 40 (Formula S-6) Refer to Chart S.

A solution of R-6 of Preparation 32 (0.165 g) in methylene chloride (4.5 ml) is treated at ambient temperature under argon with S-5 of Preparation 39 (0.118 g), followed by triethylamine (30 µl), followed by diethylphosphoryl cyanide (36 µl). The reaction is stirred overnight at room temperature under argon, poured into ice-cold, saturated sodium bicarbonate (75 ml) and extracted three times with 75 ml of methylene chloride each time. The organics are dried over sodium sulfate, filtered and concentrated to a foam.

A crude product is chromatographed on flash column -1 3/8" x 8" of HPLC grade silica gel in 5% of 4 M ammonia/methanol in methylene chloride while collecting 50 ml fractions. Fractions #2-5 are combined and concentrated to give 0.231 g of the title product.

Physical characteristics are as follows:

Mass Spec (FAB): $[M+H]^+$ at 1528.6; other m/z 1374.5 277.9, 168.9.

TLC (Silica Gel GF): $R_f$ is 0.49 in 10% of 4M ammonia/methanol in methylene chloride.

Example 38 β-Man-α-N-Boc-Asn-Pro-Phe-N-MeHis-Leu[CH(OH)$CH_2$]-Val-Ile-AMP (Formula S-7) Refer to Chart S.

A solution of S-6 of Preparation 40 (0.22 g) in methanol (6 ml) is treated at ambient temperature under argon with water (1.2 ml) followed by dropwise addition of 1N aqueous potassium hydroxide (1.2 ml). The solution is stirred for 1 h at ambient temperature under argon, concentrated in vacuo and diluted with ice water (100 ml). The suspension is extracted three times with 80 ml of chloroform each time, then three times with 100 ml of ethyl acetate each time, and the organics are combined, dried over sodium sulfate, filtered and concentrated.

The crude product is chromatographed on flash column -1 3/8" x 8" of HPLC grade silica gel in 20% of 4M ammonia/methanol in methylene chloride while collecting 45 ml fractions. Fraction #19-36 are combined and concentrated to give 0.114 g of the title product.

Physical characteristics area as follows:

Mass Spec (FAB): $[M+H]^+$ at 1206; other m/z 365, 222, 211, 196, 138, 124, 109.

TLC (Silica Gel GF): $R_f$ is 0.19 in 20% of 4M ammonia/methanol in methylene chloride.

$IC_{50}$: 5.5 x $10^{-10}$ M.

Examples 39-45

Using procedures analogous to those described in Preparations 28-40 and Examples 36-38, the following compounds are prepared:

Example 39 β-N-AcGlc-α-N-Boc-Asn-Pro-Phe-N(Me)His-LVA-Ile-AMP-NO

Physical characteristics are as follows:
Mass Spec (FAB): Found: 1263.708.
Other m/z: 1247, 602, 510, 365, 342, 204, 149, 138, 124, 109, 86, 70, 57.
TLC (Silica Gel GF): $R_f$ is 0.13 in 25% of 5 M ammonia/methanol in methylene chloride. $R_f$ is 0.30 in 30% of 5 M ammonia/methanol in methylene chloride.

Example 40 β-N-AcGlc-α-Asn-Pro-Phe-N(Me)His-LVA-Ile-AMP

Physical characteristics are as follows:
Mass Spec (FAB): Found: 1147.649.
Other m/z: 1129, 586, 365, 222, 204, 124, 109, 86, 70.
TLC (Silica Gel GF): $R_f$ is 0.14 in 30% of 5 M ammonia/methanol in methylene chloride.

Example 41 β-N-AcGlc-α-Asn-Pro-Phe-N(Me)His-LVA-Ile-AMP-NO

Physical characteristics are as follows:
Mass Spec (FAB): Found: 1163.646.
Other m/z: 1147, 365, 204, 124, 109, 86, 70.
TLC (Silica Gel GF): $R_f$ is 0.11 in 30% of 5 M ammonia/methanol in methylene chloride; $R_f$ is 0.13 in 30% of 9:1 methanol-ammonium hydroxide in methylene chloride.

Example 42 β-N-AcGlc-α-N-Boc-Asn-Pro-Phe-N(Me)His-LVA-Ile-THAM

Physical characteristics are as follows:
Mass Spec (FAB): Found: 1260.
Other m/z: 713, 599, 510, 365, 204, 124, 109, 86, 70, 57.
TLC (Silica Gel GF): $R_f$ is 0.28 in 30% of 4 M ammonia/methanol in methylene chloride.

Example 43 β-N-AcGlc-C(O)-$(CH_2)_2$-C(O)-Pro-Phe-N(Me)His-LVA-Ile- AMP

Physical characteristics are as follows:
FAB (High Resolution) Mass Spec: Found: 1132.639.
Other m/z: 586, 478, 365, 327, 299, 222, 204, 152, 124, 109, 86, 70.
TLC (Silica Gel GF): $R_f$ is 0.26 in 30% of 4.5 M ammonia/methanol in methylene chloride.

Example 44 β-N-AcGlc-α-N-Boc-D-Asn-Pro-Phe-N(Me)His-LVA-Ile-AMP

Physical characteristics are as follows:
Mass Spec (FAB): Found: 1247.706 for $[M+H]^+$.
Other m/z: 586, 510, 365, 239, 222, 204, 138, 124, 109, 86, 70, 57.
TLC (Silica Gel GF): $R_f$ is 0.09 in 20% of 4 M ammonia/methanol in methylene chloride. $R_f$ is 0.26 in 30% of 4 M ammonia/methanol in methylene chloride.

Example 45 β-N-AcGlc-α-N-Boc-Asn-Pro-Phe-His-Chaψ($CH_2$NH)Phe-$NH_2$

Physical characteristics are as follows:
FAB (High Resolution) Mass Spec: Found: 1102.597.
Other m/z: 242, 204, 120, 110, 81, 70, 57.
TLC (Silica Gel GF): $R_f$ is 0.11 in 20% of 4.5 M ammonia/methanol in methylene chloride.

Preparation 41 (R)-γ-Butyrolactone-γ-carboxylic acid (Formula T-2) Refer to Chart T.

Using a modified literature procedure [O.H. Gringore and F.P. Ronessac, Org. Syn., 63, 121 (1984)], a flask equipped with a motor stirrer and 2 addition funnels is charged with D-glutamic acid (100 g), and distilled water (680 ml). A solution of sodium nitrite (57.1 g), in water 407.8 ml) is added to one additional funnel and 2 N sulfuric acid (407.8 ml) is added to the other funnel. Simultaneous addition while maintaining a temperature of 30°C requires 1 h to complete. The reaction mixture is stirred at room temperature overnight then divided into two portions and concentrated on the rotary evaporator using dry ice-acetone traps and Hi vacuum to reduce the volume to about 300 ml. The two portions are freeze dried and triturated four times with 200 ml of boiling ethyl acetate to yield 35.32 g and 34.14 g of the crude product. The structure is confirmed by NMR.

Preparation 42 (R)-γ-Butyrolactone-γ-carboxylic acid chloride (Formula T-3) Refer to Chart T.

Using modified literature procedure [C. Eguchi and A. Kakuta, Bull.Chem.Soc. Japan, 47, 1704 (1974)], two reactions are run, one using 35.32 g of the acid of Formula T-2 of Preparation 41 and 39.48 ml of thionyl chloride; and other using 34.14 g of the acid and 38.16 ml thionyl chloride. In each reaction the thionyl chloride is added dropwise over one half hour to the acid at room temperature then heated at 80°C for 2.5 h. The reactions are concentrated on the rotary evaporator, combined and distilled to give 36.4 g of product, b.p. 129-134°C (0.9 mm Hg). A crystalline pot residue from the distillation is starting material; this is recycled using 20 ml of thionyl chloride, to give 44.5 g of additional product, b.p. 110-115°C (0.8 mmHg), (lit. b.p. 117.10 (4.5 mm Hg)]. The NMR supports the structure.

Preparation 43 (4R,5R)-4,5-Dihydroxy-6-cyclohexylhexanoic acid, γ-lactone (Formula T-5) Refer to Chart T.

To an nitrogen covered, dry ice:acetone cooled solution of 20.0 g of the acid chloride of Formula T-3 of Preparation 42 in 120 ml of tetrahydrofuran is added over 2.4 h a solution of the Grignard reagent prepared from 3.27 g of magnesium and 17.1 ml of cyclohexylmethyl bromide in 160 ml of tetrahydrofuran. The cold bath is removed and the reaction is allowed to warm over 1.75 h at room temperature. The reaction is then cooled in an ice bath and there is added 240 ml of brine followed by 300 ml of diethyl ether. The organic layer is separated and washed once with diethyl ether. The organic layers are combined, washed once with brine, dried over magnesium sulfate and concentrated in vacuo to yield 26.43 g of the crude ketone of Formula T-4 as an oily residue. This residue is concentrated in vacuo twice from toluene and then solublized in 500 ml of tetrahydrofuran. The solution is covered with nitrogen and cooled in dry ice:acetone. There is then added 122 ml of 1 M L-Selectride in tetrahydrofuran, dropwise over 40 min. After stirring in the cold for 1 h and while still cold there is added 130 ml of 3 N sodium hydroxide. The mixture is then warmed to 0°C with top water and then held at that temperature with an ice bath. There is then added 130 ml of 30% hydrogen peroxide over 10 min. The temperature is maintained at 40-45°C during the addition. The ice bath is allowed to melt and the reaction warmed to room temperature and stirred for 16 h. With mechanical stirring the reaction is cooled to 10°C with an ice bath and there is added over 10 min 260 ml of 3 N hydrochloric acid. The cold bath is removed and the mixture is stirred at room temperature for 2.5 h. Brine and diethyl ether are then added to effect a two phase mixture. The aqueous layer is separated and extracted twice with diethyl ether. The organic layers are combined, washed with brine, dried over magnesium sulfate and concentrated in vacuo. The residue is chromatographed over a 3000 ml silica gel column (eluted with 10% ethyl acetate:methylene chloride and 31 ml fractions are collected. Fractions 280-444 are combined to yield crude product. Fractions 445-522 are combined to yield 1.64 g of a mixture that is rechromatographed over a 175 ml silica gel column (eluted with 10% ethyl acetate:methylene chloride) and 4.8 ml fractions are collected. Fractions 116-182 are combined and then combined with crude product from the initial column. These combined products are recrystallized from diethyl ether:pentane to yield 7.668 g, m.p. 67-71°C; 1.295 g, m.p. 62-69°C; and 1.173 g, m.p. 67-70°C of the title product. The analytical sample is crystallized from diethyl ether and has m.p. 76-77°C and $[\alpha]_D$-17°C (ethanol). The structure is supported by IR, NMR and mass spectra.

Physical characteristics are as follows:

Anal. Found: C, 67.68: H, 9.51.

Preparation 44 (2S,4R,5R)-4,5-Dihydroxy-2-isopropyl-6-cyclohexylhexanoic acid, γ-lactone (Formula T-10) Refer to Chart T.

A. (Formula T-6) Refer to Chart T.

To an nitrogen covered solution of 9.836 g of the hydroxy lactone of Preparation 43 in 290 ml of methylene chloride is added 5.26 ml of dihydropyran followed by 0.96 g of pyridinium tosylate. After stirring at room temperature for 2.5 h there is added an additional 1.1 ml of dihydropyran. Then after 4.5 h there is added an additional 0.2 g of pyridinium tosylate. After a total of 6.5 h the reaction mixture is concentrated to about one-quarter volume in vacuo. The residue is diluted with 350 ml of diethyl ether, washed in turn with dilute brine, aqueous sodium bicarbonate and again with dilute brine, dried over magnesium sulfate and concentrated in vacuo. The residue is chromatographed over a 2000 ml silica gel column (elution with 2% ethyl acetate: methylene chloride to fraction 320 and then 4% ethyl acetate:methylene chloride). Thirty-five ml fractions are collected to fraction 290 and then 275 ml fractions are collected. Fractioned 295-337 are combined to yield 13.203 g of the tetrahydropyranyl ether. Mass spectrum supports the proposed structure.

B. (Formula T-7) Refer to Chart T.

To an nitrogen covered, dry ice:acetone cooled solution of 6.87 ml of diisopropyl amine in 40 ml of tetrahydrofuran is added 30.63 ml of 1.6 N n-butyllithium in hexane dropwise over 12 min. After stirring in the cold for 15 min there is added a solution of 13.20 g of the tetrahydropyranyl lactone of Part A in 40 ml of tetrahydrofuran over 20 min. Then after stirring for an additional 25 min] there is added 6.54 ml of acetone over 6 min. Following an additional 30 min of stirring in the cold there is added dropwise 18.7 ml of 2.4 N hydrochloric acid. The cold bath is removed and the mixture is stirred at room temperature for 1.25 h. There is then added 200 ml of diethyl ether. The organic layer is separated, washed with brine, dried over magnesium sulfate and concentrate in vacuo to yield 17.38 g of crude product. The structure is supported by a mass spectrum.

C. (Formula T-8) Refer to Chart T.

To an nitrogen covered ice bath cooled solution of 17.30 g of the crude tertiary alcohol from Part B in 435 ml of pyridine is added 86.37 of thionyl chloride dropwise over 35 min. After stirring in the cold for 3.5 h the reaction mixture is poured into 1 L. of ice-water at a rate to keep the temperature below 15° C. The mixture is extracted with three 600 ml portions of diethyl ether. The combined extracts are washed once with brine, dried over magnesium sulfate and concentrated in vacuo. The residue is chromatographed over a 2000 ml silica gel column (elution with 5% acetone:methylene chloride) and 300 ml fractions are collected. Fractions 25-57 are combined to yield 12.40 g of unsaturated product. The structure is supported by NMR and mass spectra.

D. (Formula T-9) Refer to Chart T.

A mixture of 0.5 g of the alkene of Part C and 0.11 g of 10% Pd/C in 30 ml of absolute ethanol is hydrogenated at atmospheric pressure for 3 hrs. The catalyst is removed by filtration and the filtrate is concentrated in vacuo. The residue is chromatographed over a 150 ml silica gel column (elution with 5% ethyl acetate: hexane) and 4.7 ml fractions are collected. Fractions 255-420 are combined to yield 0.410 g of the product as a gum. The structure is supported by NMR and mass spectra.

E. (Formula T-9) Refer to Chart T.

A mixture of 11.4 g of the alkene of Part C and 2.5 g of 10% Pd/C in 150 ml of absolute ethanol is hydrogenated under an initial pressure (of 40 psi) on a Parr apparatus for 2 h. The catalyst is removed by filtration and the filtrate is concentrated in vitro to yield 10.92 g of the crude product as a gum. The NMR compares with the product from Part D.

F. (Formula T-10) Refer to Chart T.

To an nitrogen covered solution of 11.82 of the tetrahy dropyranyl ether of Part D or E in 280 ml of absolute ethanol is added 1.14 g of pyridinium tosylate. The solution is heated at 50° C for 2.75 h and then concentrated in vacuo. The residue is chromatographed over a 1000 ml silica gel column (elution with 0.75% acetone:methylene chloride to fraction 241; 1% acetone:methylene chloride to fraction 432 and then 2% acetone:methylene chloride) and 432 twenty-six ml, 3 five hundred ml and 2 one thousand ml fractions are collected. Combined fractions from 200 to the end and recrystallized from diethyl ether to yield 1.60 g, m.p. 106-108° C, 1.59 g, m.p. 106-107.5, 1.63 g, m.p. 106.5-108.5° C and 1.20 g, m.p. 104-107.5° C of the title product. The remaining material from the above recrystallization is rechromatographed over a 250 ml silica gel column (elution with 10% ethyl acetate:hexane) and 13.2 ml fractions are collected. The more polar material, fractions 217-290, is recrystallized from diethyl ether to yield 0.132 g, m.p. 105-107° C and 0.133 g, m.p. 100-104.5° C of additional product. The analytical sample has m.p., 106-108° C and $[\alpha]_D$-22° C (ethanol). The IR and mass spectra support the proposed structure. The assigned stereochemistry is established by an NOE-Difference experiment.

Physical characteristics are as follows:

Anal. Found: C, 70.52; H, 10.25.

The less polar material from the second column, fractions 112-146, is recrystallized from diethyl etherpentane to give 0.20 g, m.p. 120-121 and 0.089 g, m.p. 118-120° C. The analytical sample has m.p. 120-121° C and $[\alpha]_D$-10° C (ethanol). The IR, NMR and mass spectra are identical to those of the (2S,4S,5S) enantiomer (Formula T-10, refer to Chart T)

Physical characteristics are as follows:

Anal. Found: C, 71.46, 71.58; H, 10.20, 10.50.

Fractions 147-216 contain a mixture that amounts to 1.13 g.

Preparation 45 (2S,4S,5S)-4,5-Dihydroxy-2-isopropyl-6-cyclohexylhexanoic acid, γ-lactone (Formula T-12) Refer to Chart T.

A. (Formula T-11) Refer to Chart T.

To an nitrogen covered, ice methanol cooled solution of 6.30 g of the hydroxy lactone of Preparation 44 in 75 ml of methylene chloride is added a solution of 2.1 ml of methanesulfonyl chloride in 13 ml of methylene chloride over 8 min. A heavy precipitate comes out of solution. The cold bath is removed and the mixture is stirred at room temperature for 1.25 h. The reaction mixture is then poured into 50 ml of brine. The aqueous layer is separated and extracted once with methylene chloride. All of the organic fractions are combined, washed in turn with 1N hydrochloric acid, aqueous sodium bicarbonate and dilute brine, dried over magnesium sulfate and concentrated in vacuo to yield 9.03 g of crude product as a gummy residue. The structure is supported by NMR and I.R. spectra.

B. (Formula T-12) Refer to Chart T.

To an nitrogen covered solution of 9.03 g of the crude mesylate from Part A in 63 ml of acetonitrile is added a solution of 4.41 g of potassium hydroxide and 0.13 g of tetrabutylammonium hydrogen sulfate in 13 ml of water over 7 min. The two phase mixture is vigorously stirred for 22 h and then cooled in an ice bath. There is then added 9.2 ml of glacial acetic acid over 3 min followed by 3.3 ml of concentrated hydrochloric acid over 1 min. The ice bath is removed and the mixture is stirred at room temperature for 3.5 h and then poured into 50 ml of brine. Diethyl ether (100 ml) is added and the two phases are mixed well. The aqueous layer is separated and extracted twice with diethyl ether. The organic fractions are combined, washed with brine, dried over magnesium sulfate and concentrated in vacuo. The residue is then concentrated in vacuo twice from toluene. The crystalline residue is recrystallized from diethyl ether to yield the following six crops of crystalline title product: 1.014 g, m.p. 119.5-120.5° C; 1.061 g, m.p. 120-120.5° C; 0.824 g, m.p. 119-120° C; 0.698 g, m.p. 118.5-119° C; 0.846 g, m.p. 118-119° C; 0.454 g, m.p. 114-117° C.

The analytical sample has m.p. 119-120 and $[\alpha]_D$+10° C (ethanol). The structure is supported by IR, NMR and mass spectra, and the stereochemistry is established by an NOE-Difference experiment.

Physical characteristics are as follows:

Anal. Found: C, 70.81; H, 10.73.

Preparation 46 (2S,4S,5R)-5-Chloro-6-cyclohexyl-4-hydroxy-2-isopropylhexanoic acid, γ-lactone (Formula T-13) Refer to Chart T.

To an nitrogen covered, ice-bath cooled solution of 8.09 g of triphenylphosphine in 50 ml of carbon tetrachloride is added a solution of 5.231 g of the alcohol of Preparation 45 in 50 ml of acetonitrile over 13 min. After stirring in the cold for 1.75 h the ice bath is removed and the mixture is stirred at room temperature for 21 h. The residue following concentration in vacuo is dissolved in methylene chloride and washed once with aqueous sodium bicarbonate and once with water. The organic fraction is dried over magnesium sulfate, and diluted to 1000 ml with hexane. A precipitate is collected on a filter and washed with 5% ethyl acetate:hexane. The combined filtrates are concentrated in vacuo. The residue is treated with 250 ml of 5% ethyl acetate:hexane, slurried and then filtered. the solid filter cake is washed with 5% ethyl acetate:hexane and the combined filtrates are concentrated in vacuo. The residue is chromatographed over a 600 ml silica gel column (elution with 45% methylene chloride:hexane) and 22 ml fractions are collected. Fractions 68-132 are combined and recrystallized from diethyl ether:pentane to yield 2.610 g, m.p. 81.5-82.5° C, 0.731 g, m.p. 82-83.5° C and 0.327 g, m.p. 82-83° C of the title product. Fractions 133-188 (a mixture) are combined and recrystallized to yield an additional 0.235 g, m.p. 80.5-81.5° C of the title product.

The analytical sample has m.p. 81.5-82.5° C and $[\alpha]_D + 47°$ C (ethanol). The structure is supported by NMR, IR and mass spectra.

Physical characteristics are as follows:

Anal. Found: C, 66.18; H, 9.72; Cl, 1301.

Preparation 47 (2S,4S,5S)-5-(tert-Butoxycarbonylamino)-6-cyclohexyl-4-hydroxy-2-isopropylhexanoic acid, γ-lactone (Formula T-16) Refer to Chart T.

A. (Formula T-14) Refer to Chart T.

A mixture of 3.626 g of the chlorolactone of Preparation 46 and 2.59 g of sodium azide in 40 ml of DMSO is heated under nitrogen in an oil bath at 85-90° C. Within 10 min everything is in solution. After heating for a total of 26 h the reaction mixture is allowed to cool and poured into 200 ml of ice-water. The mixture is extracted with four 100 ml portions of ethyl acetate. The combined extracts are washed once with water, once with brine, dried over magnesium sulfate and concentrated in vacuo. The residue is chromatographed over a 400 ml silica gel column (elution with 2.5% ethyl acetate:hexane) and 394 fourteen ml fractions and six 250 ml fractions are collected. Fractions 197-400 are combined to yield 2.822 g of the title product. The structure is supported by NMR and mass spectra.

B. (Formula T-15) Refer to Chart T.

A mixture of 2.822 g of the azide of Part A and 1.0 g of 5% Pd/C in 100 ml of absolute ethanol is hydrogenated at atmospheric pressure. At intervals of 15 min, 1.5 h, 2.75 h and 3.5 h the system is evacuated and rechanged with hydrogen. After 4.25 h the catalyst is removed by filtration and the filtrate concentrated in vacuo. A solution of the residue in ethyl acetate is washed once with aqueous sodium bicarbonate, once with brine, dried over magnesium sulfate and concentrated in vacuo. The residue is concentrated two additional times from toluene to yield 2.8 g of crude amine. The structure is supported by NMR and mass spectra.

C. (Formula T-16) Refer to Chart T.

To an nitrogen covered solution of 2.8 g of crude amine from Part B in 60 ml of tetrahydrofuran is added a solution of 5.51 g of di-tert-butyl dicarbonate in 50 ml of tetrahydrofuran over 8 min. The solution is stirred at room temperature for 18.75 h and then concentrated in vacuo. A solution of the residue in ethyl acetate is washed once with aqueous sodium bicarbonate, once with brine, dried over magnesium sulfate and concentrated in vacuo. The residue is chrmatographed over a 600 ml silica gel column (elution with 1% ethyl acetate:methylene chloride) and 22.5 ml fractions are collected. Fractions 90-250 are combined and

recrystallized from diethyl ether:pentane to yield 1.35 g, m.p. 106.5 °C-108.5 °C of crystalline title product. The mother liquors are concentrated in vacuo to yield 1.48 g of additional title product. The analytical sample has m.p. 106.5-108.5 °C and $[\alpha]_D$-32 °C(ethanol). The NMR, IR and mass spectra support the proposed structure.

Physical characteristics are as follows:
Anal. Found: C,67.70; H, 10.27; N, 3.93.

Preparation 48 (2S,4S,5S)-5-(tert-Butoxycarbonylamino)-4-(tert-butyldimethylsilyloxy)-6-cyclohexyl-2-isopropylhexanoic acid (Formula T-18) Refer to Chart T.

A. (Formula T-17) Refer to Chart T.

To a solution of 3.8 g of the Boc amino lactone of Preparation 47 in 167 ml of acetonitrile and 133 ml of water is added 12.2 ml of the 0.97 N potassium hydroxide. The cloudy solution becomes clear after stirring at room temperature for 20 min. After stirring for 21 h the reaction mixture is concentrated in vacuo to an aqueous residue. This latter residue is then lyophilized.

To an nitrogen covered solution of the residue from the lyophilization in 75 ml of dimethylformamide is added 3.75 g of imidzaole and 4.16 g of tert butyldimethylsilyl chloride. The cloudy solution is stirred at room temperature for 72 h and treated with 100 ml of hexane. The lower phase is separated and extracted two additional times with hexane. The hexane fractions are combined, washed with brine, dried over magnesium sulfate and concentrated in vacuo to yield 6.01 g of crude disilylated product. The NMR supports the structure.

B. (Formula T-18) Refer to Chart T.

To an nitrogen covered solution of 3.35 g of crude disilylated product from Part A in 50 ml of methanol is added a solution of 0.85 g of potassium carbonate in 4 ml of water over 1.5 min. A resulting suspension is stirred at room temperature for 40 min and then concentrated in vacuo to an aqueous residue. A mixture of this residue in 16 ml of brine and 25 ml of ethyl acetate is cooled in an ice-bath and treated with 25 ml of 0.5 M potassium bisulfate at a rate to keep the temperature below 10 °C. After mixing well the aqueous layer is separated and extracted twice with ethyl acetate. The organic layers are combined, washed with brine, dried over magnesium sulfate and concentrated in vacuo. The residue is chromatographed over a 350 ml silica gel column (elution with 3% methanol:methylene chloride) and 12.6 ml fractions are collected. Fractions 57-84 are combined and recrystallized from acetonitrile to give 1.54 g, m.p. 156.5-159 °C, 0.299 g, m.p. 156-159 °C and 0.074 g, m.p. 155-157 °C of crystalline title product. The analytical sample has m.p. 156.5-159 °C and $[\alpha]_D$-31 °C (ethanol). The structure is supported by NMR, IR and mass spectra.

Physical characteristics are as follows:
Anal. Found: C, 64.51; H, 10.28; N, 2.96.

Preparation 49 [Tris(trimethylsilyloxymethyl)]-methyl carbamic acid, phenyl ester

A solution of 24.2 g of tris(hydroxymethyl)aminomethane in 1250 ml of tetrahydrofuran is treated with 121.4 g of N-methyl-N-(trimethylsilyl)trifluoroacetamide (MSTFA), and the resulting mixture is stirred at 25° for 22-24 h (sustained but gentle exotherm). The reaction mixture is treated with 16.9 ml of pyridine, cooled to 3-7 °C in an ice bath, and finally treated dropwise over 15 min with a solution of 25.0 ml of phenyl chloroformate in a small amount of tetrahydrofuran. The mixture is stirred for 30 min at 5°, then 5-6 h at room temperature. the solids (pyridine hydrochloride) are removed by filtration, and the filtrate is concentrated in vacuo. The material is maintained on a Kugelrohr apparatus (0.2 mm) until excess MSTFA has been removed. The remaining viscous residue weighs 89.4 g and, due to its hydrolytic instability, is used in subsequent coupling reactions without further purification.

Physical characteristics are as follows:
NMR ($\delta$, $CDCl_3$): 7.3-6.9, 3.66, 0.00.
$^{13}C$-NMR shows the presence of numerous minor impurities in this sample.

Example 46 N-[[[2-Hydroxy-1,1-bis(hydroxymethyl)ethyl]-amino]carbonyl]-Pro-Phe-N(Me)His-LVA-Ile-AMP, pyridine N-oxide (Alternative Process for Making the Compound of Formula B-4)

A stirred solution of 465 mg of Pro-Phe-N(Me)His-LVA-Ile-AMP, prepared as described in Example 4, Part A, and 0.56 ml of triethylamine in 4 ml of anhydrous dimethylformamide is treated dropwise with 1.28 g of the tris(trimethylsilyloxymethyl) urethane of Preparation 49, and the resulting mixture is stirred in a nitrogen atmosphere at 35° C for 64 h. The dimethylformamide is then removed in vacuo, and the residue is dissolved in 20 ml of methanol containing a trace of ammonia (pH 9-10). The solution is stirred and treated with 2 ml of 1 M hydrochloric acid/methanol. The resulting solution has a pH under 1. After 1 h at 25°, 5 ml of 1 M aqueous potassium hydroxide is added, and the methanol is removed under pressure. The residue is taken up in brine and chloroform and then extracted with three 100 ml portions of chloroform. The extracts are dried over anhydrous sodium sulfate and evaporated, thereby affording to crude product weighing 902 mg. TLC analysis (16% 7M ammonia-methanol/84% trichloromethane) showed no starting peptide remaining; the major product is THAM urea, the title product of Example 2, contaminated with 1-5% of what appears to be the less polar oxazoline impurity based on 'TLC mobility.

The crude product is dissolved in 10 ml of chloroform and treated with 133 mg of m-chloroperbenzoic acid added in one portion. After 60 min in at 25°, TLC analysis (16% 7 M ammonia-methanol/84% trichloromethane) shows about 90% title product and 10% of something which appears to be starting material. An additional 20 mg of m-chloro-perbenzoic acid is added., but after 30 min, TLC indicates that the reaction has progressed no further. The reaction mixture is evaporated to dryness in vacuo, and the residue is taken up in 12% 7 M ammonia-methanol/88% trichloromethane (the anticipated chromatography solvent). Part of the product dissolves readily, leaving behind a white, easily-filtered solid. The solids are filtered off using a medium-porosity sintered glass funnel and washed with three 2 ml portions of the 12% mixed solvent. Concentration of the combined filtrate yields 900 mg of the crude product. (TLC analysis of the solids shows that they are excess m-chloro-perbenzoic acid and the corresponding reduced acid, and contains none of the desired product.)

The crude product is chromatographed on a column containing 125 g of 40-60 silica gel, packed and eluted with 12% 7 M ammonia-methanol/88% trichloromethane (12 ml fractions). Fractions 76-102 are combined and evaporated. Three 4 ml portions of chloroform are sequentially added and evaporated to insure complete removal of the methanol. The product is then dissolved in 4 ml of hot chloroform and added dropwise to 30 ml of vigorously stirred pentane. The solids are filtered off, washed with 10 ml of pentane, and dried superficially by pulling nitrogen through the funnel for 10 min. More thorough drying at 65-70° for 18 h (0.05 mm) affords 210 mg of pure title product, completely homogeneous by TLC (16% 7 M ammonia- methanol/84% trichloromethane).

Physical characteristics are as follows:

Melt solvate: 0.04% chloroform; trace (under 0.01%) pentane.
KF water: 0.99%
Mass spectrum (FAB): $[M+H]^+$ at m/z 993.
UV (ethanol): $\lambda_{max}$262 ($\epsilon$ = 11,220) and 210 nm ($\epsilon$ = 42,400).
$[\alpha]_D$ (methanol): -87°.
Anal. Found: C, 59.71; H, 7.75; N, 13.87.
HPLC: Major component 99.36% [30 cm Waters $C_{18}$ column 16; 50% 0.05 M ammonium acetate/25% methanol/25% acetonitrile; 1 ml/min; $t_r$ 9.2 min].

Example 47 N-[[[2-Hydroxy-1,1-bis(hydroxymethyl)ethyl]-amino]carbonyl]-Pro-Phe-N(Me)His-LVA-Ile-AMP, pyridine N-oxide Salts

A. Hydrochloride

A 100 mg sample of the title product of Example 46 in 5 ml of water and 5 ml of 0.02 M hydrochloric acid is stirred until a homogeneous clear solution results. The mixture is lyophilized, and the resulting solids are dried for 18 h at 65-70° C, 0.05 mm, thereby affording 106 mg of the desired hydrochloride salt. After drying, the salt presents no electrostatic handling problems. The salt softens at 145° and becomes completely clear, but not mobile, at 160-170° C.

B. Methanesulfonate;

A 0.02 M solution of methanesulfonic acid in water is prepared (192.2 mg in 100 ml of water). A suspension of 100 mg of the title product of Example 46 in 5 ml of water and 5 ml of the stock methanesulfonic acid solution is stirred until it becomes a clear solution. Lyophilization followed by drying of the solids at 65-70° C for 18 h, 0.05 mm, yields 113 mg of the methanesulfonate salt (no electrostatic problems). The salt softens at 135° and becomes clear, but not mobile, at 160-170°.

C. Fumarate

A mixture of 100 mg of the title product of Example 46 in 5 ml of water and 5 ml of 0.02 M aqueous fumaric acid (232.2 mg fumaric acid in 100 ml of water) is stirred until a homogeneous solution results. Freeze-drying followed by drying of the solids at 65-70° for 18 h, 0.05 mm, afford 114 mg of the desired fumarate salt, which remains very fluffy, electrostatically charged and difficult to handle. (Shrinks at 120°, becomes clear, but not mobile at 165°).

D. Succinate

A mixture of 100 mg of the title product of Example 46, 5 ml of water and 5 ml of 0.02 M aqueous succinic acid (236.2 mg of succinic acid in 100 ml of water) is stirred until it becomes homogeneous. Lyophilization and drying, as above, yields 112 mg of the desired succinate salt. This material shrinks during drying from a very light, fluffy, electrostatically-charged solid into a much denser, low-volume, non-charged, easily-handled amorphous solid, which softens at 125° and becomes clear (not mobile) at 135-145°.

Physical characteristics are as follows:

Hydrochloride: % CHN (corrected for water): C, 58.31; H, 7.45; N, 13.60.

Methanesulfonate: % CHN (corrected for water): C, 56.09; H, 7.37; N, 12.85.

Fumarate: % CHN (corrected for water): C, 58.56; H, 7.50; N, 12.92.

Succinate: % CHN (corrected for water): C, 58.22; H, 7.48; N, 12.66.

This CHN% data was obtained on samples that had been equilibrated for eighteen hours in a 50% relative humidity atmosphere. Water content was measured by Karl Fischer analysis and CHN% figures were corrected for the water content.

## FORMULA CHART

$X\text{-}A_6\text{-}B_7\text{-}C_8\text{-}D_9\text{-}E_{10}\text{-}F_{11}\text{-}G_{12}\text{-}H_{13}\text{-}I_{14}\text{-}Z$ I

$R_{120}N(R_{124})\text{-}C(O)\text{-}$ II

$\text{-}NR_{120}(R_{124})$ III

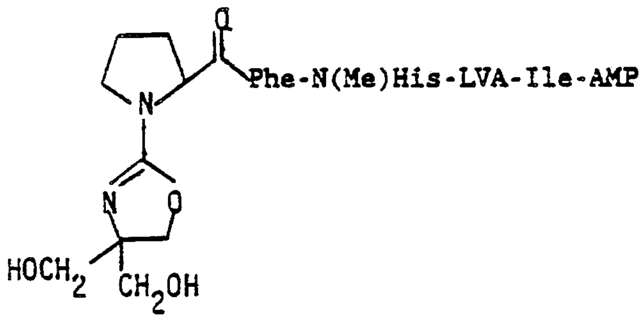

IV

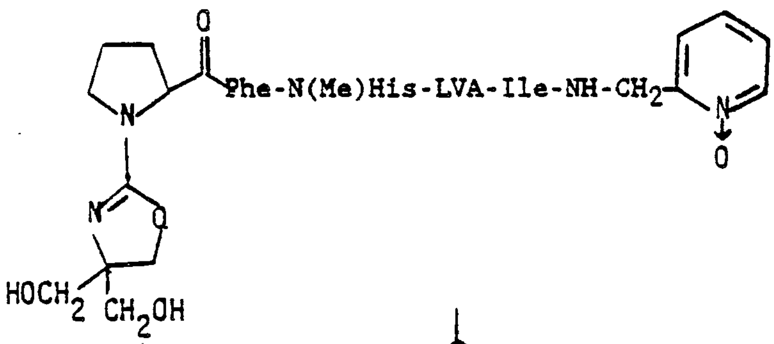

V

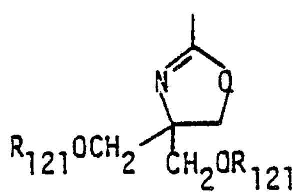

VI

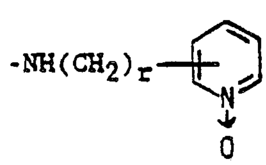

VII

FORMULA CHART (Continued)

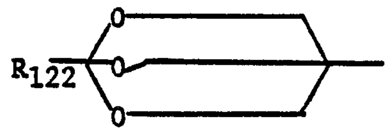

VIII

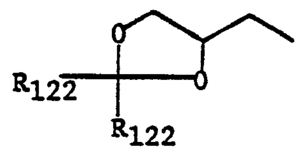

IX

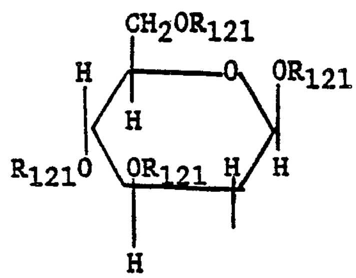

X

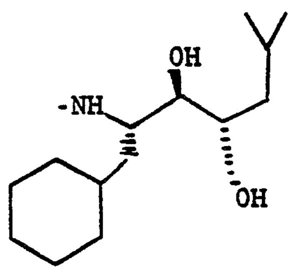

XI

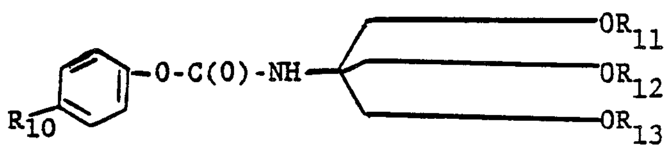

XX

CHART A

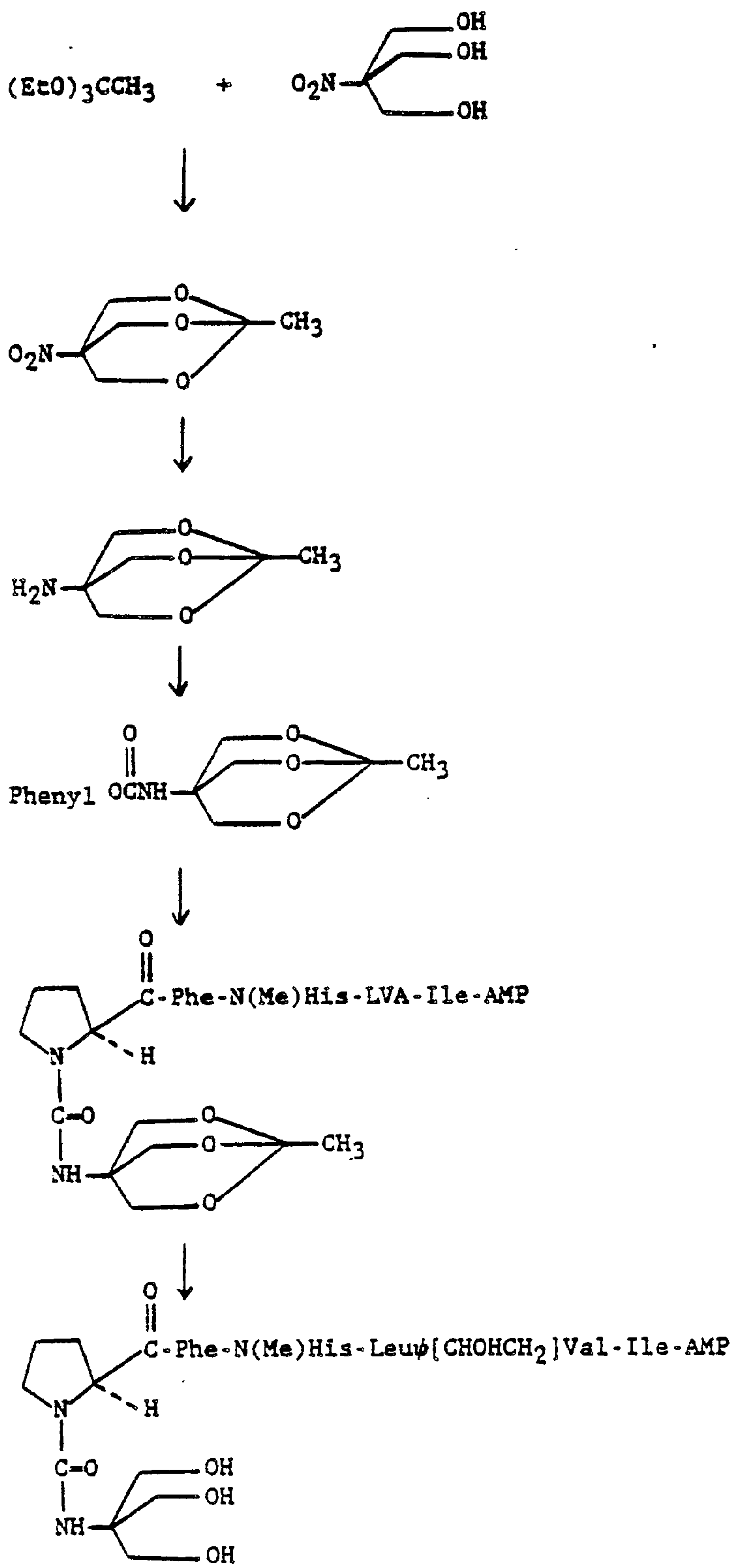

A-1

A-2

A-3

A-4

A-5

A-6

A-7

CHART B

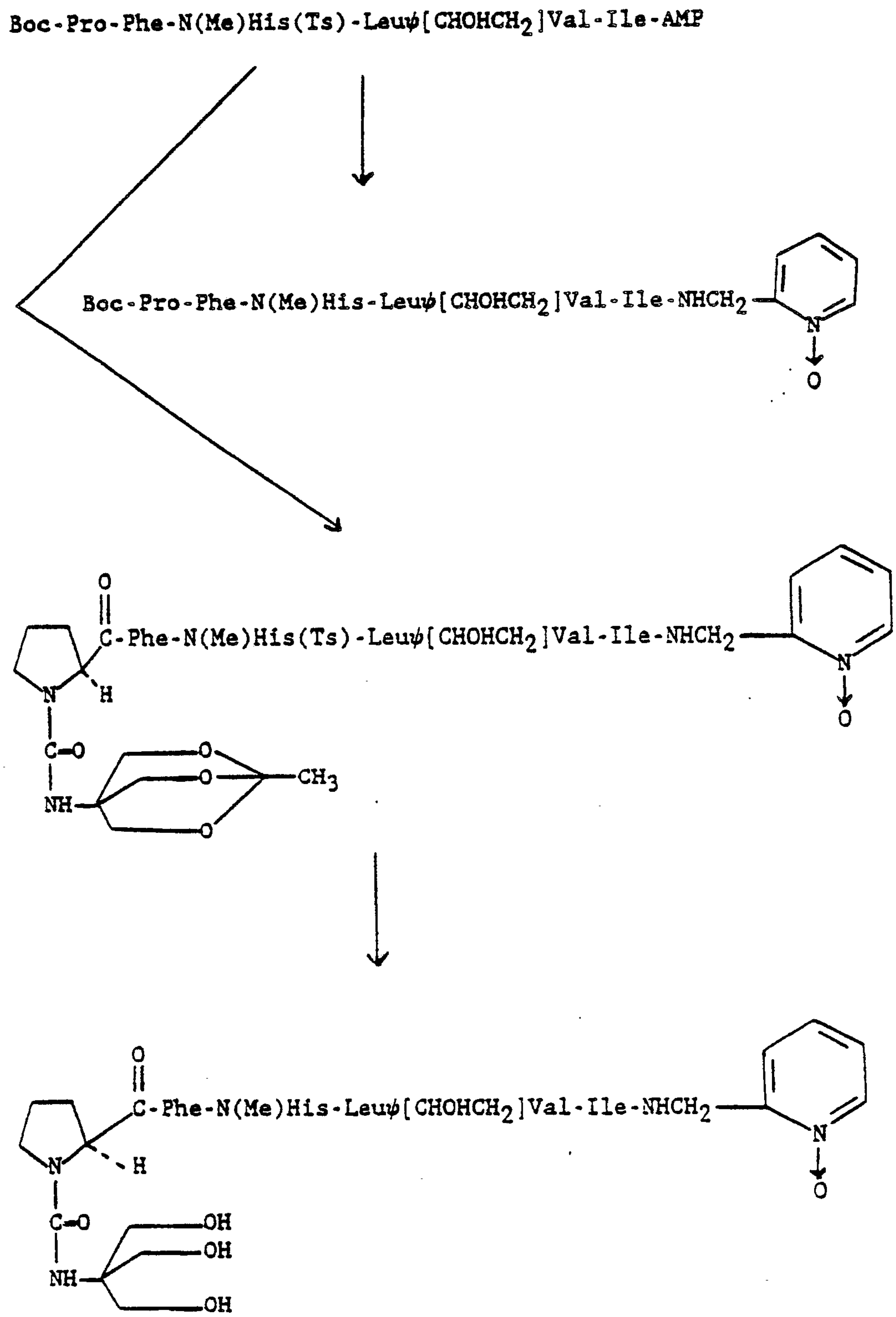

B-1

B-2

B-3

B-4

CHART C

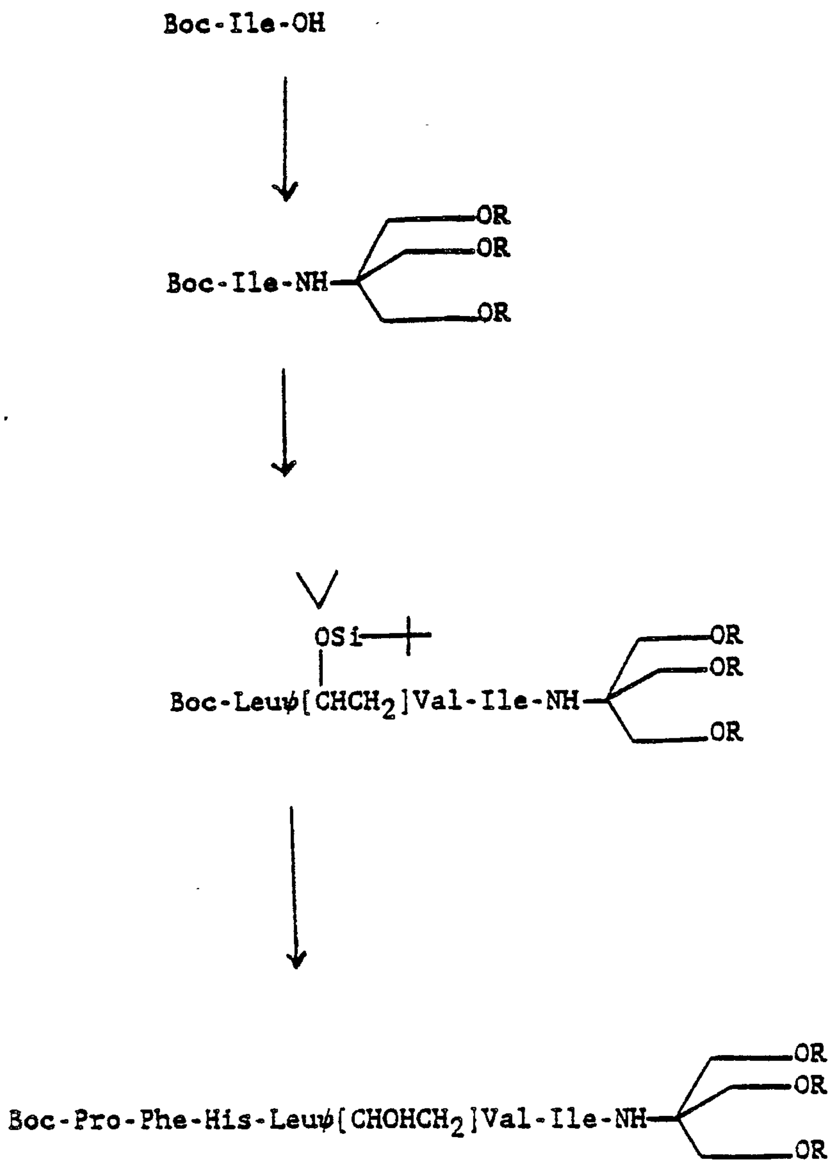

C-1

C-2

C-3

C-4

CHART D

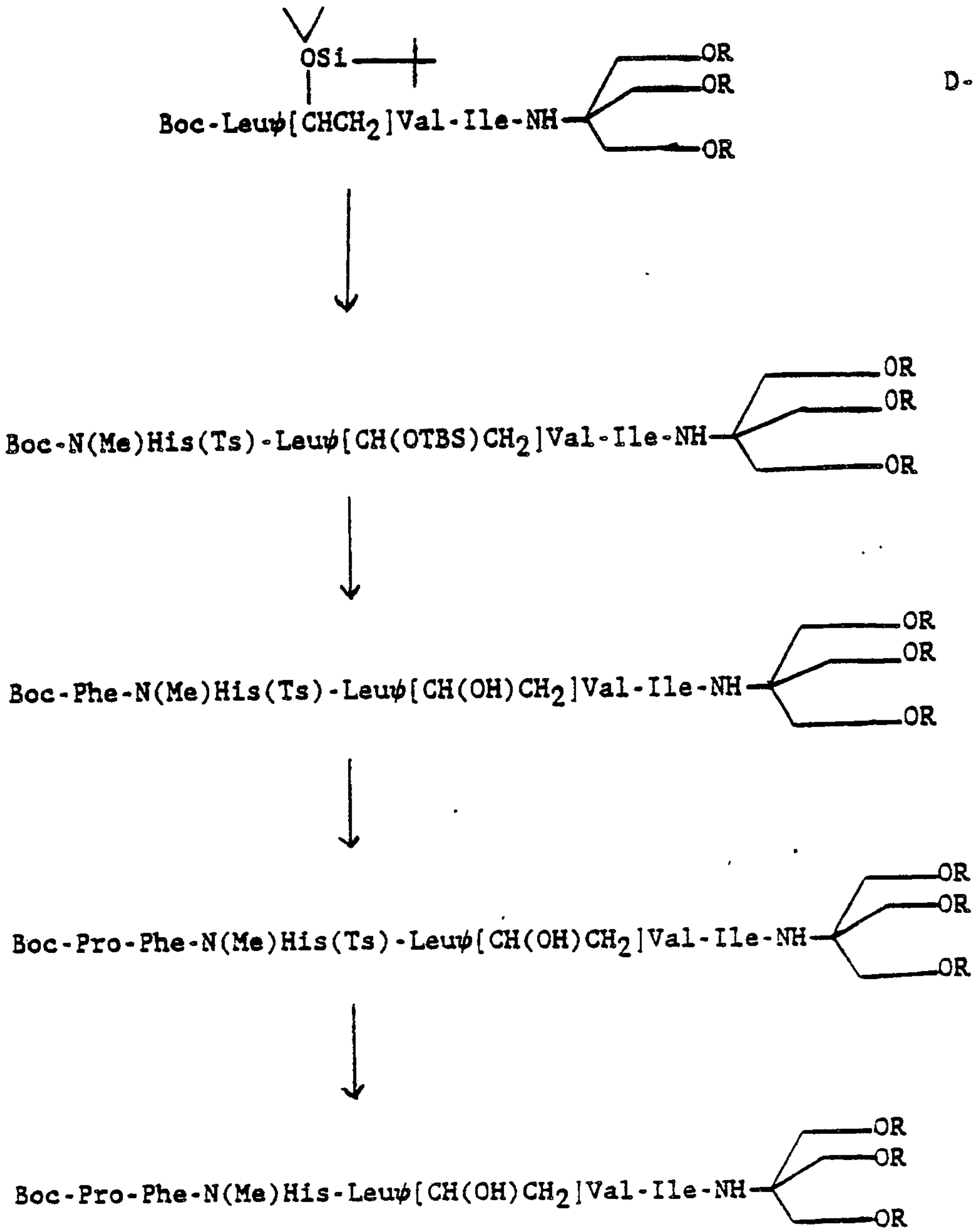

D-1 (C-3)

D-2

D-3

D-4

D-5

CHART E

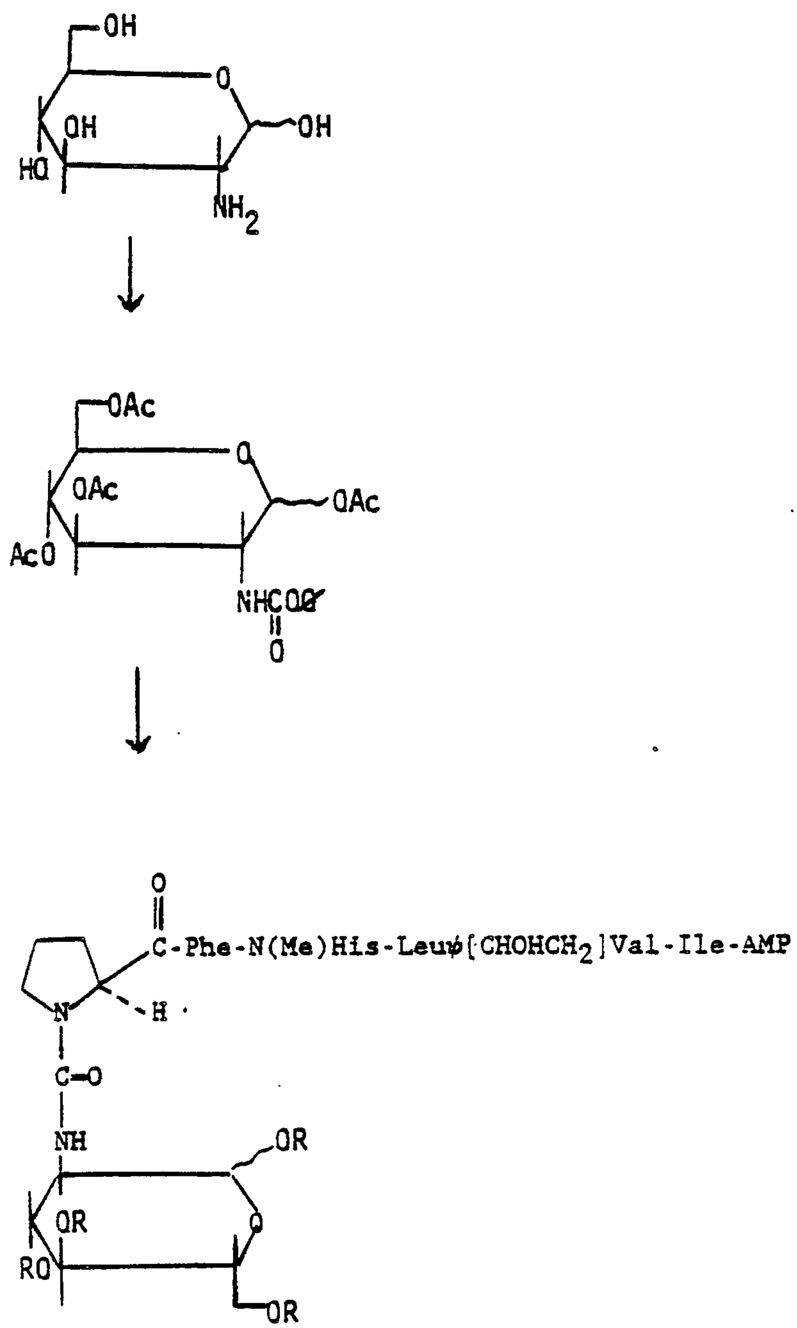

E-1

E-2

E-3

CHART F

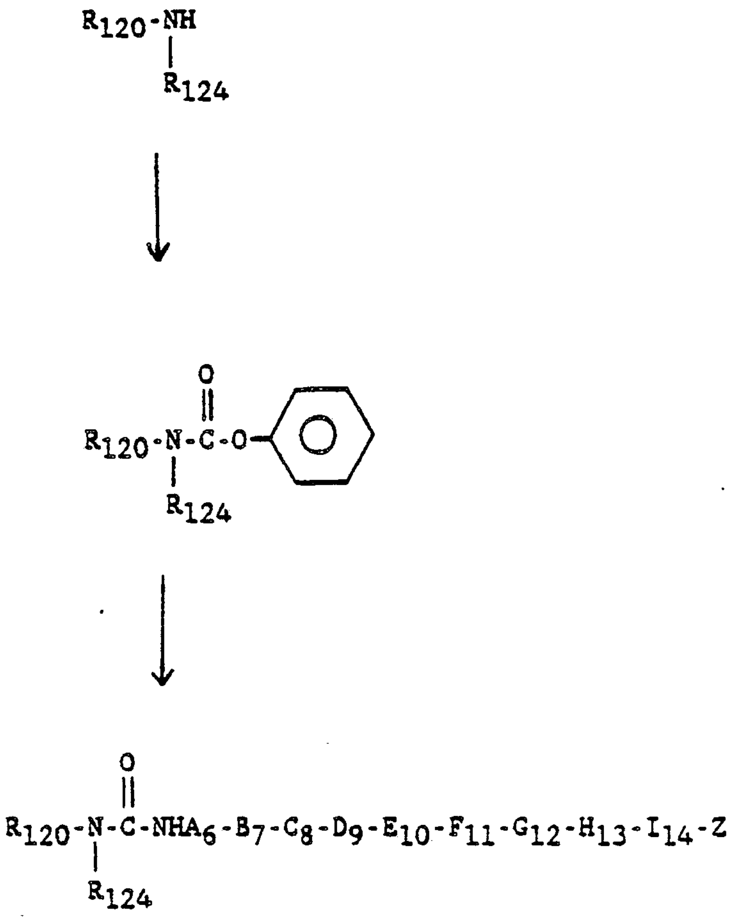

F-1

F-2

F-3

CHART G

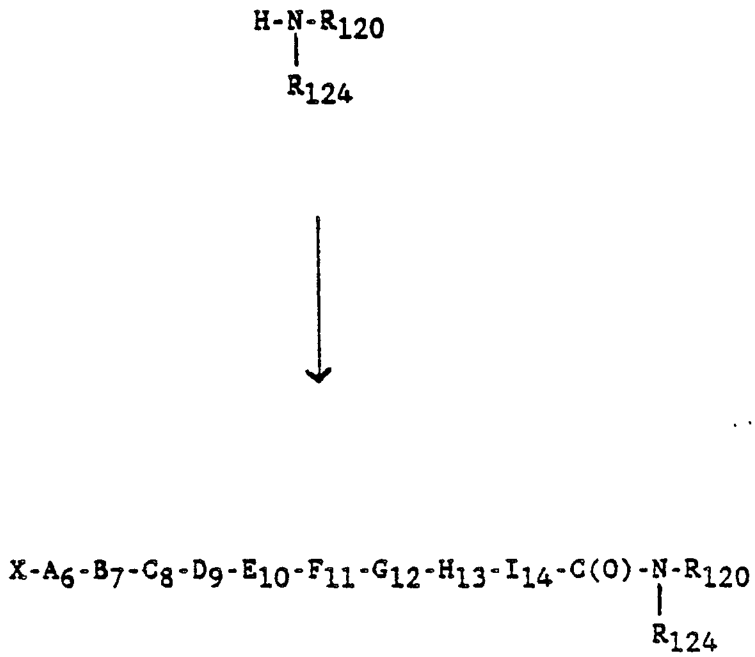

G-1

G-2

CHART H

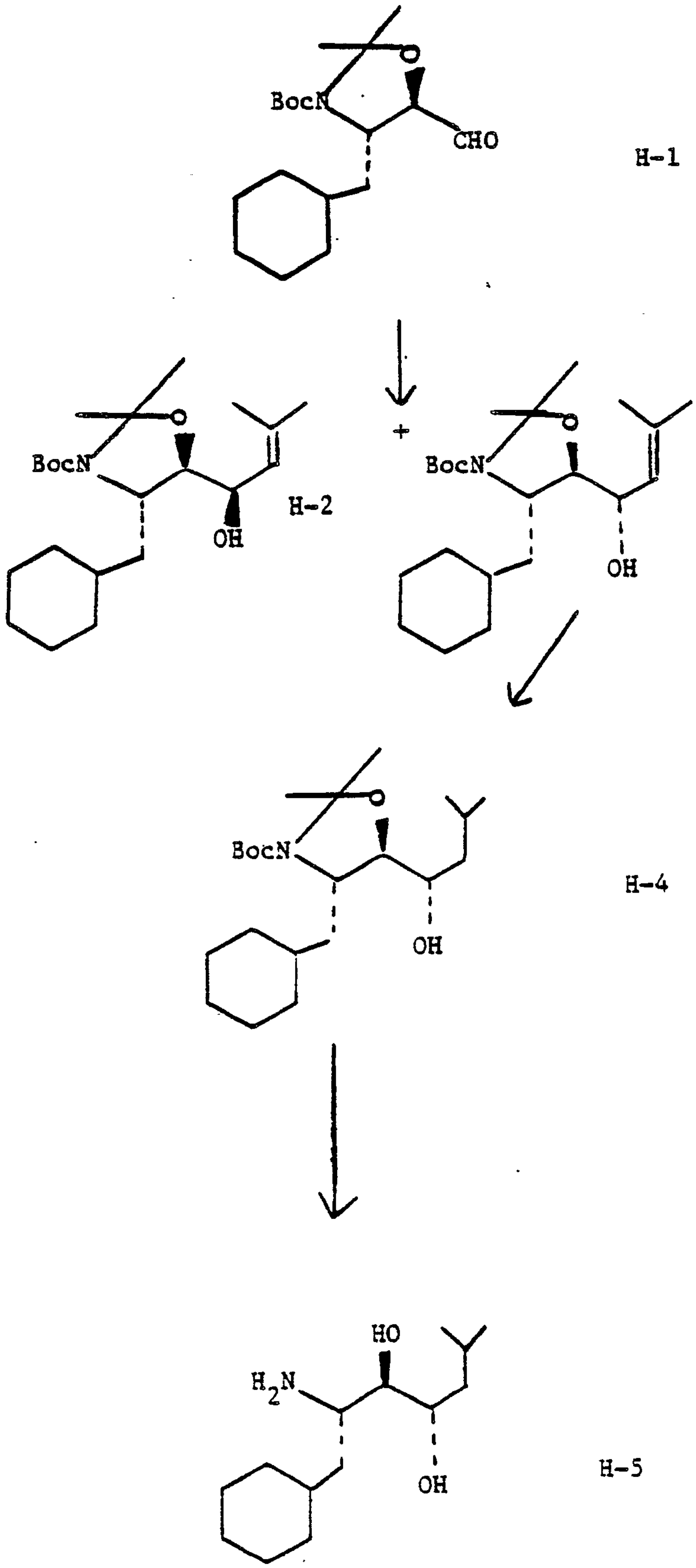

H-1

H-2 + H-3

H-4

H-5

CHART I

I-1

I-2

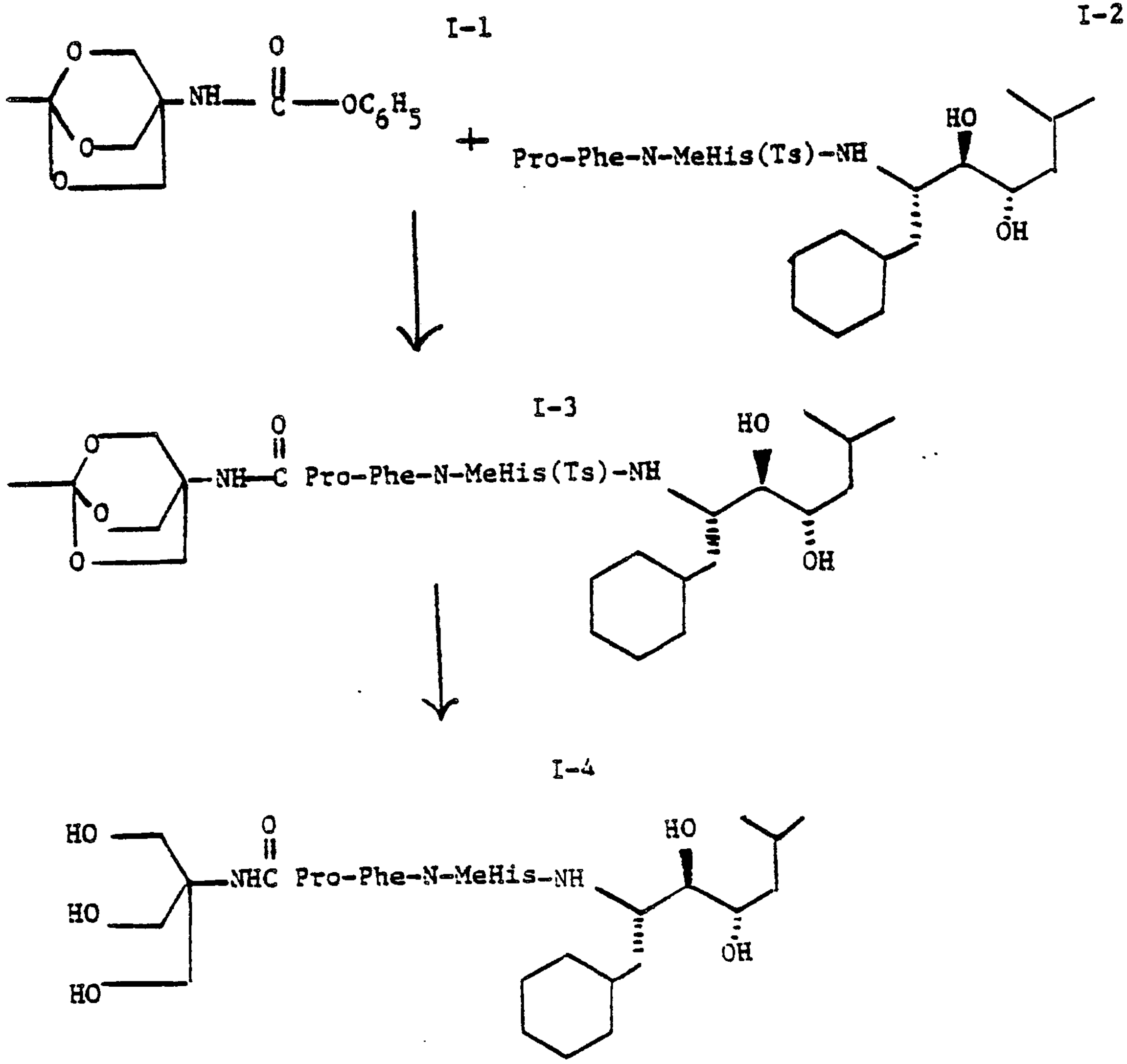

I-3

I-4

CHART J

J-1 J-2

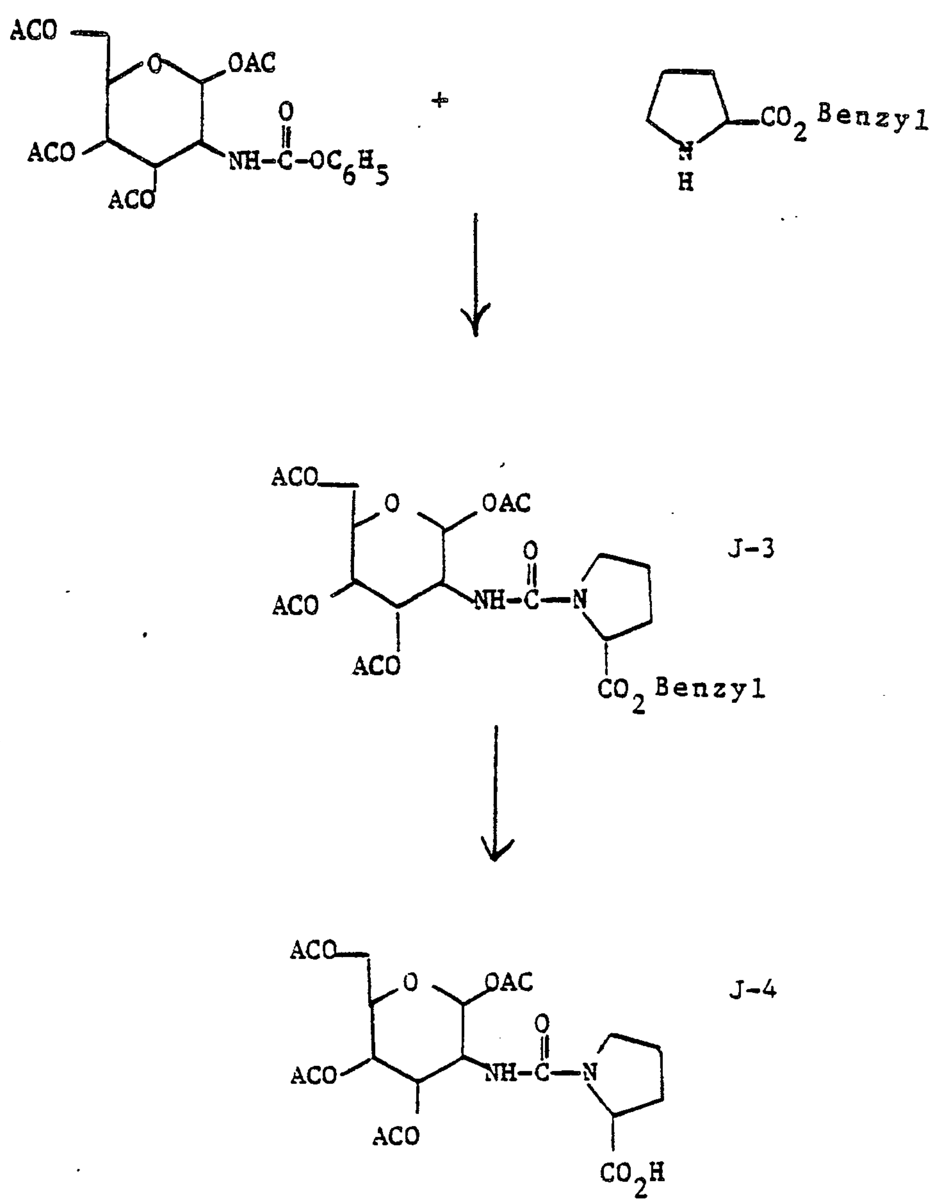

J-3

J-4

CHART K

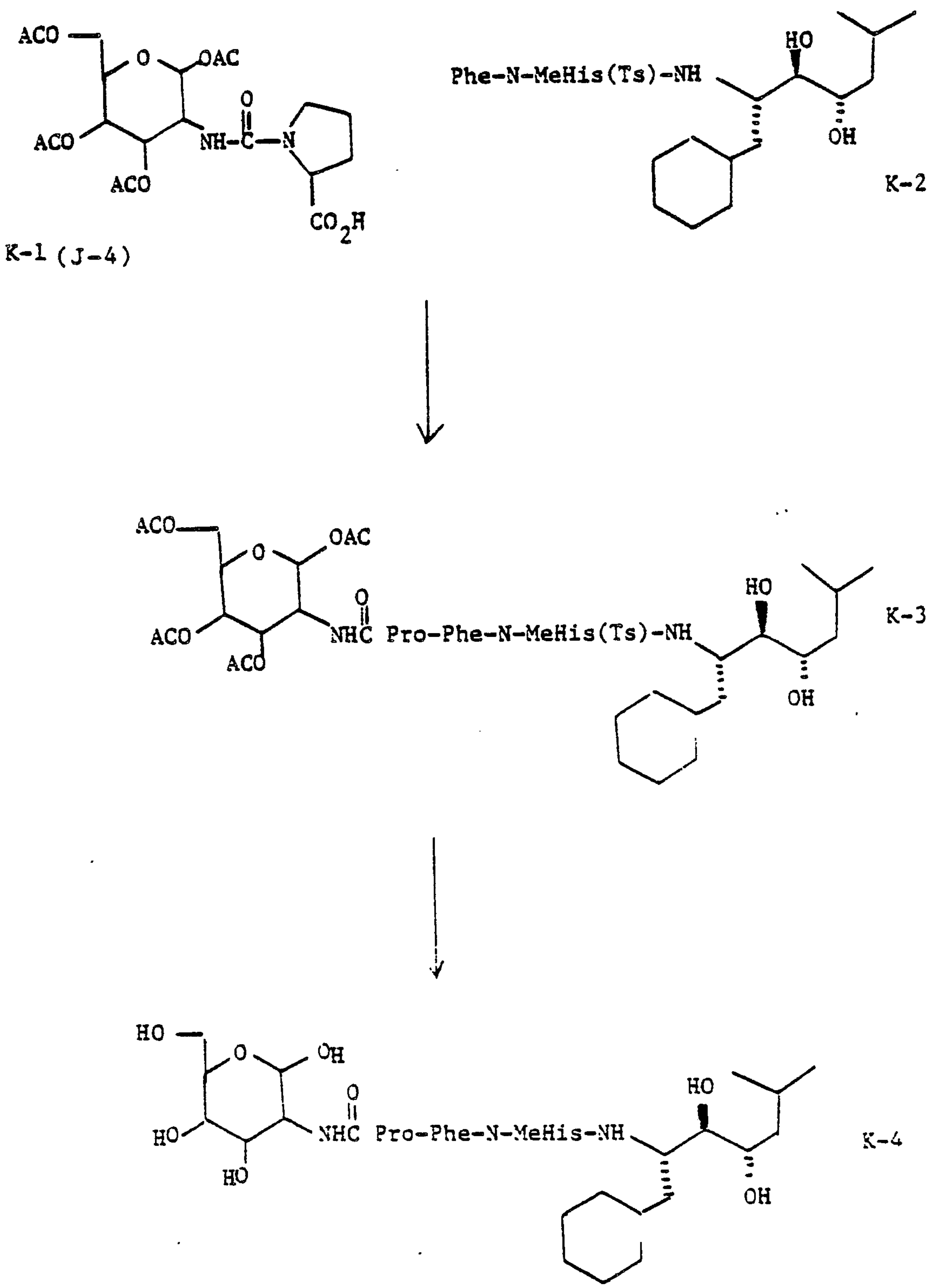

K-1 (J-4)

K-2

K-3

K-4

CHART L

Boc-Phe-N($CH_3$)-His(Ts)-LVA-Ile-AMP L-1

$CF_3CO_2H/CH_2Cl_2$

Phe-N($CH_3$)-His(Ts)-LVA-Ile-AMP L-2

$RCO_2H$/DEPC/DIEA

RCO-Phe-N($CH_3$)-His(Ts)-LVA-Ile-AMP L-3

1-HOBT/THF

RCO-Phe-N($CH_3$)-His-LVA-Ile-AMP L-4

a, R=

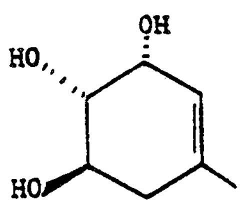

b, R= $(HOCH_2)_2C(CH_3)$-

CHART M

$H_2NCH(CH_2OH)_2$ M-1

(1) phenyl chloroformate

(2) $Ac_2O$/pyridine

Phenyl-OCONHCH$(CH_2OAc)_2$ M-2

Pro-Phe-N$(CH_3)$-His-LVA-Ile-AMP

$Et_3N$/DMF/50°C

$(AcOCH_2)_2$CHNHCO-Pro-Phe-N$(CH_3)$-His-LVA-Ile-AMP M-3

4M $NH_3$/MeOH

$(HOCH_2)_2$CHNHCO-Pro-Phe-N$(CH_3)$-His-LVA-Ile-AMP M-4

MCPBA

$(HOCH_2)_2$CHNHCO-Pro-Phe-N$(CH_3)$-His-LVA-Ile-AMP(N-oxide) M-5

CHART N

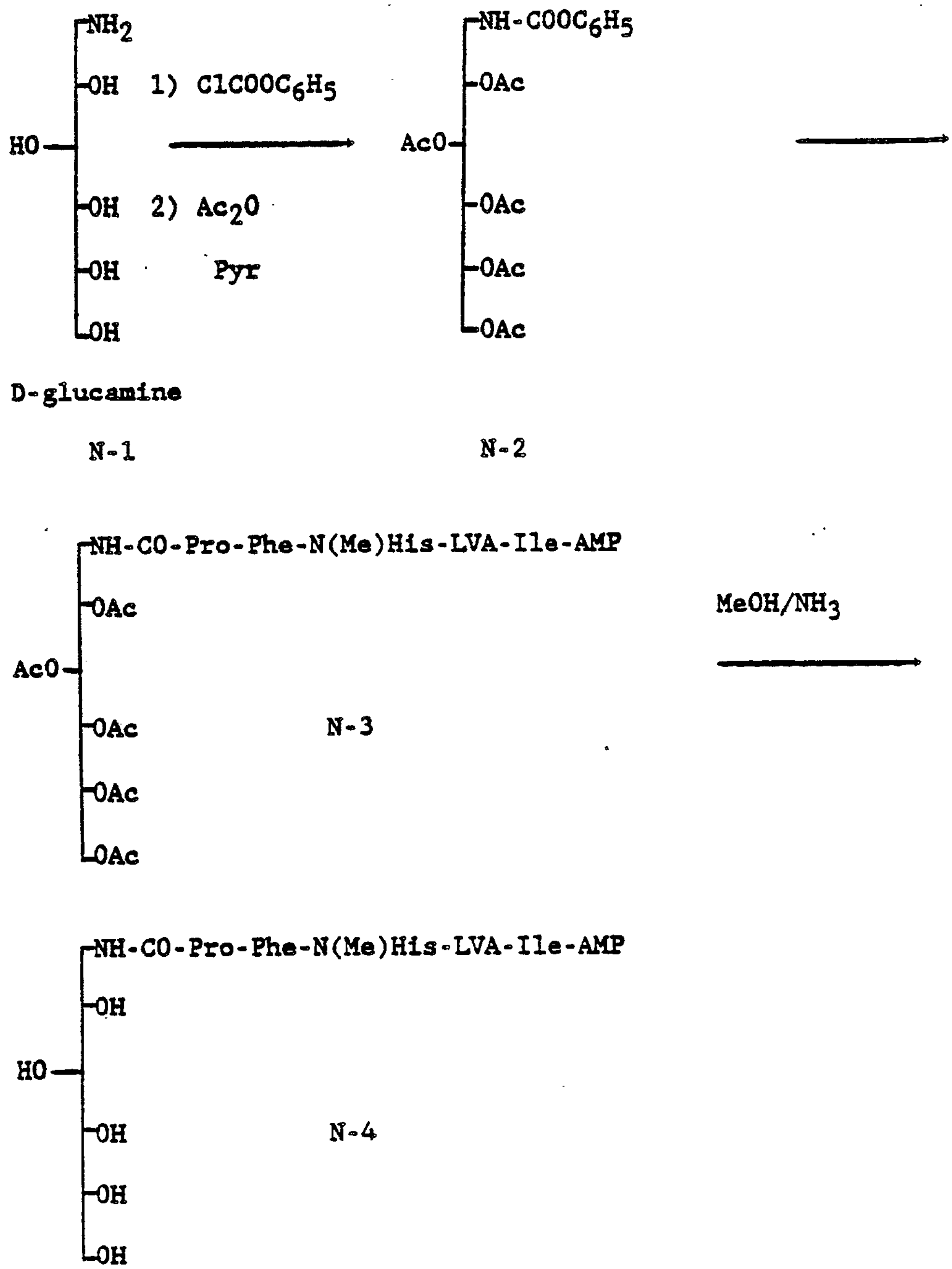


D-glucamine

N-1

N-2

N-3

N-4

CHART O

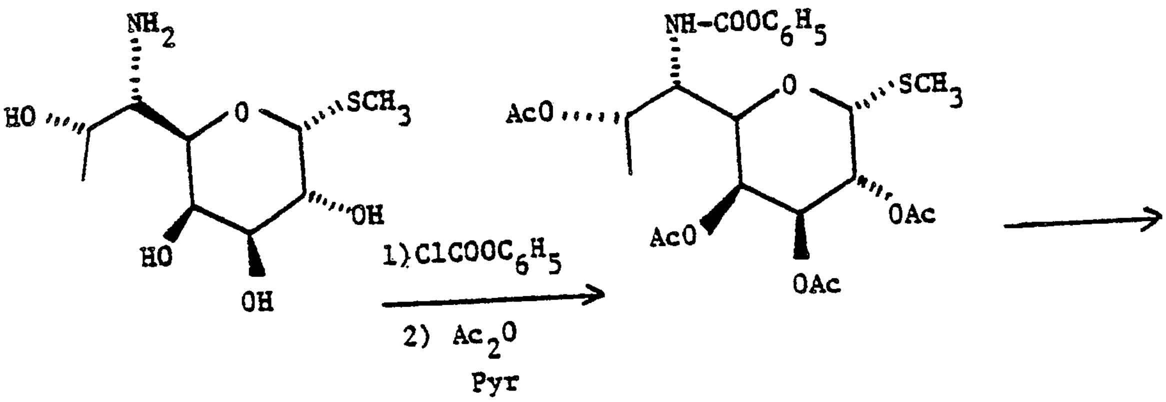


O-1

O-2

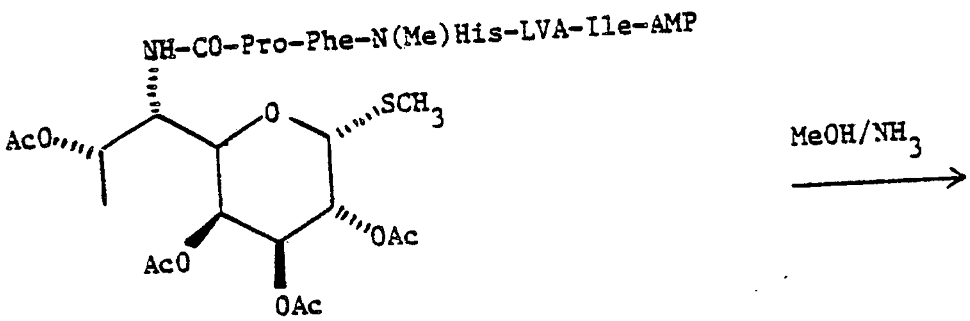


O-3

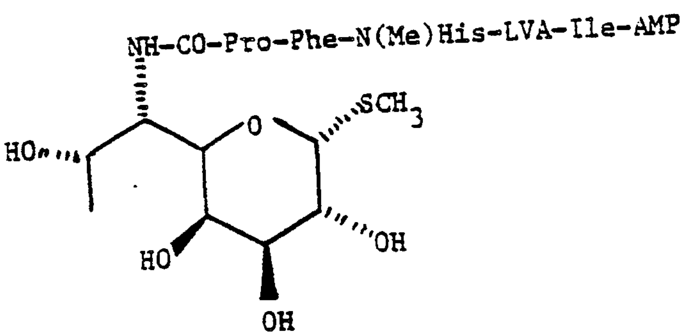

O-4

CHART P

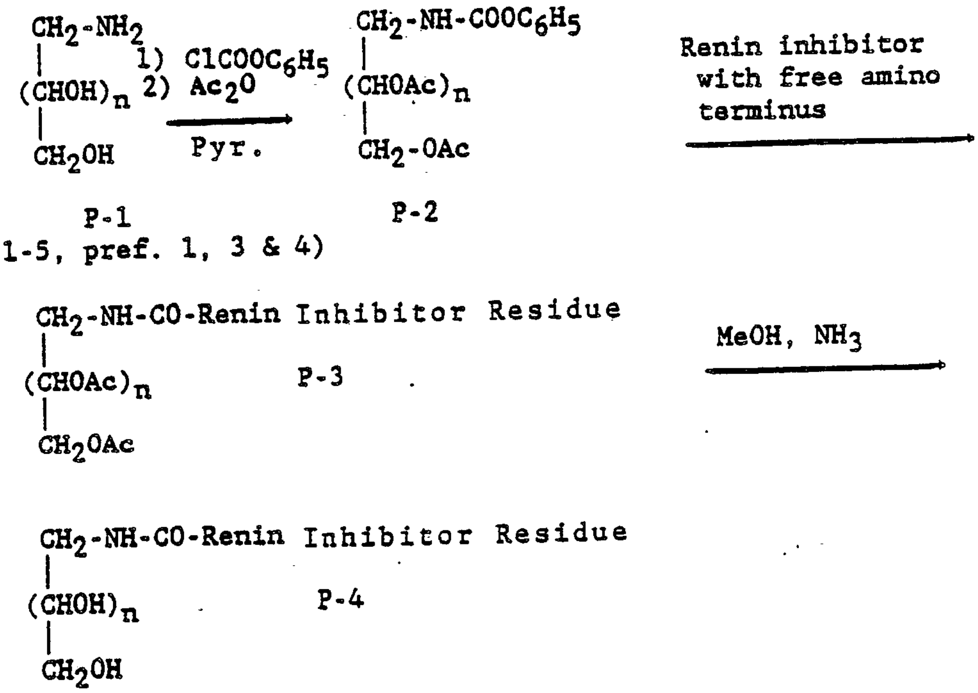


P-1 P-2

(n = 1-5, pref. 1, 3 & 4)

P-3

P-4

CHART Q

Aldose + Renin inhibitor with free amino terminus

Q-1

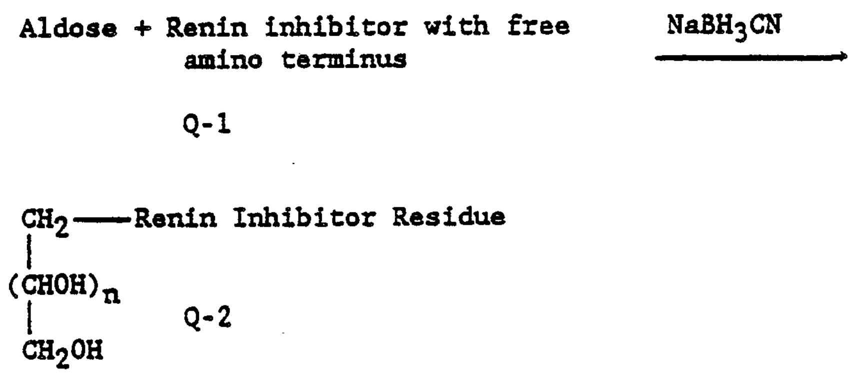


Q-2

(n = 1-5, pref. 3 or 4)

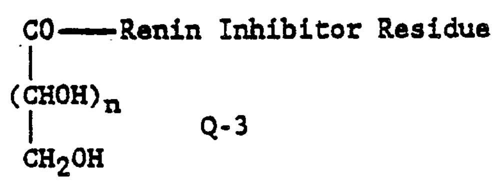

Q-3

(n = 2-5, pref. 3 or 4)

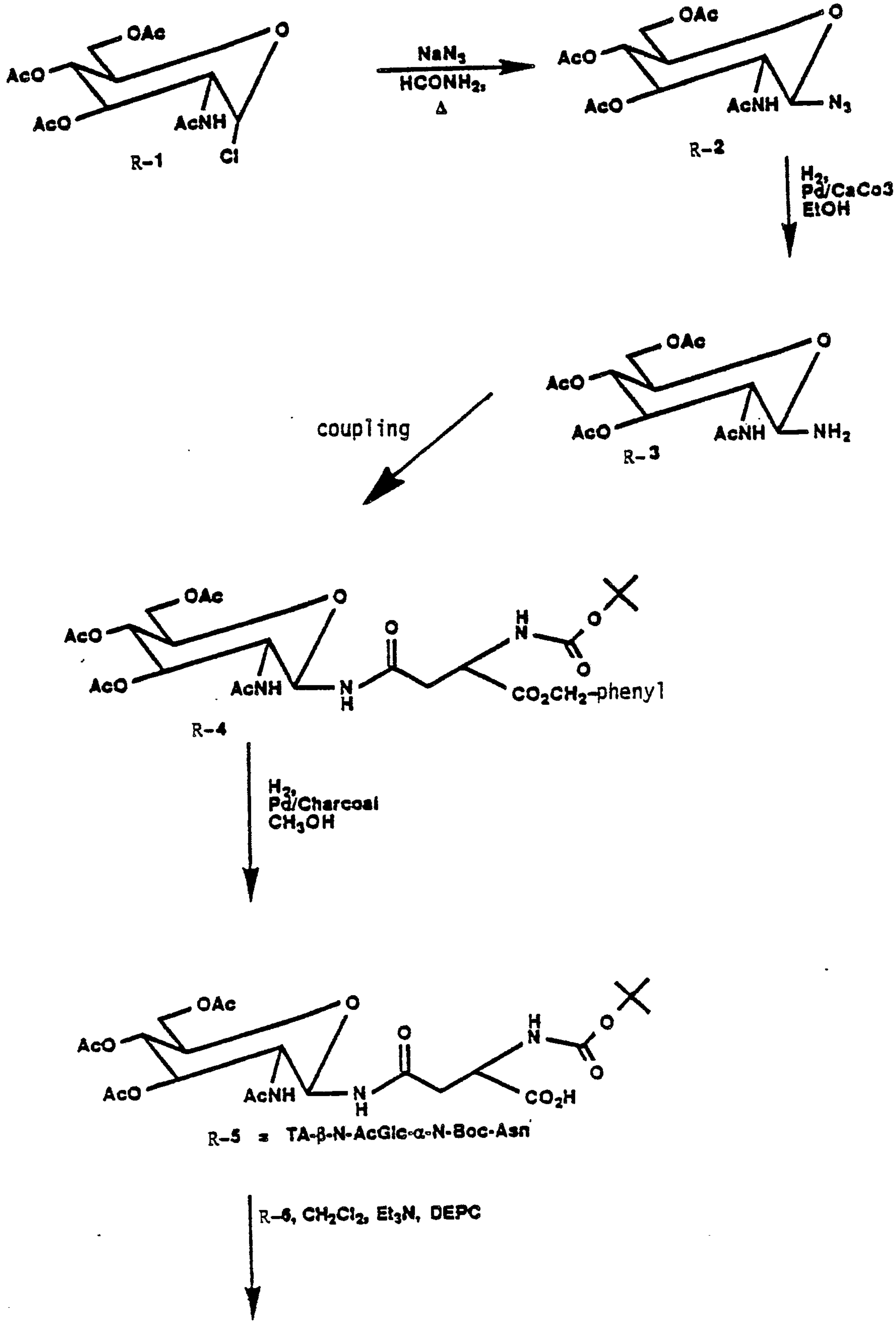


R-1

R-2

R-3

R-4

R-5 = TA-β-N-AcGlc-α-N-Boc-Asn

CHART R (Continued)

TA-β-N-AcGlc-α-N-Boc-Asn-Pro-Phe-N-MeHis(Ts)-Leu[CH(OH)$CH_2$]-Val-Ile-Amp

R-7

KOH-$H_2O$
$CH_3OH$

β-N-AcGlc-α-N-Boc-Asn-Pro-Phe-N-MeHis-Leu[CH(OH)$CH_2$]-Val-Ile-Amp

R-8

---

R-5

R-9, $CH_2Cl_2$, $Et_3N$, DEPC

TA-β-N-AcGlc-α-N-Boc-Asn-Phe-N-MeHis(Ts)-Leu[CH(OH)$CH_2$]-Val-Ile-Amp

R-10

KOH-$H_2O$
$CH_3OH$

β-N-AcGlc-α-N-Boc-Asn-Phe-N-MeHis-Leu[CH(OH)$CH_2$]-Val-Ile-Amp

R-11

---

Boc-Pro-Phe-N-MeHis(Ts)-Leu[CH(OH)$CH_2$]-Val-Ile-Amp

$CH_2Cl_2$, TFA

Pro-Phe-N-MeHis(Ts)-Leu[CH(OH)$CH_2$]-Val-Ile-Amp

R-6

---

Boc-Phe-N-MeHis(Ts)-Leu[CH(OH)$CH_2$]-Val-Ile-Amp

$CH_2Cl_2$, TFA

Phe-N-MeHis(Ts)-Leu[CH(OH)$CH_2$]-Val-Ile-Amp R-9

## CHART S

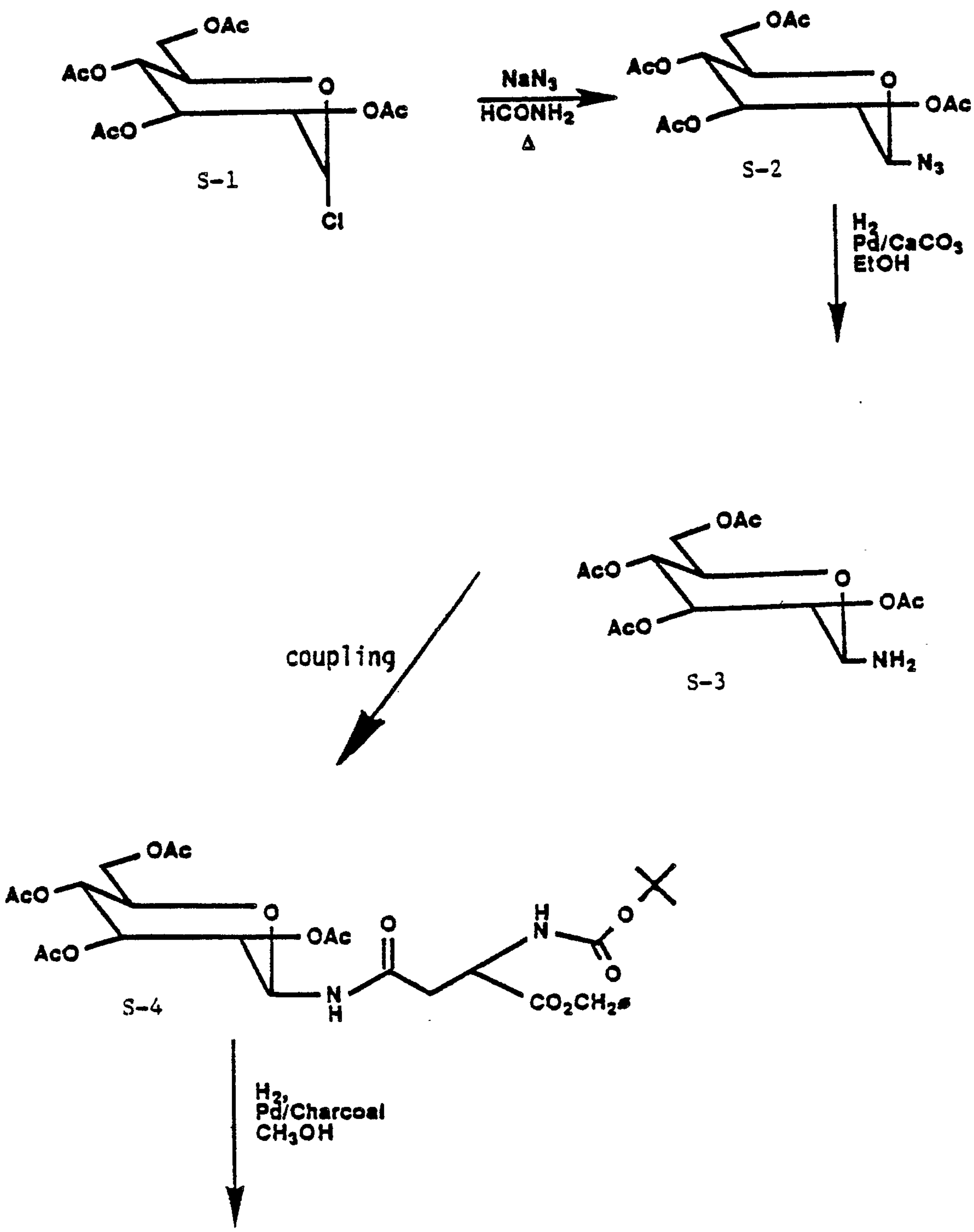


S-1

S-2

S-3

S-4

CHART S (Continued)

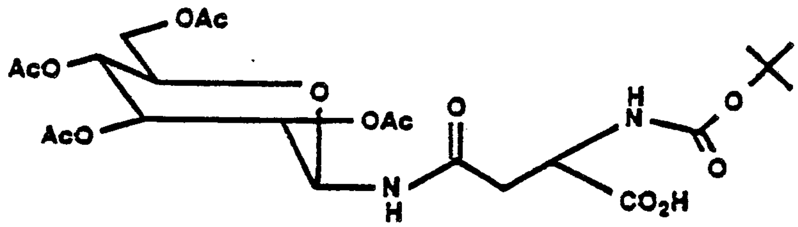

S-5 = TA-β-Man-α-N-Boc-Asn

R-6, $CH_2Cl_2$, $Et_3N$
DEPC

β-Man-α-N-Boc-Asn-Pro-Phe-N-MeHis-Leu[CH(OH)$CH_2$]-Val-Ile-Amp

S-6

KOH-$H_2O$
$CH_3OH$

β-Man-α-N-Boc-Asn-Pro-Phe-N-MeHis-Leu[CH(OH)$CH_2$]-Val-Ile-Amp

S-7

CHART T

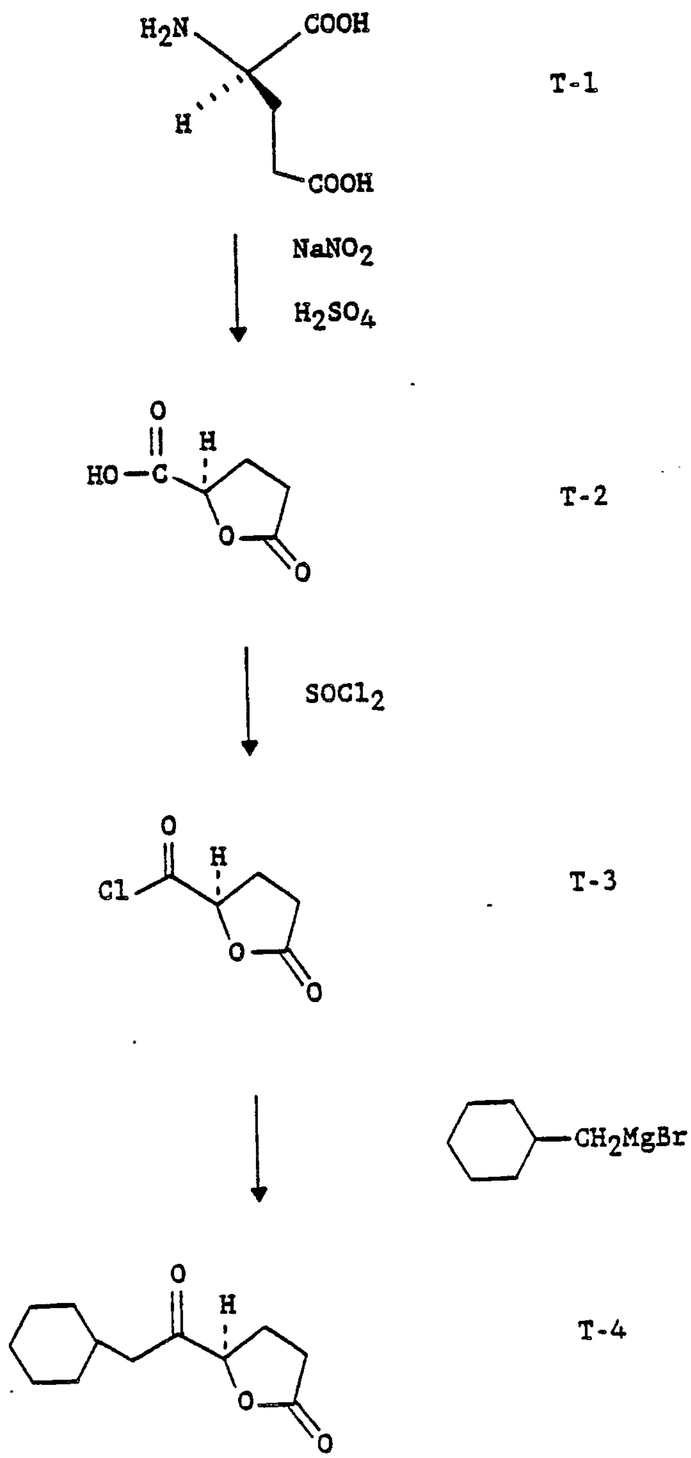


T-1

T-2

T-3

T-4

CHART T (Continued)

T-4

L-Selectride

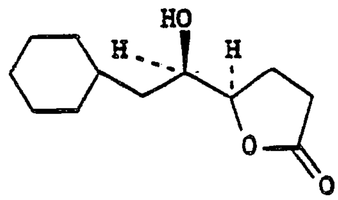

T-5

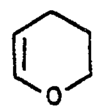

,

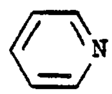

·pTSA

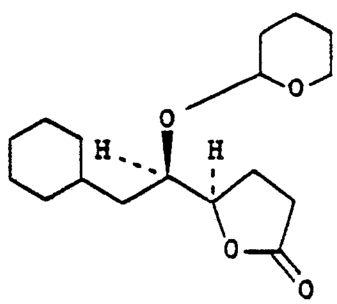

T-6

LDA,

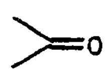

T-7

CHART T (Continued)

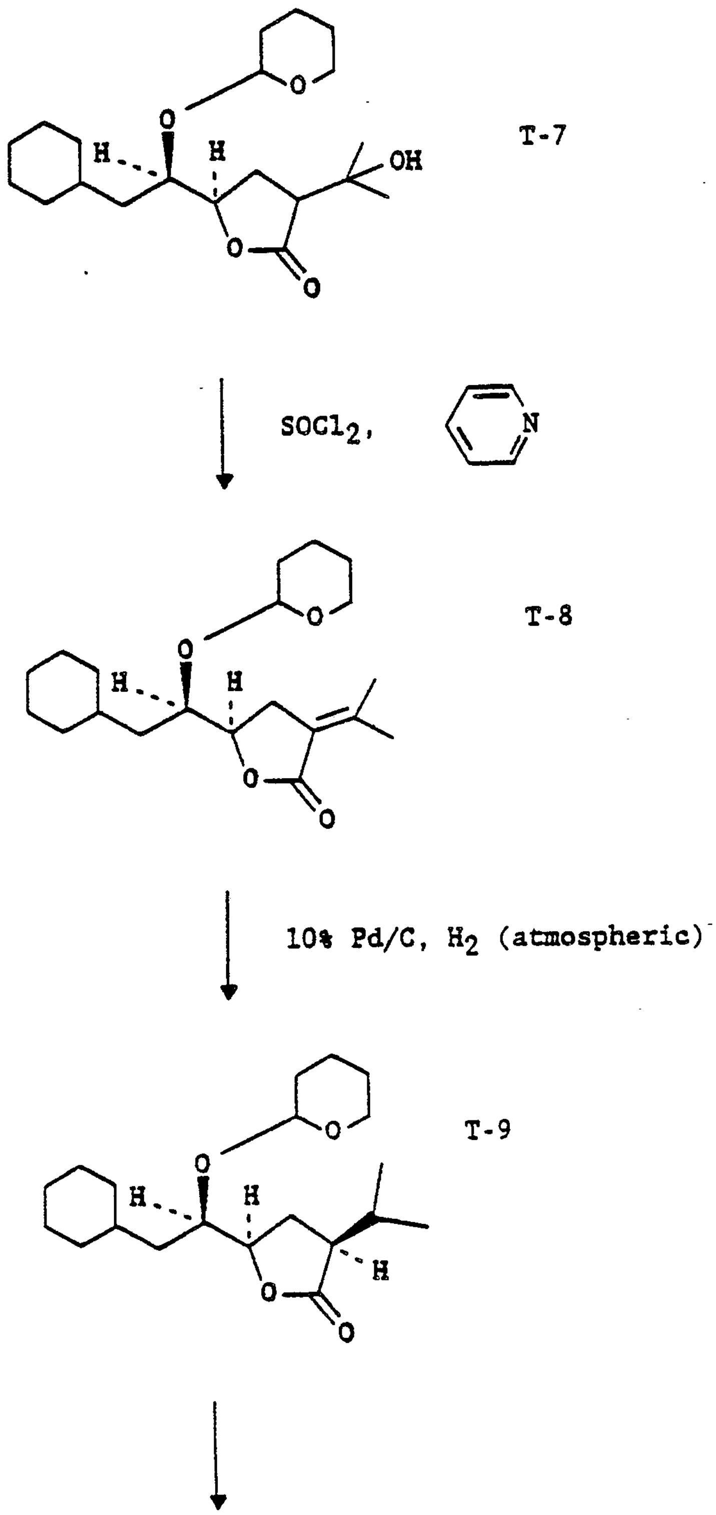


T-7

T-8

T-9

CHART T (Continued)

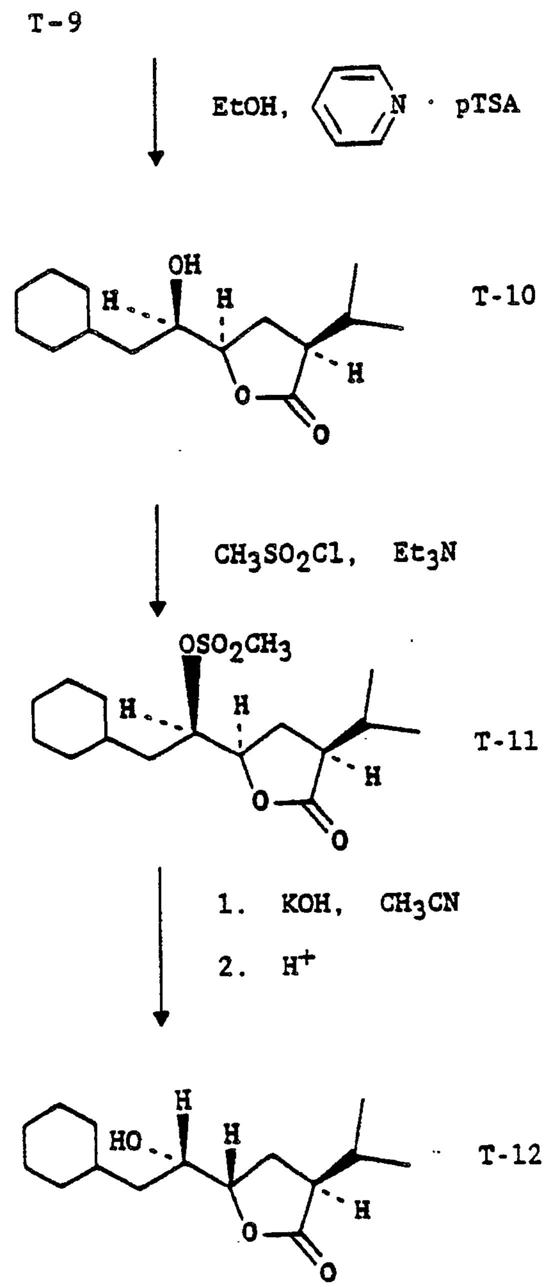

CHART T (Continued)

T-12

$CCl_4$, $(Phenyl)_3P$

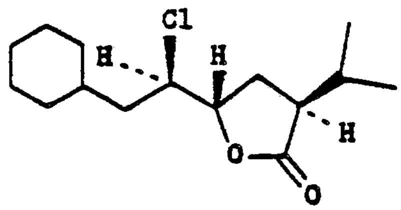

T-13

$NaN_3$

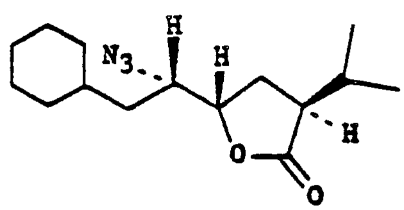

T-14

5% Pd/C, $H_2$

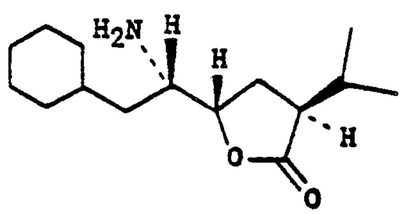

T-15

CHART T (Continued)

T-15

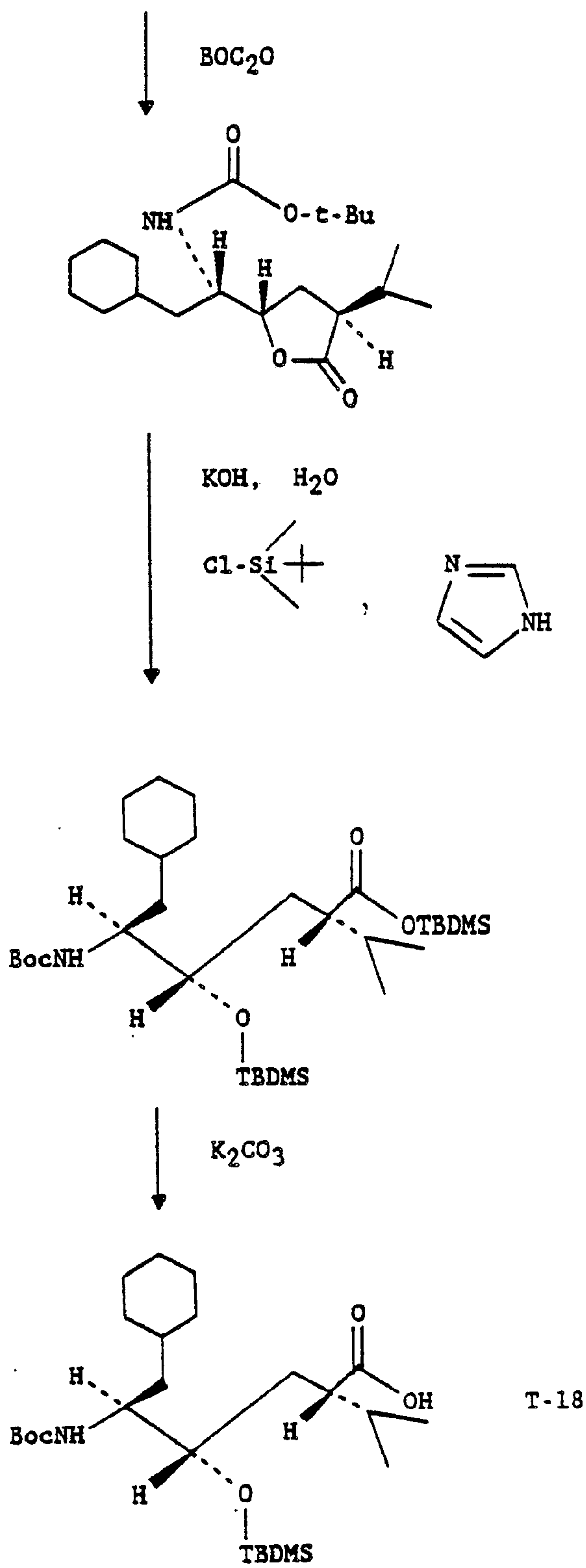


T-16

T-17

T-18

TABLE A

| Compound | X | $B_7$ | $C_8$ | $D_9$ | $E_{10}/F_{11}$ | $G_{12}$ | Z |
|---|---|---|---|---|---|---|---|
| 1. | THAM-$\overset{O}{\overset{\|}{C}}$- | Pro | Phe | N(Me)His | CVA | | $NH_2$ |
| 2. | S-$\overset{O}{\overset{\|}{C}}$- | Pro | Phe | N(Me)His | CVA | | $NH_2$ |
| 3. | THAM-$\overset{O}{\overset{\|}{C}}$- | Pro | Phe | N(Me)His | CVA | | $NH(CH_2)_2SO_2NH_2$ |
| 4. | S-$\overset{O}{\overset{\|}{C}}$- | Pro | Phe | N(Me)His | CVA | | $NH(CH_2)_2SO_2NH_2$ |
| 5. | THAM-$\overset{O}{\overset{\|}{C}}$- | Pro | Phe | N(Me)His | CVA | | $NH(CH_2)_2$(2-pyridyl) |
| 6. | S-$\overset{O}{\overset{\|}{C}}$- | Pro | Phe | N(Me)His | CVA | | $NH(CH_2)_2$(2-pyridyl) |
| 7. | THAM-$\overset{O}{\overset{\|}{C}}$- | Pro | Phe | N(Me)His | CVA | | $NH(CH_2)_2$(2-pyridyl), N-oxide |
| 8. | S-$\overset{O}{\overset{\|}{C}}$- | Pro | Phe | N(Me)His | CVA | | $NH(CH_2)_2$(2-pyridyl), N-oxide |
| 9. | THAM-$\overset{O}{\overset{\|}{C}}$- | Pro | Phe | N(Me)His | CVA | | $NH-CH_2$(2-pyridyl) |
| 10. | THAM-$\overset{O}{\overset{\|}{C}}$- | Pro | Phe | N(Me)His | CVA | | $NH-CH_2C(CH_2OH)_3$ |
| 11. | S-$\overset{O}{\overset{\|}{C}}$- | Pro | Phe | N(Me)His | CVA | | $NH-CH_2C(CH_2OH)_3$ |
| 12. | THAM-$\overset{O}{\overset{\|}{C}}$- | Pro | Phe | N(Me)His | CVA | | $NH-(CH_2)_2C(CH_2OH)_3$ |
| 13. | THAM-$\overset{O}{\overset{\|}{C}}$- | Pro | Phe | N(Me)His | CVA | | $NH(CH_2)_4CH(NH_2)$-(COOH) |
| 14. | $(HOCH_2)_3CCH_2NH\overset{O}{\overset{\|}{C}}$- | Pro | Phe | N(Me)His | CVA | Ile | $NH-CH_2$(2-pyridyl), N-oxide |
| 15. | $(HOCH_2)_3CCH_2NH\overset{O}{\overset{\|}{C}}$- | Pro | Phe | N(Me)His | CVA | Ile | $NH-CH_2$(2-pyridyl), N-oxide |
| 16. | $(HOCH_2)_3CCH_2C$ | Pro | Phe | N(Me)His | CVA | Ile | $NH-CH_2$(2-pyridyl), N-oxide |

S = 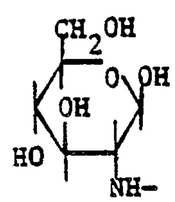

THAM = $(HOCH_2)_3CNH$-

$A_6$, $H_{13}$ and $I_{14}$ are absent.

## Claims

1. A renin inhibitory peptide having a noncleavable transition state insert corresponding to the 10,11-positicn of the renin substrate (angiotensinogen) and a polyhydroxy-substituted alkyl moiety bonded directly through carbon or nitrogen to the N-terminus.

2. A renin inhibitory peptide of claim 1, wherein there are at least three amino acid residues between the N-terminus and the transition state insert and with the proviso that when the N-terminus is substituted by THAM, the C-terminus is substituted by a heterocyclic or hetercyclic N-oxide moiety or a polyhydroxy-substituted alkyl moiety.

3. A renin inhibitory peptide of Claim 2 having one of the following moieties at the N-terminus

(a) $R_{120}N(R_{124})$-C(O)-,

(b) 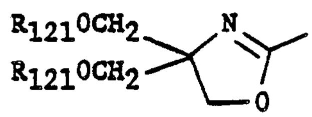 VI,

(c) $R_{120}N(R_{125})(CH_2)_qC(O)$-,
(d) $R_{127}C(O)N(R_{123})(CH_2)_qC(O)$-,
(e) $R_{120}N(R_{123})C(O)(CH_2)_nC(O)$-,
(f) $R_{127}C(0)$-, or
(g) $R_{121}OCH_2(CHOR_{121})_xCH_2$-;
wherein $R_{120}$ is
(a) $R_{121}OCH_2$-$C(CH_2OR_{121})_2$,

(b) 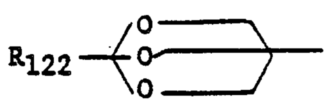 VIII,

(c) $R_{123}OC(O)$-$C((CH_2)_n$-$CO_2R_{123})_2$-,
(d) HO-$CH_2$-CH(OH)-$CH_2$-,

(e) 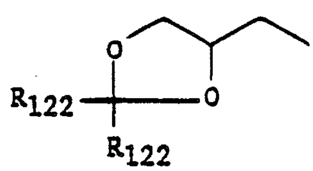 IX,

(f) 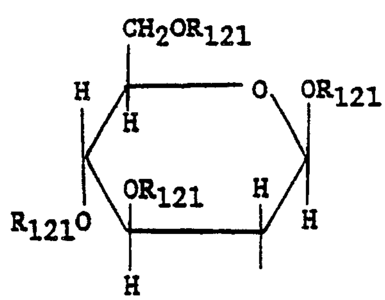 X,

(g) $R_{126}C[(CH_2)_qOR_{121}]_2(CH_2)_q$-,

(h) 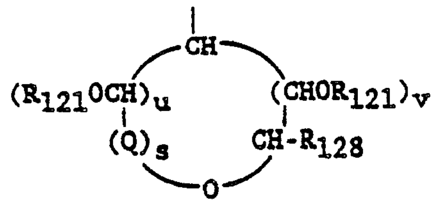 ,

(i) 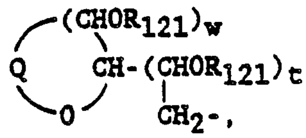

(j) $-CH_2(CHOR_{121})_xCH_2OR_{121}$,

(k) $R_{121}OCH_2(CHOR_{121})_y\overset{|}{C}H-(CHOR_{121})_z(CH_2OR_{121}$, or

(l) 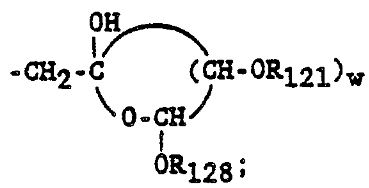

(m) 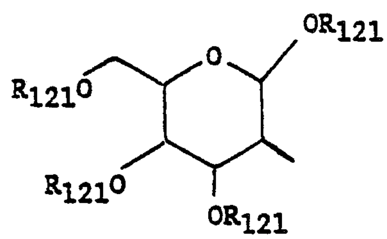 ,

(n) 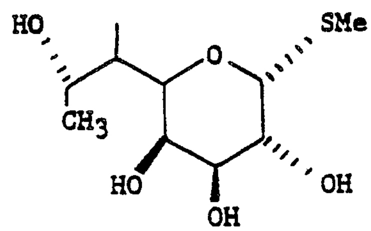 ,

(o) 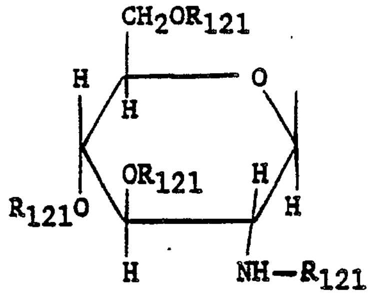 , or

(p) 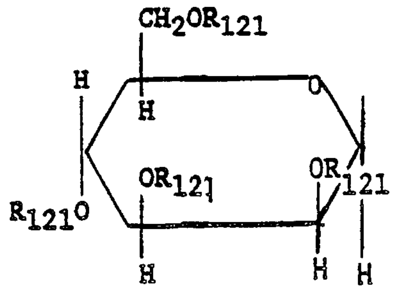

wherein $R_{121}$ is
(a) hydrogen,
(b) $C_1$-$C_6$ alkyl,
(c) $-(CH_2)_n$-aryl, or
(d) $-C(O)R_{123}$;
wherein $R_{122}$ is
(a) hydrogen,
(b) $C_1$-$C_6$ alkyl,
(c) trifluoromethyl, or
(d) aryl;
wherein $R_{123}$ is
(a) hydrogen,
(b) $C_1$-$C_6$ alkyl, or
(c) $-(CH_2)_n$-aryl;
wherein $R_{124}$ is
(a) hydrogen,
(b) $HO-CH_2-CH(OH)-CH_2-$, or

(c) 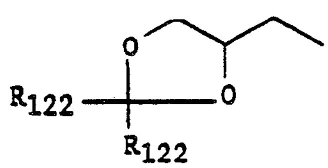 ;

provided that when $R_{124}$ is other than hydrogen, $R_{120}$ and $R_{124}$ are the same;
wherein $R_{125}$ is:
(a) hydrogen,
(b) $C_1$-$C_6$alkyl,
(c) $-(CH_2)_n$aryl, or
(d) $-C(O)R_{123}$;
wherein $R_{126}$ is:
(a) hydrogen, or
(b) $(CH_2)_nOR_{121}$;
wherein $R_{127}$ is:
(a) $-CHOR_{121}(CHOR_{121})_xCH_2OR_{121}$,

(b) 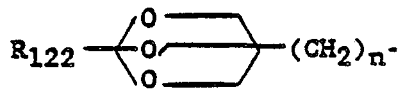 ,

(c) $-(CH_2)_nC[(CH_2)_qOR_{121}]_2R_{126}$,

(d) 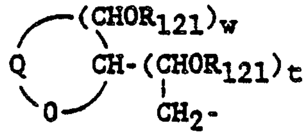 ,

(e) 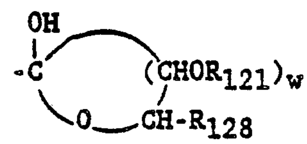 , or

(f) 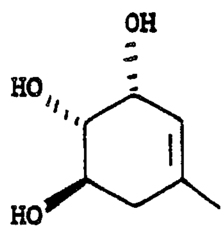 ;

wherein $R_{128}$ is:
(a) hydrogen, or
(b) $-(CHOR_{121})_tCH_2OR_{121}$;
wherein Q is:
(a) $CH_2$,
(b) $CHOR_{121}$, or
(c) C(O);
wherein for each occurrence n is independently an integer of zero to five, inclusive;
wherein q is an integer of one to five, inclusive;
wherein u is an integer of zero to three, inclusive;
wherein v is an integer of zero to four, inclusive;
wherein s is an integer of zero or one so that the sum of u plus v plus s is three or four;
wherein t is an integer of zero to three, inclusive;
wherein w is an integer of two or three;
wherein x is an integer of two to seven, inclusive;
wherein y is an integer of zero to six, inclusive; and
wherein z is an integer of zero to six so that the sum of y plus z does not exceed six;
wherein aryl is phenyl or naphthyl substituted by zero to three of the following:
(a) $C_1$-$C_3$alkyl,
(b) hydroxy,
(c) $C_1$-$C_3$alkoxy,
(d) halo,
(e) amino,
(f) mono- or di-$C_1$-$C_3$alkylamino,
(g) -CHO,
(h) -COOH,
(i) $COOR_{26}$,
(j) $CONHR_{26}$,
(k) nitro,
(l) mercapto,

(m) $C_1$-$C_3$alkylthio,
(n) $C_1$-$C_3$alkylsulfinyl,
(o) $C_1$-$C_3$alkylsulfonyl,
(p) -N($R_4$)-$C_1$-$C_3$alkylsulfonyl,
(q) $SO_3H$,
(r) $SO_2NH_2$,
(s) -CN, or
(t) -$CH_2NH_2$;
wherein $R_4$ at each occurrence is the same or different and is
(a) hydrogen,
(b) $C_1$-$C_5$ alkyl,
(c) -$(CH_2)_p$-aryl,
(d) -$(CH_2)_p$-Het,
(e) -$(CH)_p$-$C_3$-$C_7$ cycloalkyl, or
(f) 1- or 2-adamantyl;
wherein $R_{26}$ is
(a) hydrogen,
(b) $C_1$-$C_3$ alkyl, or
(c) phenyl-$C_1$-$C_3$ alkyl;
wherein -Het is a 5- or 6-membered saturated or unsaturated ring containing from one to three heteroatoms selected from the group consisting of nitrogen, oxygen, and sulfur; and including any bicyclic group in which any of the above heterocyclic rings is fused to a benzene ring or another heterocycle; and if chemically feasible, the nitrogen and sulfur atoms may be in the oxidized forms; and optionally substituted by zero to three of the following;
(a) $C_1$-$C_5$-alkyl,
(b) hydroxy,
(c) hydroxy ($C_1$-$C_5$alkyl),
(d) halogen,
(e) amino,
(f) amino ($C_1$-$C_5$alkyl),
(g) -CHO,
(h) -$CO_2H$,
(i) -$CO_2$-($C_1$-$C_5$alkyl),
(j) -$CONH_2$,
(k) -CONH-($C_1$-$C_5$alkyl),
(l) -nitro,
(m) mercapto,
(n) mercapto ($C_1$-$C_5$alkyl),
(o) -$SO_3H$,
(p) -$SO_2NH_2$,
(q) -CN, or
(r) -O-$C_1$-$C_5$alkyl;
or a carboxy-, amino- or other reactive group protected form thereof;
or a pharmaceutically acceptable acid or base addition salts thereof.

4. A renin inhibitory peptide of Claim 3 having one of the following moieties at the C-terminus
(a) -$NR_{120}(R_{124})$,
(b) AMP, or

(c) 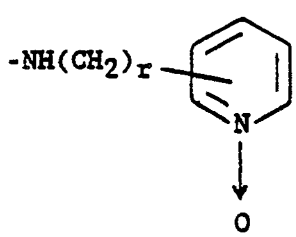 VII,

wherein r is an integer of zero to five inclusive.

5. A renin inhibitory peptide of Claim 3 of the formula
$X_1$-$B_7$-$C_8$-$D_9$-$E_{10}$-$F_{11}$-$G_{12}$-$Z_1$
wherein $X_1$ is

(a) 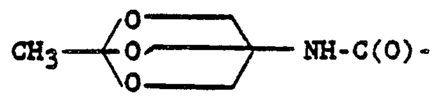 ,

(b) THAM-C(O)-,

(c) 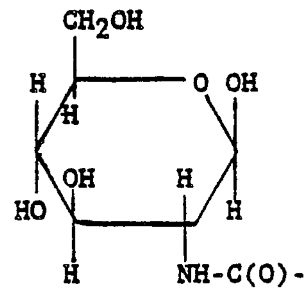 ,

(d) 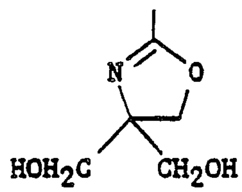 , or

(e) 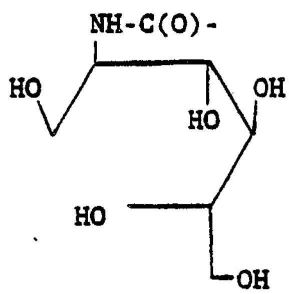 ,

wherein $B_7$ is Pro or a derivative thereof;
wherein $C_8$ is Phe or a derivative thereof;
wherein $D_9$ is His, N(Me)His or a derivative thereof;
wherein $E_{10}$-$F_{11}$ is LVA or a derivative thereof;
wherein $G_{12}$ is Ile or a derivative thereof;
wherein $Z_1$ is
(a) AMP, or
(b) AMP-NO.

6. A renin inhibitory peptide of Claim 5 selected from the group consisting of:
N-(1-Methyl-2,6,7-trioxabicyclo[2.2.2.]oct-4-yl-aminocarbonyl)-Pro-Phe-N(Me)His-LVA-Ile-AMP;
N-[[[2-Hydroxy-1,1-bis(hydroxymethyl)ethyl]amino]carbonyl]-Pro-Phe-N(Me)His-LVA-Ile-AMP;
N-[[[2-Hydroxy-1,1-bis(hydroxymethyl)ethyl]amino]carbonyl]-Pro-Phe-N(Me)His-LVA-Ile-AMP, pyridine N-oxide;
N-(Glucosaminocarbonyl)-Pro-Phe-N(Me)His-LVA-Ile-AMP;
N-[4,4-Bis(hydroxymethyl)-4,5-dihydrooxazol-2-yl]-Pro-Phe-N(Me)His-LVA-Ile-AMP;
N-[4,4-Bis(Hydroxymethyl)-4,5-dihydrooxazol-2-yl]-Pro-Phe-N(Me)His-LVA-Ile-AMP, pyridine N-oxide;
(Glucosaminocarbonyl)-Pro-Phe-N(Me)His-LVA-Ile-AMP, N-oxide; and
L-Histidinamide 1-[[(2-deoxy-L-glucitol-2-yl)amino]carbonyl]-L-prolyl-L-phenylalanyl-N-[2-hydroxy-5-methyl-

1-(2-methylpropyl)-4-[[[2-methyl-1-[[(2-pyridinylmethyl)amino]carbonyl]butyl]amino]carbonyl]hexyl]-N.alpha.-methyl-, N-oxide;
N-[[[2-Hydroxy-1,1-bis(hydroxymethyl)ethyl]amino]carbonyl]-Pro-Phe-N(Me)His-LVA-Ile-AMP, pyridine N-oxide, hydrochloride;
N-[[[2-Hydroxy-1,1-bis(hydroxymethyl)ethyl]amino]carbonyl]-Pro-Phe-N(Me)His-LVA-Ile-AMP, pyridine N-Oxide, methanesulfonate;
N-[[[2-Hydroxy-1,1-bis(hydroxymethyl)ethyl]amino]carbonyl]-Pro-Phe-N(Me)His-LVA-Ile-AMP, pyridine N-oxide, fumarate; and
N-[[[2-Hydroxy-1,1-bis(hydroxymethyl)ethyl]amino]carbonyl]-Pro-Phe-N(Me)His-LVA-Ile-AMP, pyridine N-oxide, succinate.

7. A renin inhibitory peptide of Claim 3 of the formula
$X_2$-$B_7$-$C_{81}$-$D_9$-$E_{101}$-$F_{111}$-$G_{121}$-$Z_2$
wherein $X_2$ is
(a) THAM-C(O)-, or

(b) 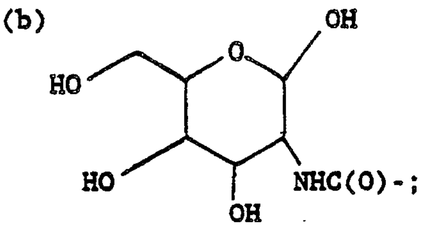 (Glu)

wherein $B_7$ is Pro or a derivative thereof;
wherein $C_{81}$ is Phe, $(OCH_3)$Tyr or a derivative thereof;
wherein $D_9$ is His, N(Me)His or a derivative thereof;
wherein $E_{101}$-$F_{111}$ is

(a) 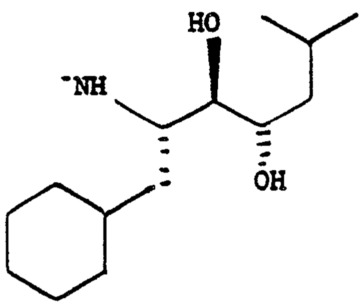 XI ,

(Cha-Val-glycol or CVG)

(b) LVA,
(c) CVA, or a derivative thereof;
wherein $G_{121}$ is absent, Ile or a derivative thereof;
wherein $Z_2$ is
(a) absent,
(b) AMP,
(c) AMP-NO,
(d) MBA, or
(e) 4-(aminomethyl)piperidine (Ampip).

8. A renin inhibitory peptide of claim 7 selected from the group consisting of
L-Histidinamide, 1-[[[2-hydroxy-1,1-bis(hydroxymethyl)ethyl]-amino]carbonyl]-L-prolyl-L-phenyl-alanyl-N-[1-(cyclohexylmethyl)-2,3-dihydroxy-5-methylhexyl]-N.alpha.-methyl-, [1S-(1R*,2S*,3R*)]; or THAM-Pro-Phe-N-MeHis-CVG;
L-Histidinamide, 1-[[[2-hydroxy-1,1-bis-hydroxymethyl)ethyl]-amino]carbonyl]-L-prolyl-O-methyl-L-tyrosyl-N-[1-cyclohexylmethyl)-2,3-dihdyroxy-5-methylhexyl]-, [1S-(1R*,2S*,3R*)]; or THAM-Pro-Tyr($OCH_3$)-His-CVG;
L-Histidinamide, 1-[[2-deoxy-D-glucopyranos-2-yl)amino]carbonyl]-L-prolyl-L-phenylalanyl-N-[1-

(cyclohexylmethyl)-2,3-dihydroxy-5-methylhexyl]-N.alpha.-methyl-, [1S-(1R*,2S*,3R*)]; or Glu-Pro-Phe-N-MeHis-CVG;
L-Histidinamide, 1-[[2-deoxy-D-glucopyranos-2-yl)amino]carbonyl]-L-prolyl-L-tyrosyl-N-[1-(cyclohexylmethyl)-2,3-dihdyroxy-5-methylhexyl]-, [1S-(1R*,2S*,3R*)]; or Glu-Pro-Tyr($OCH_3$)-His-CVG;
L-Histidinamide, 1-[[2-deoxy-D-glucopyranos-2-yl)amino]carbonyl]-L-prolyl-L-phenylalanyl-N-[4-[[[5-amino-1-[[(4-piperidinylmethyl)amino]carbonyl]pentyl]amino]carbonyl]-2-hydroxy-5-methyl-1(2-methylpropyl)hexyl]-N.alpha.-methyl-, [1S[1R*,2R*,4R*(R*)]]-, triacetate (salt); or Glu-Pro-Phe-N-MeHis-LVA-Lys-Ampip; and
L-Histidinamide, 1-[[2-deoxy-D-glucopyranos-2-yl)amino]carbonyl]-L-prolyl-L-phenylalanyl-N-[1-(cyclohexylmethyl)-2-hydroxy-5-methyl-4-[[(2-methylbutyl)amino]carbonyl]hexyl]-N.alpha.-methyl-, [1S-[1R*,2R*,4R*(R*)]]-; or Glu-Pro-Phe-N-MeHis-CVA-MBA.

9. A renin inhibitory peptide of Claim 3 of the formula
$X_3$-$B_7$-$C_8$-$D_9$-$E_{10}$-$F_{11}$-$G_{12}$-$Z_3$
wherein $X_3$ is
(a) $(HOCH_2)_2$-C(Me)-C(O)-,
(b) $(HOCH_2)_2$-CH-NHC(O)-,
(c) -C(O)-NH-$CH_2$-CHOH-CHOH-CHOH-CHOH-$CH_2$OH,
(d) -$CH_2$-CHOH-CHOH-CHOH-$CH_2$OH,
(e) -$CH_2$-CHOH-CHOH-CHOH-CHOH-$CH_2$OH,
(f) -C(O)-CHOH-CHOH-CHOH-CHOH-$CH_2$OH,

(g) 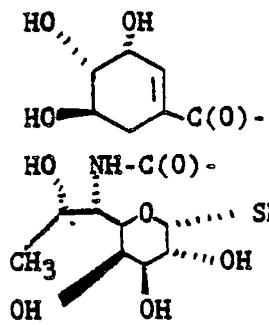 or
(h) ;

wherein $B_7$ is Pro, or a derivative thereof;
wherein $C_8$ is Phe or a derivative thereof;
wherein $D_9$ is His, N(Me)His or a derivative thereof;
wherein $E_{10}$-$F_{11}$ is LVA or a derivative thereof;
wherein $G_{12}$ is Ile or a derivative thereof;
wherein $Z_3$ is
(a) AMP, or
(b) AMP-NO.

10. A renin inhibitory peptide of Claim 9 selected from the group consisting of:
$[HOCH_2]_2$CHNHCO-Pro-Phe-N[$CH_3$]-His-LVA-Ile-AMP;
$[HOCH_2]_2$CHNHCO-Pro-Phe-N[$CH_3$]-His-LVA-Ile-AMP-NO;
N-[Glucaminocarbonyl]-Pro-Phe-N[$CH_3$]-His-LVA-Ile-AMP;
N-[S-Methyl-1-thiolincosaminide]carbonyl-Pro-Phe-N[$CH_3$]-His-LVA-Ile-AMP;
N-[2,3,4,5-Tetrahydroxypentyl]-Pro-Phe-N[$CH_3$]-His-LVA-Ile-AMP;
N-[2,3,4,5,6-Pentahydroxyhexyl]-Pro-Phe-N[$CH_3$]-His-LVA-Ile-AMP; and
N-D-Gluconoyl-Pro-Phe-N[$CH_3$]-His-LVA-Ile-AMP.

11. A renin inhibitory peptide of Claim 3 of the formula
$X_4$-$B_7$-$C_8$-$D_9$-$E_{102}$-$F_{112}$-$Z_4$
wherein $X_4$ is
(a) THAM-C(O)-or

(b) 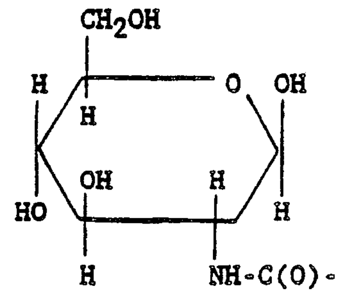 (DGLU);

wherein $B_7$ is Pro or a derivative thereof;
wherein $C_8$ is Phe or a derivative thereof;
wherein $D_9$ is His, N(Me)His or a derivative thereof;
wherein $E_{102}$-$F_{112}$ is CVA or a derivative thereof;
wherein $Z_4$ is

(a) (2-hydroxypropyl)amino,
(b) (2,3-dihydroxypropyl)amino,
(c) (OMe)Glu-AMP,
(d) AMP, or
(e) AMP-NO.

12. A renin inhibitory peptide of Claim 11 selected from the group consisting of:
DGLU-C(O)-Pro-Phe-N(Me)His-CVA-(2-hydroxypropyl)amino;
THAM-C(O)-Pro-Phe-N(Me)His-CVA-(2,3-dihydroxypropyl)amino;
DGLU-C(P)-Pro-Phe-N(Me)His-CVA-(2,3-dihydroxypropyl)amino;
THAM-C(O)-Pro-Phe-N(Me)His-CVA-(OMe)Glu-AMP;
THAM-C(O)-Pro-Phe-N(Me)His-CVA-AMP-NO;
DGLU-C(O)-Pro-Phe-N(Me)His-CVA-AMP; and
DGLU-C(O)-Pro-Phe-N(Me)His-CVA-AMP-NO.

13. A renin inhibitory peptide of Claim 3 of formula
$X_5$-$A_6$-$B_7$-$C_8$-$D_9$-$E_{103}$-$F_{113}$-$G_{123}$-$Z_5$
wherein $X_5$ is an N-linked monosaccharide;
wherein $A_6$ is an alkanedioic acid or a derivative thereof;
wherein $B_7$ is Pro or a derivative thereof;
wherein $C_8$ is Phe or a derivative thereof;
wherein $D_9$ is His, N(Me)His or a derivative thereof;
wherein $E_{103}$-$F_{113}$ is LVA, CVA, -Cha$\psi$[$CH_2NH$]Phe-$NH_2$, or a derivative thereof;
wherein $G_{123}$ is absent, Ile or a derivative thereof;
wherein $Z_5$ is

(a) absent,
(b) AMP,
(c) AMP-NO, or
(d) -NH-C($CH_2OH$)$_3$.

14. A renin inhibitory peptide of Claim 13 wherein $X_5$ is

(a) 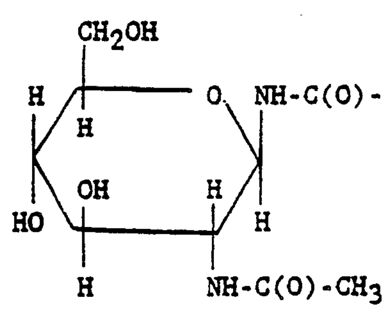 ($\beta$-N-AcGlc), or

(b) 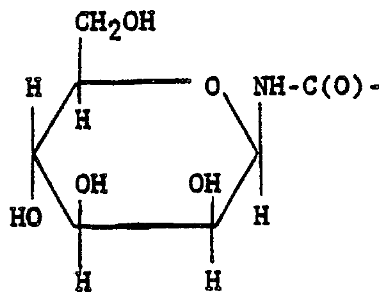 ($\beta$-Man);

wherein $A_6$ is
(a) $\alpha$-Asn,
(b) $\alpha$-N-Boc-Asn,
(c) $-C(O)-(CH_2)_2-C(O)-$, or a derivative thereof.

15. A renin inhibitory peptide of Claim 13 selected from the group consisting of:
N-[$\beta$-N-AcGlc]-$\alpha$-N-Boc-Asn-Pro-Phe-N-(Me)His-LVA-Ile-AMP;
N-[$\beta$-Man]-$\alpha$-N-Boc-Asn-Pro-Phe-N-(Me)His-LVA-Ile-AMP;
N-[$\beta$-N-AcGlc]-$\alpha$-N-Boc-Asn-Pro-Phe-N-(Me)His-LVA-Ile-AMP-NO;
N-[$\beta$-N-AcGlc]-$\alpha$-Asn-Pro-Phe-N-(Me)His-LVA-Ile-AMP;
N-[$\beta$-N-AcGlc]-$\alpha$-Asn-Pro-Phe-N-(Me)His-LVA-Ile-AMP-NO;
N-[$\beta$-N-AcGlc]-$\alpha$-N-Boc-Asn-Pro-Phe-N-(Me)His-LVA-Ile-NH-$C(CH_2OH)_3$;
N-[$\beta$-N-AcGlc]-$C(O)-(CH_2)_2-C(O)$-Pro-Phe-N-(Me)His-LVA-Ile-AMP;
N-[$\beta$-N-AcGlc]-$\alpha$-N-Boc-D-Asn-Pro-Phe-N-(Me)His-LVA-Ile-AMP; and
N-[$\beta$-N-AcGlc]-$\alpha$-N-Boc-Asn-Pro-Phe-His-Cha$\psi$[$CH_2NH$]Phe-$NH_2$.

16. A renin inhibitory peptide of Claim 1 selected from the group consisting of:
L-Histidinamide, N-[[[2-hydroxy-1,1-bis(hydroxymethyl)ethyl]-amino]carbonyl]-L-phenylalanyl-N-[2-hydroxy-5-methyl-1-(2-methylpropyl)-4-[[[2-methyl-1-[[(2-pyridinylmethyl)amino]carbonyl]butyl]amino]-carbonyl]-hexyl]-N.alpha.-methyl-, [1S-[1R*,2R*,4R*(1R*,2R*)]]-;
[3,4,5-Trihydroxy-1-cyclohexane-1-]-CO-Phe-N[$CH_3$]-His-LVA-Ile-AMP;
[$HOCH_2$]$_2$C[$CH_3$]CO-Phe-N[$CH_3$]-His-LVA-Ile-AMP; and
N-[$\beta$-N-AcGlc]-$\alpha$-N-Boc-Asn-Phe-N-(Me)His-LVA-Ile-AMP ; and
THAM-C(O)-Pro-Phe-N(Me)His-CVA-(2-hydroxypropyl)amino.

17. A compound of the formula

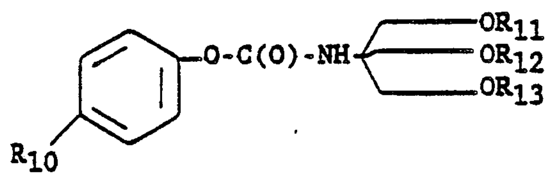

XX

wherein $R_{10}$ is an electron-withdrawing and anion-stabilizing group;
wherein $R_{11}$, $R_{12}$ and $R_{13}$ are each independently
(a) trimethylsilyl,
(b) t-butyl-dimethylsilyl, or
(c) $-C(O)R_{20}$;.
wherein $R_{20}$ is
(a) hydrogen or
(b) ($C_1$-$C_4$alkyl);
provided that only one of $R_{11}$, $R_{12}$ and $R_{13}$ may be t-butyldimethylsilyl.

18. A renin inhibitory peptide of claim 1 with one of the following moieties at the C-terminus:
(a) $-O-R_{10}$,
(b) $-N(R_4)R_{14}$,
(c) $C_4$-$C_8$ cyclic amino
(d) $-NR_{120}(R_{124})$, or

(e) 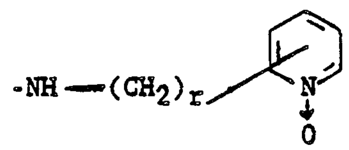

wherein $R_4$ at each occurrence is the same or different and is
(a) hydrogen,
(b) $C_1$-$C_5$alkyl,
(c) -$(CH_2)_p$-aryl,
(d) -$(CH_2)_p$-Het,
(e) -$(CH_2)_p$-$C_3$-$C_7$cycloalkyl, or
(f) 1- or 2-adamantyl;
wherein $R_{10}$ is
(a) hydrogen,
(b) $C_1$-$C_5$alkyl,
(c) -$(CH_2)_n$Het,
(d) $C_3$-$C_7$cycloalkyl, or
(e) a pharmaceutically acceptable cation;
wherein $R_{14}$ is
(a) hydrogen,
(b) $C_1$-$C_{10}$alkyl,
(c) (hydroxy $C_1$-$C_8$alkyl), or (d) -$(CH_2)_n$-$R_{18}$, or
(e) ($C_1$-$C_3$alkoxy)$C_1$-$C_8$alkyl;
wherein $R_{18}$ is
(a) amino
(b) mono-, or di- ($C_1$-$C_3$alkyl)amino,
(c) $C_4$-$C_7$cyclic amino, or
(d) $C_4$-$C_7$cycloalkylamino;
wherein p is zero to two, inclusive;
wherein -Het, aryl, $R_{120}$, $R_{124}$, and n are defined as in claim 3.

European Patent Office

# PARTIAL EUROPEAN SEARCH REPORT

which under Rule 45 of the European Patent Convention shall be considered, for the purposes of subsequent proceedings, as the European search report

Application number EP 89 30 0825

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 163 237 (MERCK)<br>* Page 75, claim 1, lines 23-26 *<br>-- | 5-17 | C 07 K 5/02<br>A 61 K 37/64 |
| D,A | EP-A-0 173 481 (UPJOHN)<br>-- | | |
| D,P,Y | US-A-4 729 985 (E.F. KLEINMANN)<br>* Whole document *<br>-- | | |
| D,P,Y | WO-A-88 02 756 (SANDOZ)<br>* Pages 103-109; claims 1-3 *<br>-- | 5,7,9,11 | |
| D,P,Y | GB-A-2 200 115(SANDOZ)<br>* Pages 64-65; claim 1 *<br>--------- | 5,7,9,11 | |

TECHNICAL FIELDS SEARCHED (Int. Cl.4)

C 07 K
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 6,8,10,12,15,16,17
Claims searched incompletely: 5,7,9,11,13,14
Claims not searched: 1-4,18

Reason for the limitation of the search:

The claims 1-4 and 18 are speculative with regard to the structure of the "renin inhibitory peptides". In claims 5,7,9,11,13,14 "derivatives" are mentioned, which are not in the slightest specified. Therefore the search was restricted to claims 6,8,10,12,15,16,17, and parts of the claims 5,7,9,11,14. (cf. articles 83 and 84 EPC)

| Place of search | Date of completion of the search | Examiner |
|---|---|---|
| The Hague | 27-04-1989 | HERMANN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82